# EUROPEAN PATENT SPECIFICATION

(11) **EP 3 064 272 B1**
(45) Date of publication and mention of the grant of the patent: **28.11.2018**
(21) Application number: 16164005.7
(22) Date of filing: 30.12.2010
(51) Int. Cl.: B01J 31/22, C07F 15/00, C08F 4/80, C08C 19/08

(54) **HIGHLY ACTIVE METATHESIS CATALYSTS SELECTIVE FOR ROMP AND RCM REACTIONS**
HOCHAKTIVE METATHESEKATALYSATOREN, DIE FÜR ROMP- UND RCM-REAKTIONEN SELEKTIV SIND
CATALYSEURS DE MÉTATHÈSE HAUTEMENT ACTIFS ET SÉLECTIFS POUR DES RÉACTIONS ROMP ET RCM

(30) Priority: 30.12.2009 WO PCT/CN2009/076226
(43) Date of publication of application: 07.09.2016
(62) Divisional of application: 10840586.1
(73) Proprietor: Zannan Scitech Co., Ltd., Shanghai 201 108 (CN)
(72) Inventor: ZHAN, Zheng-Yun James, 201108 SHANGHAI (CN)
(74) Representative: Biggi, Cristina

(56) References cited:
- US-A1- 2005 250 753
- DATABASE CA [Online] CHEMICAL ABSTRACTS SERVICE, COLUMBUS, OHIO, US; FUSTERO, SANTOS ET AL: "Organocatalytic approach to benzofused nitrogen-containing heterocycles: enantioselective total synthesis of (+)-Angustureine", XP002759803, retrieved from STN Database accession no. 2008:1448802
- DATABASE CA [Online] CHEMICAL ABSTRACTS SERVICE, COLUMBUS, OHIO, US; MINVILLE, JOANNIE ET AL: "A general synthesis of quinolinones and benzothiazine 1,1-dioxides via ring closing metathesis", XP002759804, retrieved from STN Database accession no. 2008:580903
- DATABASE CA [Online] CHEMICAL ABSTRACTS SERVICE, COLUMBUS, OHIO, US; TAYLOR, RICHARD J. K. ET AL: "The Ramberg-Backlund reaction", XP002759800, retrieved from STN Database accession no. 2008:1383616
- DATABASE CA [Online] CHEMICAL ABSTRACTS SERVICE, COLUMBUS, OHIO, US; HELLKAMP, SASCHA: "Synthesis of Tetracyclin through a Palladium-Catalyzed Domino Reaction", XP002759801, retrieved from STN Database accession no. 2014:1493839
- HAIGH, DAVID M. ET AL: "Nature of the Propagating Species in Ring-Opening Metathesis Polymerizations of Oxygen-Containing Monomers Using Well-Defined Ruthenium Initiators", MACROMOLECULES, vol. 38, no. 18, 2005, pages 7571-7579, XP002759802, ISSN: 0024-9297

## Description

### FIELD OF THE INVENTION

The present invention relates to novel carbene ligands and their incorporated ruthenium catalysts, which are highly active and selective for different kinds of metathesis reactions such as ROMP and RCM. The invention also relates to preparation of new ruthenium complexes and the use thereof in metathesis, especially effective for preparation of various functional polymers and rubbers.

### BACKGROUND OF THE INVENTION

Since Richard R. Schrock and Robert H. Grubbs prepared two kinds of metathesis catalysts with transition metal carbene structure in the 1990's, it has been drawning extensive attention in the development of more active and selective ruthenium catalysts for different kinds of olefin metathesis reactions, e.g., ring-opening metathesis polymerization (ROMP), ring-closing metathesis (RCM), and cross metathesis (CM).

So far, some useful ruthenium complexes have been reported as active metathesis catalysts (**1a-1b** and **2a-2f** in Scheme 1) for RCM and ROMP reactions (Grubbs et al., J. Am. Chem. Soc. 1992, 114, 3974-3975, Org. Lett. 1999, 1, 953-956, WO2007081987A1; Hoveyda et al., J. Am. Chem. Soc. 1999, 121, 791-799, J. Am. Chem. Soc. 2000, 122, 8168-8179; Yamaguchi et al., Chem. Commun. 1998, 1399-1400; Zhan et al., US20070043180A1, WO 2007003135A1; Grela et al., WO2004035596A1; Slugovc et al., Organometallics 2004, 23(15), 3623-3626 for catalyst **2d;** and Organometallics 2005, 24(10), 2255-2258 for catalyst **2e**). However, a disadvantage of all reported ruthenium catalysts is obviously substrate-dependent for different kinds of ruthenium catalysts in metathesis reactions, and it is still very difficult to find some active metathesis catalysts selective for RCM and ROMP reactions, respectively. Moreover, only a few metathesis catalysts could be used effectively to make high-strength and high-stiffness polydicyclopentadiene (PDCPD) material by ROMP reaction. 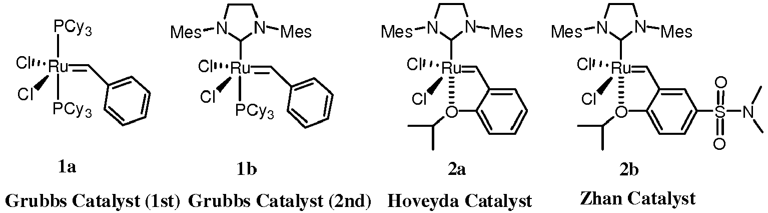 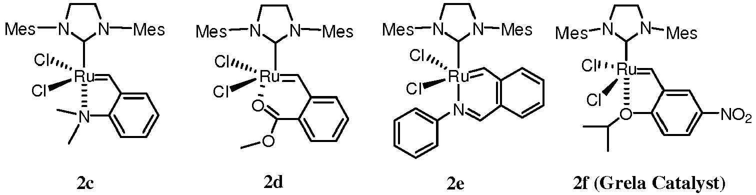 Scheme 1 Structure of Some Active Catalysts for ROMP and RCM Reaction

Currently, ROMP reaction is broadly used for preparation of various high-strength and other functional polymers. To overcome the activity and selectivity problems for ROMP catalysts, it has become a goal to develop more active and selective metathesis catalysts as an alternative for ROMP and RCM reactions, especially in ROMP for effective preparation and modification of different functional polymer materials. It is significantly important to develop more active and selective ruthenium catalyst for ROMP reactions with different kinds of olefin substrates to prepare highly functional polymer materials and also to improve polymer properties.

It was reported by David M. Haigh etc. (Nature of the Propagating Species in Ring-Opening Metathesis Polymerizations of Oxygen-Containing Monomers Using Well-defined Ruthenium Initiators, Macromolecules, 2005, 38, 7571-7579) that ROMP of a range of strained bicyclic monomers could be perfomred in the presence of RuCl₂(PCy₃)₂(=CHPh) and RuCl₂(PCy₃)(=CH-o-O-i-PrC₆H₄) so as to produce alkylidene species. Nevertheless, the Ru-containing complexes disclosed by Haigh are completely different from those of the present application in structure.

### SUMMARY OF THE INVENTION

The present invention relates to one class of novel ruthenium complex that can be used as highly active metathesis catalysts selective for RCM, CM, and ROMP reactions, respectively. The novel metathesis catalysts are ruthenium complexes with different kinds of new functionally substituted carbene ligands. The new ruthenium complexes of the invention can catalyze different kinds of metathesis reactions in a very effective manner and offer great advantage in activity and selectivity for different kinds of metathesis reactions, especially in ROMP effective for preparation of some functional polymer materials with unique chemical and physical properties. The novel ruthenium complexes of the invention have broad uses in the polymeric and pharmaceutical industries.

In the first aspect, the present invention provides a transition metal complex having the following structure **IIb**, 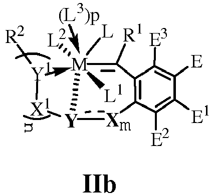 wherein:
m = 0 or 1, n = 1;
p = 0;
M is ruthenium;
L¹ and L² are the same or different and each selected from halogen anion (Cl⁻, Br⁻ or I⁻) anion;
L is an electron-donating ligand having the following structure **IIIa** or **IIId**: 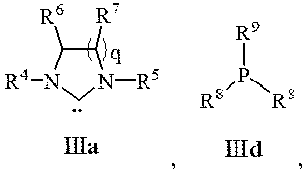
and in **IIIa**, q = 1, R⁴ and R⁵ each is 2,4,6-trimethylphenyl, R⁶ and R⁷ each is H; or in **IIId**, R⁸ and R⁹ each is cyclohexyl (Cy);
when m = 1, X is CH₂; Y is NH, C₁-C₂₀ alkylimino or or C₆-C₂₀ arylamino; "**Y⁻⁻⁻X**" is single bond;
when m = 0, Y is NH C₁-C₂₀ alkylimino, or C₆-C₂₀ arylamino;
When n = 1 and p = 0, X¹ and Y¹ are each oxygen, carbonyl, C₆-C₂₀ aryl or CH₂;
R¹ is H;
R² is H, or C₁-C₂₀ alkyl;
E is H, halogen, nitro, C₁-C₄ alkoxy, C₁-C₄ alkoxycarbonyl, or C₁-C₈ alkylaminosulfonyl;
E¹ and E² are each H, or halogen;
E³ is H;
E⁴ is H or C₁-C₄ alkyl;
E⁵ and E⁶ is H, halogen, C₁-C₄ alkyl or C₁-C₆ alkoxy;
E⁷ is H or C₁-C₄ alkyl.

In one preferable embodiment of the present application, R² is methyl, ethyl, or isopropyl.

In another one preferable embodiment of the present application, the transition metal complex is represented by any one of the following structures,

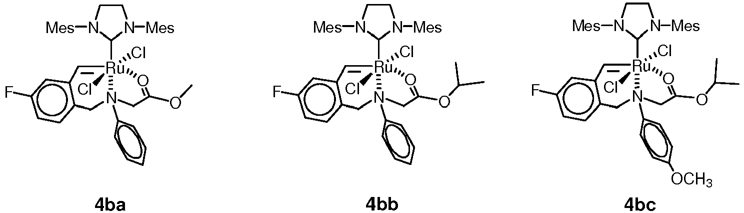

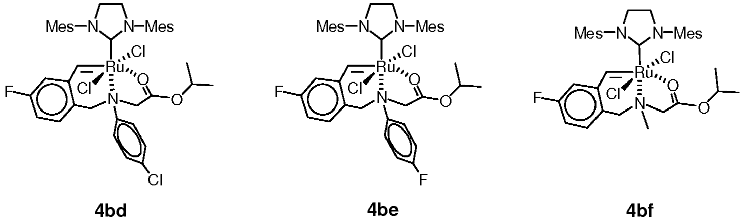

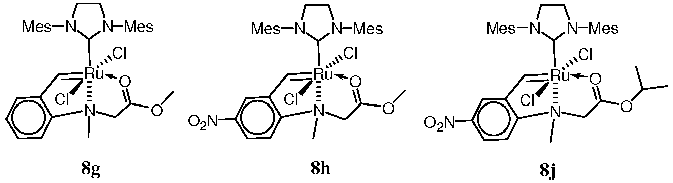

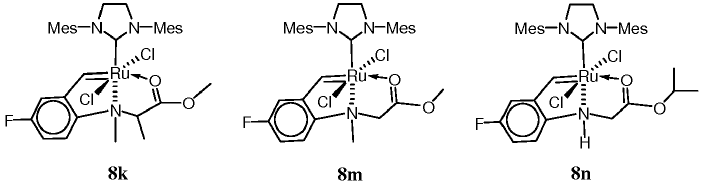

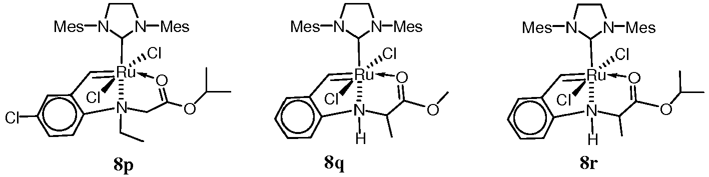

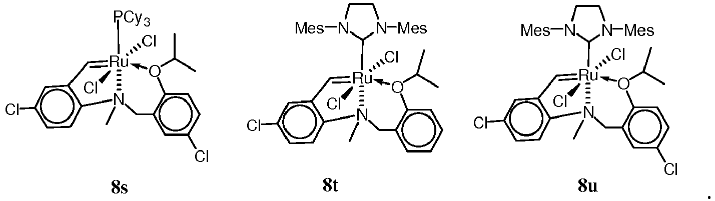

In the second aspect of the present application, the present application provides a method of carrying out a metathesis reaction with olefin substrate, comprising intramolecular ring-closing metathesis (RCM), intermolecular cross metathesis (CM), acyclic diene metathesis (ADMET) or ring-opening metathesis polymerization (ROMP) of cyclo-olefin substrate in the presence of one or more transition metal complexes of the present application.

In one preferable embodiment of the present application, the cyclo-olefin substrate for ROMP is selected from dicyclopentadiene (DCPD), norbornene, cyclooctene, or a kind of tensional cycloolefin; each is optionally substituted or unsubstituted with one or more of F, Cl, Br, C₁-C₁₅ alkyl, C₁-C₁₅ alkoxy, C₁-C₁₅ alkylthio, C₂-C₁₅ alkenyloxy, C₁-C₁₅ silanyl, C₁-C₁₅ alkylsilyloxy, C₆-C₁₅ aryl, C₆-C₁₅ aryloxy, C₁-C₁₅ alkylcarbonyl, C₆-C₁₅ arylcarbonyl, C₁-C₁₅ alkoxycarbonyl, C₆-C₁₅ aryloxycarbonyl, C₁-C₁₅ alkylaminocarbonyl, C₆-C₁₅ arylaminocarbonyl, C₁-C₁₅ alkylamido, C₆-C₁₅ arylamido, C₁-C₁₅ alkylaminosulfonyl, C₆-C₁₅ arylaminosulfonyl, C₁-C₁₅ sulfonylamido, C₃-C₁₅ heteroaryl or C₂-C₁₅ heterocyclic group.

In another one preferable embodiment of the present application, the tensional cycloolefin is a cycloolefin substrate having the following structure **VIa-VIc**; 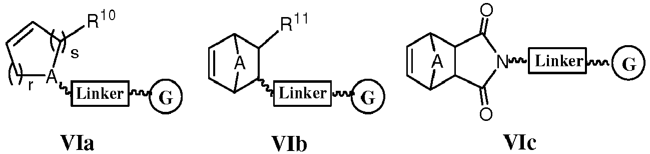
Wherein, r = 1, 2, 3 or 4; s = 1, 2, 3 or 4;
**A** is O, S, C₁-C₁₅ alkyl, C₁-C₁₅ alkoxy, C₁-C₁₅ aryloxy, C₁-C₁₅ alkylthio, C₁-C₁₅ alkoxycarbonyl, C₁-C₁₅ alkylamino, C₆-C₁₅ arylamino, C₁-C₁₅ alkylaminocarbonyl, C₆-C₁₅ arylaminocarbonyl, C₁-C₁₅ alkylamido, C₆-C₁₅ arylamido, or C₁-C₁₅ heterocyclic amido group;
**G** is a group of compounds with specific properties and uses; each is optionally selected from commercial drugs or liquid crystal monomers;
R¹⁰ and R¹¹ are each H, halogen, C₁-C₁₅ alkyl, C₁-C₁₅ alkoxy, C₁-C₁₅ alkylthio, C₁-C₁₅ alkylsilyoxy, C₆-C₁₅ aryloxy, C₆-C₁₅ aryl, C₂-C₁₅ heterocyclic, C₃-C₁₅ heterocyclic aryl, C₁-C₁₅ alkylcarbonyl, C₁-C₁₅ alkyloxycarbonyl, C₆-C₁₅ aryloxycarbonyl, C₁-C₁₅ alkylaminocarbonyl, C₆-C₁₅ arylaminocarbonyl, C₁-C₁₅ alkylamido, C₁-C₁₅ alkylsulfonyl, C₁-C₁₅ alkylsulfonamido, liquid crystal monomer or modified pro-drug;
"Linker" is C₁-C₁₅ alkyl, C₁-C₁₅ alkoxy, C₁-C₁₅ alkylthio, C₁-C₁₅ alkylsilyoxy, C₆-C₁₅ aryloxy, C₆-C₁₅ aryl, C₁-C₁₅ alkoxycarbonyl, C₆-C₁₅ aryloxycarbonyl, C₁-C₁₅ alkylaminocarbonyl, C₆-C₁₅ arylaminocarbonyl, C₁-C₁₅ alkylamido, C₆-C₁₅ arylamido, C₁-C₁₅ alkylsulfonamido, C₆-C₁₅ arylsulfonamido, C₃-C₁₅ heteroaryl or C₂-C₁₅ heterocyclic group.

In another one preferable embodiment of the present application, r =1, 2, 3 or 4; s = 1, 2, 3 or 4;
**A** is O, S, C₁-C₈ alkyl, C₁-C₈ alkoxy, C₁-C₈ aryloxy, C₁-C₁₅ alkylthio, C₁-C₈ alkoxycarbonyl, C₁-C₈ alkylamino, C₆-C₁₂ arylamino, C₁-C₈ alkylaminocarbonyl, C₆-C₁₂ arylaminocarbonyl, C₁-C₈ alkylamido, C₆-C₁₂ arylamido, or C₁-C₈ heterocyclic amido group;
**G** is a kind of compounds with specific properties and uses; each is optionally selected from commercial liquid crystal monomers or modified prodrugs;
R¹⁰ and R¹¹ are each H, halogen, C₁-C₈ alkyl, C₁-C₈ alkoxy, C₁-C₈ alkylthio, C₁-C₈ alkylsilyoxy, C₆-C₁₂ aryloxy, C₆-C₁₂ aryl, C₂-C₈ heterocyclic, C₃-C₁₂ heterocyclic aryl, C₁-C₈ alkylcarbonyl, C₁-C₈ alkyloxycarbonyl, C₆-C₁₂ aryloxycarbonyl, C₁-C₈ alkylaminocarbonyl, C₆-C₁₂ arylaminocarbonyl, C₁-C₈ alkylamido, C₁-C₈ alkylsulfonyl, C₁-C₈ alkylsulfonamido, liquid crystal monomer or modified pro-drug;
"Linker" is C₁-C₈ alkyl, C₁-C₈ alkoxy, C₁-C₈ alkylthio, C₁-C₈ alkylsilyoxy, C₆-C₁₂ aryloxy, C₆-C₁₂ aryl, C₁-C₈ alkoxycarbonyl, C₆-C₁₂ aryloxycarbonyl, C₁-C₈ alkylaminocarbonyl, C₆-C₁₂ arylaminocarbonyl, C₁-C₈ alkylamido, C₆-C₁₂ arylamido, C₁-C₈ alkylsulfonamido, C₆-C₁₂ arylsulfonamido, C₃-C₁₂ heteroaryl or C₂-C₈ heterocyclic group.

In another one preferable embodiment of the present application, in structure **VIa-VIc**, r = 1 or 2, and s = 1 or 2;
**A** is O, CH₂, C₁-C₅ alkyl-amino, C₁-C₅ alkoxy, C₁-C₅ alkylaminocarbonyl or C₁-C₅ heterocyclic amido group;
"Linker" is C₁-C₆ alkyl, C₁-C₅ alkoxy, C₁-C₅ alkylthio, C₁-C₅ alkoxycarbonyl, C₁-C₅ alkylaminocarbonyl, C₆-C₁₂ arylaminocarbonyl, C₁-C₅ alkylamido or C₆-C₁₂ arylamido group;
R¹⁰ and R¹¹ are each H, C₁-C₅ alkoxy, C₆-C₁₂ aryloxy, C₁-C₅ alkoxycarbonyl, C₆-C₁₂ aryloxycarbonyl, C₁-C₅ alkylaminocarbonyl, C₆-C₁₂ arylaminocarbonyl, C₁-C₅ alkylamido, C₆-C₁₂ arylamido, liquid crystal monomer or modified prodrugs.

In another one preferable embodiment of the present application, **G** is a kind of optionally modified prodrug of commercial drug Lipitor having the following structure **VIIa-VIId**: 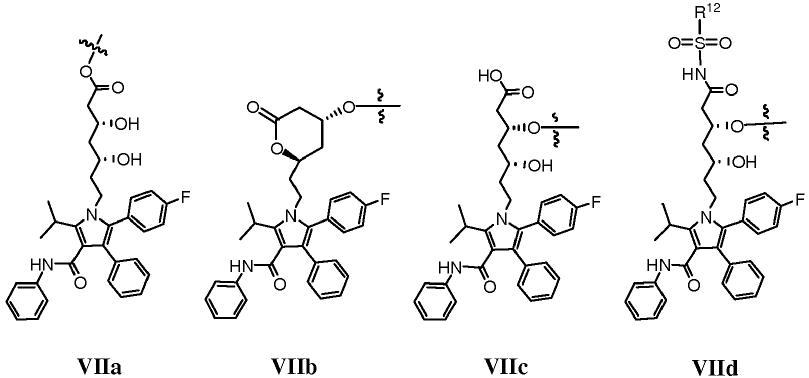
wherein R¹² is cyclopropyl, C₁-C₁₅ alkly, C₃-C₁₅ cycloalkyl, C₁-C₁₅ alkoxy, C₆-C₁₅ aryl, C₆-C₁₅ aryloxy, C₁-C₁₅ alkylamino, C₆-C₁₅ arylamino, C₁-C₁₅ alkylsulfonamido, C₆-C₁₅ arylsulfonamido, C₃-C₁₅ heterocyclic aryl or C₂-C₁₅ heterocyclic group.

In another one preferable embodiment of the present application, R¹² is cyclopropyl, C₁-C₆ alkly, C₃-C₆ cycloalkyl, C₁-C₆ alkoxy, C₆-C₁₂ aryl, C₆-C₁₂ aryloxy, C₁-C₆ alkylamino, C₆-C₁₂ arylamino, C₁-C₆ alkylsulfonamido, C₆-C₁₂ arylsulfonamido, C₃-C₁₂ heterocyclic aryl or C₂-C₆ heterocyclic group.

The present application also provides a method of making a modified nitrile butadiene rubber (NBR) or styrene-butadiene rubber (SBR) by depolymerization in the presence of one or more transition metal complexes of the present application at 30-100°C.

The present application also provides a method of making a depolymerized HNBR (hydrogenated nitrile butadiene rubber) or styrene-butadiene rubber (SBR) by adding one or more transition metal complexes of the present application first to carry out depolymerization of NBR, followed by adding hydrogen into the reaction under high pressure for hydrogenation at 60-150°C.

The present application also provides a method of making a hydrogenated nitrile butadiene rubber (HNBR) or styrene-butadiene rubber by adding hydrogen under high pressure first, followed by adding one or more transition metal complexes of the present application at 60-150°C.

The present application also provides a use of transition metal complexes of the present application in depolymerization of a rubber comprising at least one carbon-carbon double bond.

The present application also provides a use of transition metal complexes of the present application in hydrogenation of a rubber comprising at least one carbon-carbon double bond.The third aspect, the present invention provides the following synthetic methods of making different kinds of transition metal complexes **IIb**.

First of all, in the present invention, when Z is CH₂, the complex ligands **Ib** could be prepared by the following Suzuki reaction:

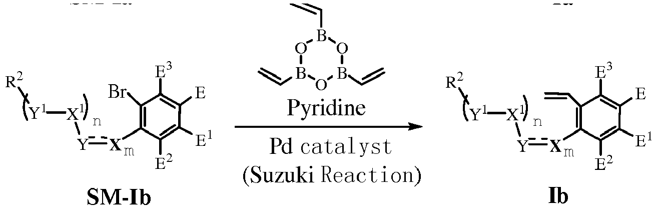

Wherein Y, Y¹, R¹, R², E, E¹, E² and E³ each is as defined above.

The ligands **Ib** can be prepared by the coupling of chemical SM-Ib with vinyl borane reagent in organic solvent such as DMF in the prence of Pd catalyst. SM-Ib was ordered by custom synthesis from Zannan Pharma Ltd, in China.

Method **1** in the following Scheme 1:

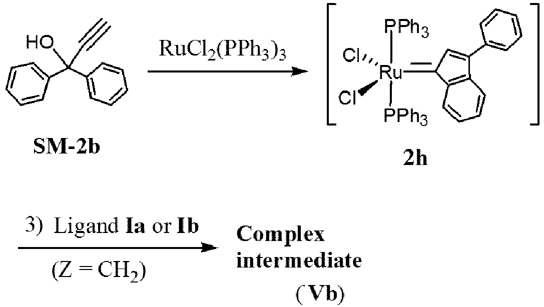

The intermediate of transition metal complex (**Vb**) having the following structure: 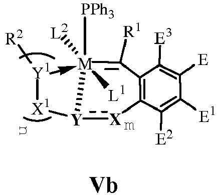
(1) The Ru complex **2h** is prepared by the reaction of reagent **SM-2b** and RuCl₂(PPh₃)₃ in anhydrous DCM in a three-neck flask filled with inert gas (Ar).
(2) The Ru complex **2h** obtained by step (1) is reacted with complex ligand **Ib** to prepare another Ru complex **Vb** in a flask filled with inert gas (Ar); wherein, **Vb** are compounds **IIb** when L is PPh₃; M, L¹, L², Y, Y¹, R¹, R², E, E¹, E² and E³ are each as defined above.

More preferred, L¹ and L² each is chloride anion (Cl⁻).

Wherein, in step (1), the preferred one of E and X¹ is hydrogen; the preferred usage of anhydrous organic solvent is 5-30 times weight of **SM-2;** the more preferred usage is 15 times; the preferred reaction temperature is 25-75°C, the more preferred temperature is 50-65 °C.

In step (2), the preferred reaction temperature is -50°C to -85°C, the more preferred temperature is -60°C to -75°C; the preferred usage of ML¹L²L₃ is 0.3-1.0 times molar ratio of **SM-2,** the more preferred usage is 0.6-0.7 times; the preferred compound of ML¹L²L₃ is RuCl₂(PPh₃)₃;

In step (3) of method 1, the preferred reaction temperature is -50°C to -85°C, the more preferred temperature is -60°C to -75°C; the preferred usage of complex ligand **Ib** is 1-3 molar ratio of complex intermediate, the preferred usage is 1.5-2 eq.

When ML¹L²L is RuCl₂(PPh₃)₃, the structure of product **Vb** is as follows:

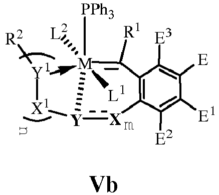

Method **2:** the complex **Vb** obtained by method 1 is reacted respectively with any electron-donating complex ligand L except PPh₃ to prepare following metal complexes **IIb**, wherein, p = 0, q = 1, definition of M, L, L¹, L², Y, Y¹, R¹, R², E, E¹, E² and E³ each is as defined above;

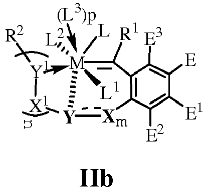

Wherein, the preferred one, in structure of transition metal complexs as the product **IIb**, where a preferred ligand L is **IIIa** or **IIId**. The preferred reaction termperature is 20°C to 75°C, the more preferred reaction temperature reacted with complex ligand IIIa is 60°C to 75°C, the more preferred reaction temperature reacted with complex ligand IIId is 20°C to 35°C; The preferred usage of **IIIa** or **IIId** is 1-3 times molar ratio of complex intermediate **Vb,** the more preferred molar ratio is 1.5-2 eq;

Method **3:** when L is PCy₃ or PPh₃, the **IIb** is reacted respectively with any electron-donating complex ligand L (**IIIa**) or L³ to prepare the metal complex **IIb**, wherein p = 0, M, L¹, L², Y, Y¹, R¹, R², E, E¹, E², E³ is each as defined above.

Method **4:** when L is PCy₃ or **IIIa**, the **IIb** is reacted respectively with any electron-donating complex ligand L³ to prepare the metal complex **IIb**, wherein p = 1, M, L¹, L², Y, Y¹, R¹, R², E, E¹, E², E³ is each as defined above. In method **4,** the preferred reaction temperature is 20°C to 35°C.

From method **1** to method **4**, L¹ and L² each is chloride anion.

Based on currently developed technology, the metal complex **IIb** of the present invention could also be prepared by the following two alternative procedures described in Schemes 2 & 3:

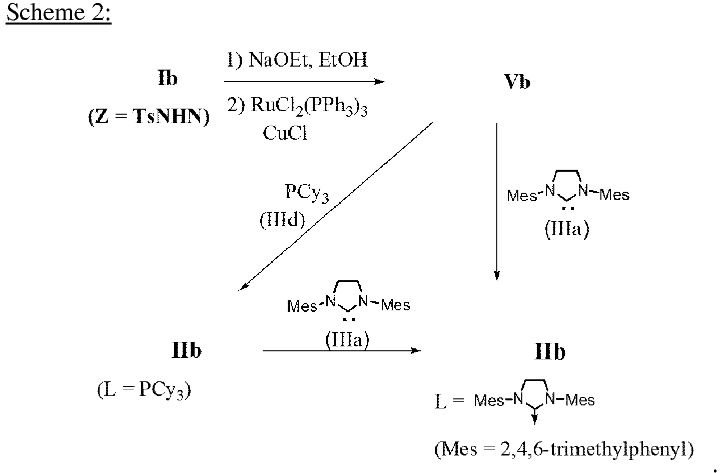

In the above procedure, Z is TsNHN in structure **Ib**.

In Scheme 2, **Ib** reacts with NaOEt in anhydrous EtOH to form carbene in a flask filled with inert gas, followed by reacting with RuCl₂P(Ph₃)₃ to form complex **Vb.** The complex **Vb** is reacted with **IIIa** or **IIId** effectively to obtain the complex **IIb** in inert gas, respectively.

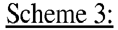

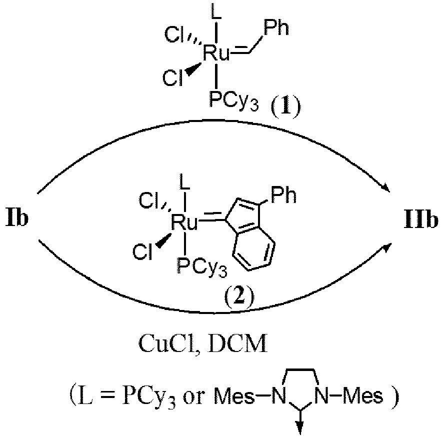

Complexes **IIb** could also be prepared by other two alternative synthetic routes as shown in Scheme 3.

In Scheme 3, **Ib** reacts with ruthenium complex **1** or **2** directly to form the desired complex **IIb** in a flask filled with inert gas (Ar), respectively.

Currently, the present invention provides the following significant achievements:
1. One class of novel carbene ligands and ruthenium complexes have been designed and prepared, which has different structure and activity with five or six coordinate bonds, especially for the new Ru complexes with six coordinate bonds to form at least one "Ru-N" coordinate bond from the new developed ligand **I**b. Moreover, the electronic and steric effect of different substituted ligands on the catalytic activity and stability of various new Ru complexes have been studied, and it is found that some of novel Ru catalysts in the present invention have much better catalytic selectivity and variable physical diversity than Grubbs and Hoveyda catalysts in the ROMP and RCM reactions.
2. The experimental results show that some of novel Ru catalysts in the present invention have high activity and selectivity for different olefin ROMP and RCM reactions, so the invention provides a useful synthetic method of carrying out olefin metathesis reactions effectively in preparation of polymer materials and pharmaceutical intermediates.
3. The present invention provides several developed methods for preparation of carbene ligands and Ru catalysts at lower cost, and it also provides some efficient methods for preparation of various functional polymer materials with different chemical and physical properties.
4. The present invention provides several developed processes of conducting ROMP reaction with one or two more mixed of novel active Ru catalysts for preparation of the high-strength polymer materials and some functional polymers linked with small molecule pro-drugs and/or liquid crystal materials.

### BRIEF DESCRIPTION OF THE DRAWINGS

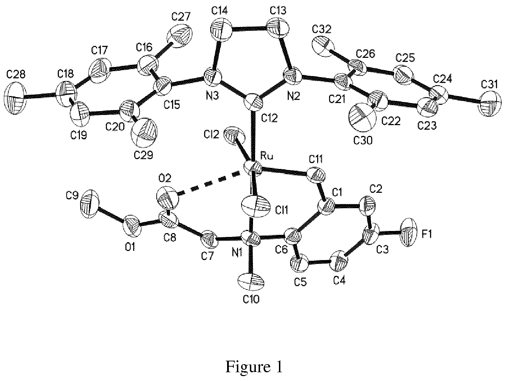

Figure 1: Single-crystal X-ray Structure of Ru Catalyst **8m.**

### DETAILED DESCRIPTION OF THE INVENTION

The present invention comprises two novel classes of carbene ligands and ruthenium complexes as catalysts for metathesis reactions. To study the electronic and steric effects of multi-substituted benzylidene ligands on the stability and activity of Ru complexes, based on the following reported procedure in Schemes 1-3, different kinds of the complex ligands (**3ba-3bf, 7a-7r**) have been prepared and reacted with the Ru complex **1** to obtain different kinds of new Ru complex (**4ba-4bf, 8a-8r**). During preparation and activity evaluation of various Ru complexes with various substituted 2-aminobenzylidene ligands in the following Schemes 4-8, different kinds of the electron-withdrawing and/or electron-donating effect and steric effect on the stability and selective activity for ROMP and RCM reactions have been found as shown in Schemes 9-16 and Tables 1-6.

**Significant electronic effect of various substituted benzylidene ligands on the stability of Ru complexes:** Based on different described synthetic methods and procedures in Schemes 1-3, there are different kinds of new olefin or carbene ligands (**Ib**) and Ru complexes (**IIb**) prepared in the present invention. Moreover, significant substituent effect of different substituted benzylidene ligands on the stability and activity of Ru complexes has been observed and developed selectively for ROMP and RCM reactions, and some novel Ru catalysts have been prepared much more active and selective than prior reported Ru catalysts for different kinds of ROMP and RCM reactions.

According to previously described synthetic methods, various new Ru complexes **4ba-4bf** have been prepared by the reaction listed in Scheme 4, and the corresponding metathesis activity of each Ru complex has been studied for RCM and ROMP reactions with different olefin substrates, respectively.

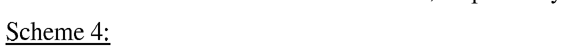

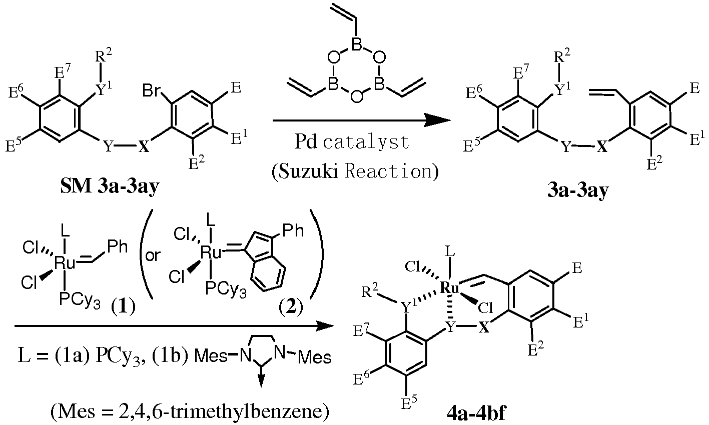

Some selected structure of prepared ligands **3ba-3bf** and corresponding ruthenium complexes **4ba-4bf** (**la**: Cy = cyclohexyl, **1b** = 2,4,6-trimethylbenzene) are listed as follows:

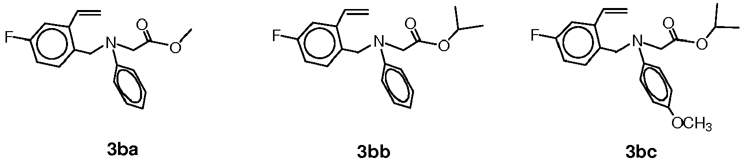

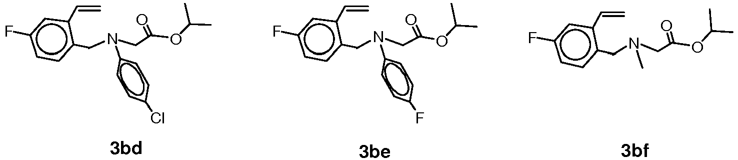

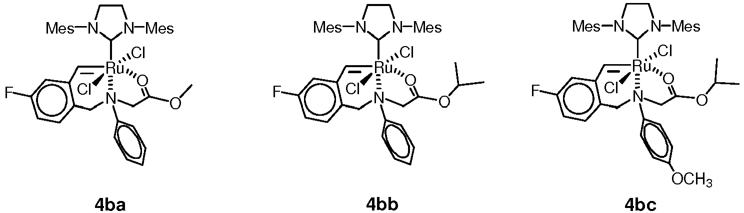

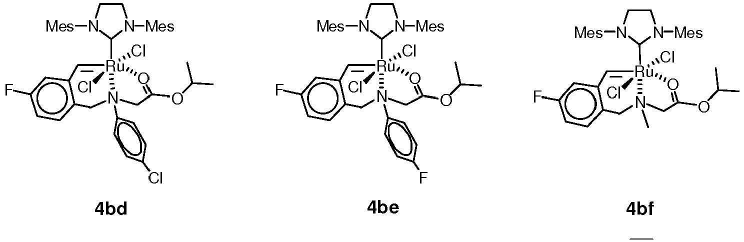

According to previously described synthetic methods, various new Ru complexes **8a-8r** have been prepared by the reaction listed in Scheme 6, and the corresponding metathesis activity of each Ru complex has been studied for RCM and ROMP reactions with different olefin substrates, respectively.

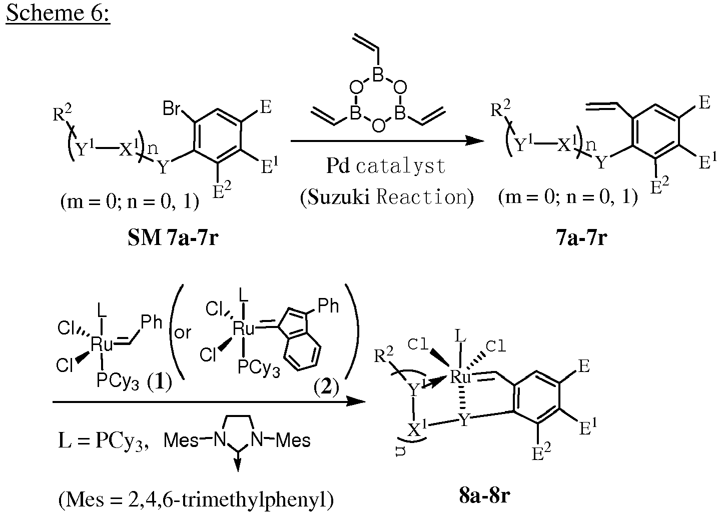

Some selected structure of prepared ligands **7a-7r** and corresponding ruthenium complexes **8a-8r** (**la:** Cy = cyclohexyl, **1b** = 2,4,6,- trimethylbenzene) are listed as follows:

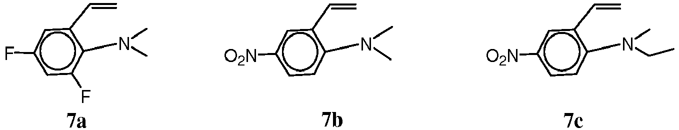

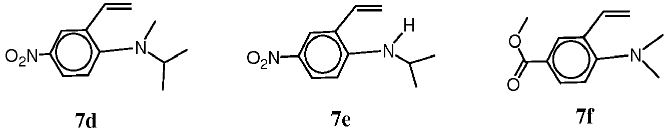

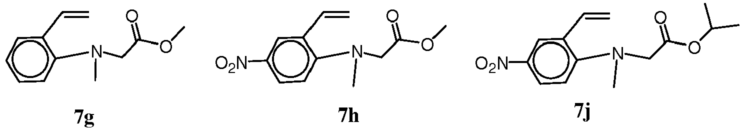

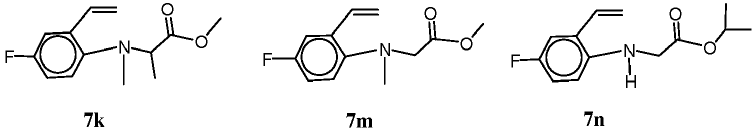

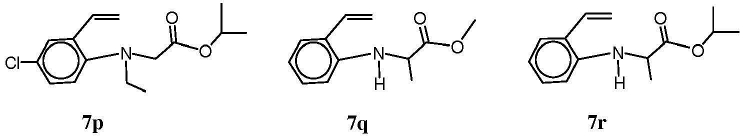

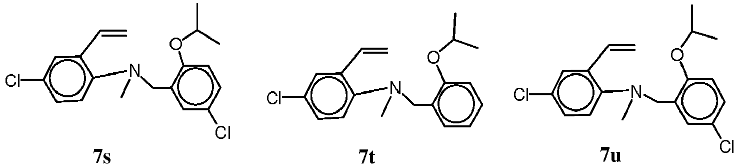

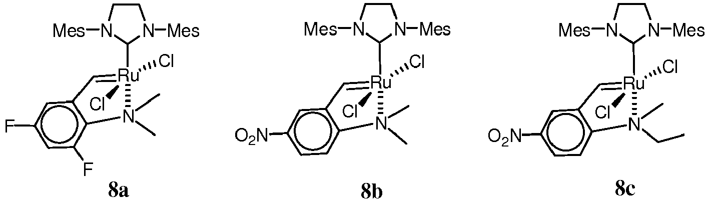

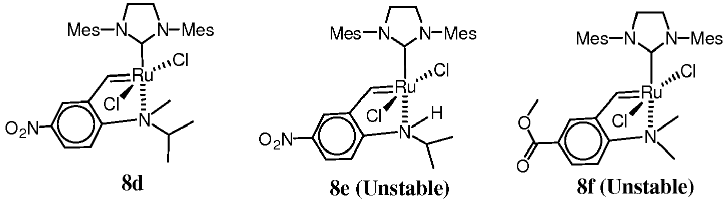

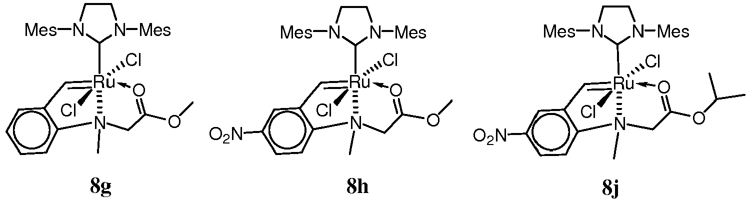

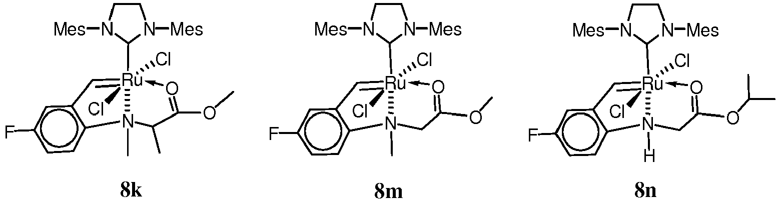

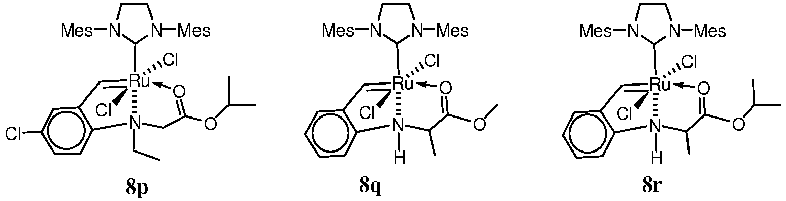

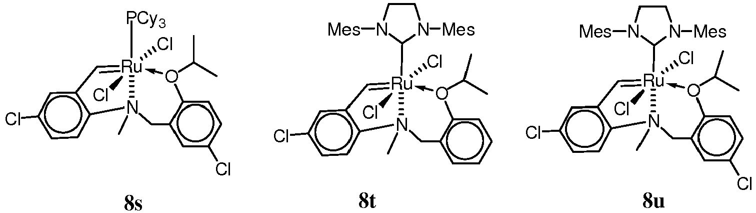

The structure of Ru catalyst **8m** is confirmed by single-crystal X-ray as shown in Figure 1.

So far, to study the relative activity and catalytic selectivity of above prepared catalysts **4ba-4bj, 8a-8u,** two olefin substrates **15** and **17** in Equations 1 and 2 were chosen for RCM reactions, and different kinds of cyclic olefin substrates **19, 21, 23, 25, 27, 29** and **31** in Equations 3-9 were selected for ROMP reactions, and the kinetic results of different conducted RCM and ROMP reactions for each new catalyst are listed in Tables 1, 2, 3, 4 and 5, respectively. Other eight prior known Ru catalysts **1a-1b and 2a-2f** listed in Scheme 1 are also selected for evaluation of metathesis activity study with various substrates **15, 17, 19, 21, 23, 25, 27, 29** and **31** in comparison to all new Ru catalysts in the present invention.

The evaluation of catalytic activity for RCM in Equation 1 with different catalysts **4a-4bj, 8a-8u** has been done under the same reaction condition, and the valuable experimental data for different Ru catalysts are selected and listed in Tables 1-1 to 1-4, respectively.

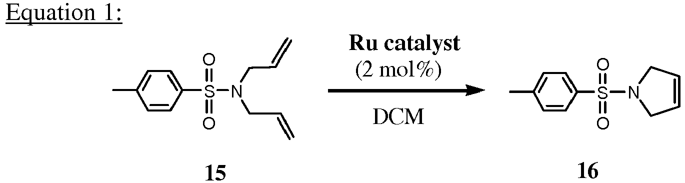

**Table 1-1: Activity Results of Some Selected Complexes 4ba-4bj for Substrate 15**

| Entry | Catalyst | Conversion (% by HPLC) | | | |
|---|---|---|---|---|---|
| | | 10 min | 30 min | 1.5 hr | 3.0 hr |
| 16 | **4ba** | 62 | 73 | 79 | 85 |
| 17 | **4bb** | 100 | | | |
| 18 | **4bc** | 94 | 100 | | |
| 19 | **4bd** | 68 | 81 | 84 | 89 |
| 20 | **4be** | 100 | | | |

Among Ru complexes **4ba-4bj,** only some of new complexes (such as **4bb** and **4be**) show high catalytic activity, the rest of them not listed in Table 1-1 have lower or very poor activities for RCM reaction. Based on the determined results in Table 1-1, the activity of Ru complexes **4ba-4bj** for RCM is significantly affected by the electronic and steric effect of different substituents incorporated in various new ligands **3ba-3bj.** However, some of complexes **4ba-4bj** non-active for RCM can be used effectively in the following ROMP (Equations 3-9) with high activity and selectivity.

**Table 1-3. Activity Results of Some Selected Complexes 8a-8u for Substrate 15**

| Entry | Catalyst | Conversion (% by HPLC) | | | |
|---|---|---|---|---|---|
| | | 10 min | 30 min | 1.5 hr | 3.0 hr |
| 1 | **8b** | 73 | 79 | 98 | |
| 2 | **8c** | 21 | 36 | 53 | 66 |
| 3 | **8g** | 0 | 0 | 0 | 0 |
| 4 | **8h** | 96 | 98 | 99 | |
| 5 | **8t** | 53 | 76 | 88 | 99 |
| 6 | **8u** | 79 | 93 | 100 | |
| 7 | **2c** | 47 | 69 | 82 | 92 |

Among complexes **8a-8u,** only a few complexes (such as **8b, 8h** and **8r**) show good activity and much better than the reported catalyst **2c,** the rest of them not listed in Table 1-3 have worse or very poor activities. Based on the determined results in Table 1-3, the activity of Ru complexes **8a-8u** for RCM is significantly affected by the electronic and steric effect of different substituents incorporated in various new ligands **7a-7u.** However, some of non-active complexes **8a-8u** for RCM can be used effectively in the following ROMP (Equations 3-9) with high activity and selectivity.

In order to find some new catalysts with better activity and selectivity, it is designed to carry out a RCM reaction with a phenyl-substituted diene substrate **17** as shown in Equation 2 instead of unsubstituted diene substrate **15** for further evaluation of some active catalysts selected from the catalysts **4ba-4bj, 8a-8u** according to activity results in Tables 1-1 to 1-5. The experimental results of RCM activity for substrate **17** are listed in Tables 2.

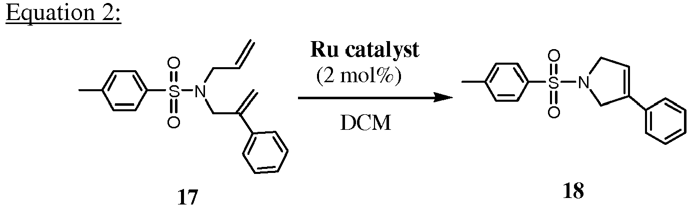

**Table 2: Activity Results of Some Selected Ru Complexes for Substrate 17**

| Entry | Catalyst | Conversion (% by HPLC) | | | |
|---|---|---|---|---|---|
| | | 10 min | 30 min | 1.5 hr | 3.0 hr |
| 8 | **4be** | 49 | 73 | 86 | 91 |
| 10 | **8h** | 75 | 88 | 96 | 99 |

To develop more effective ROMP catalysts and prepare better quality of new functional polymers, and also better to measure the difference of various active Ru catalysts, the evaluation of catalytic activity for different ROMP reactions in Equations 3-9 with different catalysts **4ba-4bj, 8a-8u** has been done under the same reaction condition, and some valuable results for different Ru catalysts are selected or listed in Tables 3 to 6, respectively. Based on the broad test, it is useful to find some active and selective catalysts for ROMP and RCM reactions, respectively.

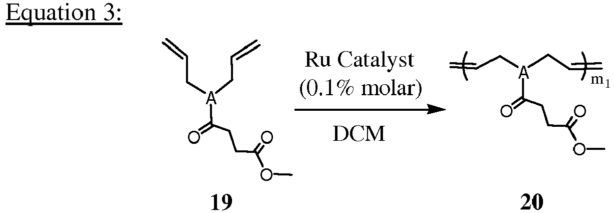

After screening with most of new Ru catalysts, it is found that some catalysts such as **8g** and **8m** could selectively catalyze the ROMP reaction effectively.

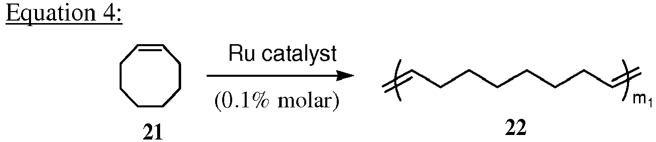

After screening with most of new Ru catalysts, it is found that some catalysts such as **8j** could catalyze the ROMP reaction effectively.

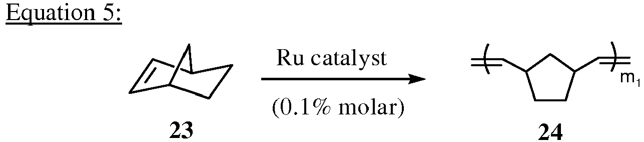

The ROMP results show that the catalysts **8b** and **8h** of the present invention have better activity and selectivity for norbornene (**23**) polymerization. Catalytic polymerization was completed in 10-60 min, and the polymer product (**24**) has better tensile-strength when it is prepared as film.

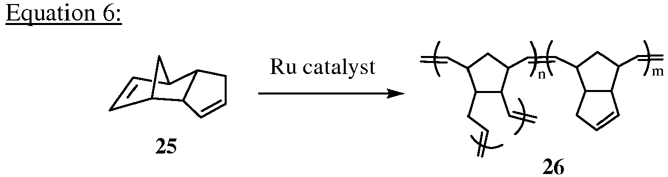

The ROMP results show that the catalysts **8a, 8b, 8c, 8h, 8m** and **8q** of the present invention have better activity and selectivity for **DCPD** (**25**) polymerization. The ROMP polymerization was completed in 5-60 min for different Ru catalysts. The reaction temperature is preferred to be 40-60 °C. By using one or two more mixed catalysts, it is surprised to obtain the high strength and high stiffness polymer PDCPD.

The property tests of various PDCPD (**26**) samples in the present invention show that several PDCPD products have more better tensile strength (55-62Mpa) and flexural strength (78-83Mpa) than those of commercial PDCPD products such as "Pentam, Metton, and Prometa" (tensile strength: 40-50Mpa, and flexural strength: 66-75Mpa) reported by other companies prepared with their own ROMP catalysts in Japan and USA, which advantage in the present invention will provide an alternative method of making high-quality of PDCPD material for broad uses in polymer industry.

The catalysts of the present invention can be used for depolymerization of a rubber comprising at least one carbon-carbon double bond. The depolymerization is conducted by metathesis reaction of carbon-carbon double bond in the rubber in the presence of one or more of catalysts of the present invention. The depolymerized rubber has lower molecular weight and lower mooney viscosity, which can be better used at lower temperature as lower as -40°C.

The catalysts of the present invention can be used in hydrogenation of a rubber comprising at least one carbon-carbon double bond. The carbon-carbon double bond in the rubber is hydrogenated under high pressure of hydrogen in the presence of one or more of catalysts of the present invention. The hydrogenated rubber is obtained and could be used as more stable and higher strength rubber.

The rubber comprising at least one carbon-carbon double bond can be depolymerized, and followed by hydrogenation under high pressure of hydrogen to produce a lower molecular weight and lower mooney viscosity rubber in the presence of one or more catalysts of the present invention, which can be used at lower temperature as lower as -55°C.

The rubber comprising at least one carbon-carbon double bond can be hydrogenated under high pressure of hydrogen and depolymerized simultaneously in the presence of one or more catalysts of the present invention, which can be used at lower temperature as lower as -55°C.

The representative examples of rubbers include but not limited to nitrile butadiene rubber, polybutadiene rubber, styrene-butadiene rubber (SBR), styrene-butadiene-styrene (SBS) or any rubber containing carbon-carbon double bond.

Based on the results from Equation 10 to Equation 12, it is determined that the molecular weight (Mw) and Mooney viscosity of various NBR brands (e.g., N41, DN3335, DN3350, and DN2850) are obviously reduced about 30-70% as needed by metathesis depolymerization and hydrogenation by adding hydrogen in chlorobenzene or chloroform in the presence of the Ru catalysts (e.g., **4ba-4bj, 8g-8u**) to obtain different kinds of HNBR products as needed with lower molecular weight (Mooney viscosity range: 20-100MU) and high hydrogenation degree (90-99.5%).

So far, it is found that most of the new developed Ru catalysts (**4ba-4bj, 8a-8u**) can be used to reduce molecular weight of the nitrile butadiene rubber (NBR) and butyl rubber by catalytical depolymerization. Furthermore, the quality-modified hydrogenated nitrile butadiene rubber (HNBR) with different molecular weight has been prepared by adding different new Ru catalyst and hydrogen (H₂) under high pressure (2.0-15Mpa) in some organic solvents such as chlorobenzene or chloroform solution. Just as mentioned above, the depolymerized NBR can be used in lower temperature as lower as -40°C, and the depolymerized and hydrogenated NBR (HNBR) can be used in a temperature as lower as -55°C with an improved strength and a better UV-resistance.

Based on our broad study, it is found that some of novel Ru catalysts (such as **4ba-4bj, 8g-8u)** have good activity for metathesis depolymerization to prepare different kinds of lower molecular NBR, followed by hydrogenation under high pressure of hydrogen (preferred between 4-9Mpa) to prepare high hydrogenation degree and various molecular weight of HNBR products.

Overall, based on the activity and selectivity studies in equations 1-10, it is found that some of novel Ru catalysts such as **4ab, 8g and 8h** have much better activity and selectivity than other tested and reported metathesis catalysts for the ROMP and RCM reactions, respectively. Moreover, it is found that the electronic effect of multi-substituted benzylidene ligands on the activity and selectivity of Ru complexes is one of the most important factors for the development of new active and selective metathesis catalysts for ROMP and RCM reactions. Based on the intensive study, the present invention provides some useful methods of carrying out ROMP, RCM, CM, and ADMET reactions with one or two more mixed of novel active Ru catalysts for preparation of some functional polymers, lower molecular weight rubbers and/or pharmaceutical intermediates, respectively.

### EXAMPLES

**General:** Infrared (IR) spectra were recorded on a Fourier Transform AVATAR™ 360 E.S.P™ spectrophotometer (Unit: cm⁻¹). Bands are characterized as broad (br), strong (s), medium (m), and weak (w). ¹H NMR spectra were recorded on a Varian-400 (400 MHz) spectrometer. Chemical shifts are reported in ppm from tetramethylsilane with the solvent resonance as the internal standard (CDCl₃: 7.26 ppm). Data are reported as follows: chemical shift, multiplicity (s = singlet, d = doublet, t = triplet, q = quartet, br = broad, m = multiplet), coupling constants (Hz), integration, and assignment. ¹⁹F and ³¹P NMR spectra were recorded on a Varian-400 (400 MHz) and Gemini-2000 (300MHz) spectrometers. The chemical shifts of the fluoro resonances were determined relative to trifluoroacetic acid as the external standard (CF₃CO₂H: 0.00 ppm), and the chemical shifts of the phosphorus resonances were determined relative to phosphoric acid as the external standard (H₃PO₄: 0.00 ppm). Mass spectra were obtained at Thermo Finnigan LCQ Advantage. Unless otherwise noted, all reactions were conducted in oven- (135°C) and flame-dried glassware with vacuum-line techniques under an inert atmosphere of dry Ar. THF and Et₂O were distilled from sodium metal dried flask, DCM, pentane, and hexanes were distilled from calcium hydride. Different substituted 2-alkoxystyrene ligands were prepared according to literature procedures as shown in Schemes 1-3. SM-Ia and SM-Ib chemicals were obtained from commercial sources or ordered by custom synthesis from Zannan Pharma Ltd., China. General procedures for preparation of different Ru complexes are described in examples 1 and 2, respectively. General procedures for evaluation of the RCM and ROMP reactions are described in examples 104-107, respectively.

### Example 1 (Not a part of the present application)

### Synthesis of Ru complex 4a

SM-**3a** (5.0mmol) was added into a 50 mL of three-neck round-bottom flask filled with inert gas (Ar), and followed by adding DME (10 mL) and deionized water (3 mL). K₂CO₃ (1.5 eq) was added and the solution was N₂ protected. The reaction was heated to 85°C, 2,4,6-Trivinyl-cyclotriboroxane pyridine complex (0.5 eq) and Pd(PPh₃)₄ (2%) were added until completed overnight. (monitored by TLC). The reaction mixture was filtered and extracted by DCM twice, then purified by flash column eluting with a gradient solvent (PE/EA 400/1 to 100/1) and dried under vacuum to obtain 0.9g of yellow oil products **3a** (yield: 86%). The product was confirmed by ¹HNMR.

Ligand **3a** ¹H-NMR (400 MHz, CDCl₃): δ 7.56 (d, *J* = 7.5 Hz, 1H, aromatic H), 7.37-7.18 (m, 5H, aromatic H, CH=CH₂), 7.02 (dd, *J* = 17.4 Hz, 10.8 Hz, 1H, CH=CH₂), 6.76-6.64 (m, 3H, aromatic H), 5.72 (d, *J* = 17.4 Hz, 1H, CH=CH₂), 5.34 (d, *J* = 10.8 Hz, 1H, CH=CH₂), 4.33 (s, 2H, NCH₂), 3.83 (s, 1H, NH).

(H₂IMes)(PCy₃)Cl₂Ru=CHPh (formula **1b**, 860mg, 1.0mmol) and CuCl (270 mg, 2.5mmol, 2.5 eq) were added into a 100 mL of two-neck round-bottom flask filled with inert gas (Ar), and followed by adding DCM (15 mL) and ligand **3a** (250 mg, 1.2mmol, 1.2 eq) into the DCM solution at 20-25°C. The reaction was stirred until completed in 30-60 min. (monitored by TLC). The reaction mixture was filtered and concentrated, then purified by flash column eluting with a gradient solvent (Pentane/DCM 2/1 to DCM). The purified solid product was washed with methanol, and dried under vacuum to obtain 27mg of green solid product **4a,** yield: 4%. The green product was confirmed by ¹HNMR.

Ru complex **(4a)** ¹HNMR (400 MHz, CDCl₃): δ 19.09 (s,1H, Ru=CH), 7.51-6.70 (m, 13H), 5.31 (m, 1H), 4.30 (d, *J* = 12.9 Hz, 1H), 4.04 (s, 4H, NCH₂CH₂N), 3.61 (d, *J* = 12.9 Hz, 1H), 2.45 (s, 12H), 2.33 (s, 6H).

### Example 2 (Not a part of the present application)

### Synthesis of Ru complex 4b

The synthetic procedure for preparation of ligand **3b** is the same as in Example 1 in 5.0 mmol scale. 1.15g of yellow oil product **3b** was obtained (yield: 91%).

Ligand **3b** ¹H-NMR (400 MHz, CDCl₃): δ 7.54 (d, *J* = 6.6 Hz, 1H), 7.27-7.23 (m, 3H), 7.02 (dd, *J* = 11.1 Hz, 17.0 Hz, 1H), 6.87-6.84 (m, 2H), 6.75-6.72 (m, 2H), 5.69 (dd, *J* = 1.5 Hz, 17.0 Hz, 1H), 5.35 (dd, *J* = 1.5 Hz, 11.1 Hz, 1H), 4.47 (s, 2H), 3.78 (s, 3H), 2.93 (s, 3H).

(PCy₃)₂Cl₂Ru=CHPh (formula **1a,** 830mg, 1.0mmol) and CuCl (270 mg, 2.5mmol, 2.5 eq) were added into a 100 mL of two-neck round-bottom flask filled with inert gas (Ar), and followed by adding DCM (15 mL) and ligand **3b** (250 mg, 1.2mmol, 1.2 eq) into the DCM solution at 20-25°C. The reaction was stirred until completed in 30-60 min. (monitored by TLC). The reaction mixture was filtered and concentrated, then purified by flash column eluting with a gradient solvent (Pentane/DCM 2/1 to DCM). The purified solid product was washed with methanol, and dried under vacuum to obtain 195mg of green solid product **4b,** yield: 29%. The green product was confirmed by ¹HNMR.

Ru complex **(4b)** ¹HNMR (400 MHz, CDCl₃): δ 19.31 (d, *J* = 8.4 Hz, Ru=CH), 7.57-7.50 (m, 4H), 7.31-7.29 (m, 1H), 7.15 (d, *J* = 5.6 Hz, 1H), 6.84-6.81 (m, 2H), 5.78 (d, *J* = 12.0 Hz, 1H), 3.71 (s, 3H), 3.62 (d, *J* = 12.0 Hz, 1H), 2.51 (s, 3H), 2.22-1.13 (m, 33H, PCy₃).

### Example 3 (Not a part of the present application)

### Synthesis of Ru complex 4c

The synthetic procedure for preparation of ligand **3c** is the same as in Example 1 in 5.0 mmol scale. 0.66g of yellow oil product **3c** was obtained (yield: 54%).

Ligand **3c** ¹H-NMR (400 MHz, CDCl₃): δ 7.56 (d, *J* = 7.5 Hz, 1H, aromatic H), 7.34-7.26 (m, 3H, aromatic H, CH=CH₂), 7.13 (d, *J* = 9 Hz, 1H, CH=CH₂), 6.98 (dd, *J* = 17.4 Hz, 10.8 Hz, 1H, CH=CH₂), 6.56 (d, *J* = 9 Hz, 1H, CH=CH₂), 5.71 (dd, *J* = 17.4 Hz, 1.2 Hz, 1H, CH=CH₂), 5.35 (dd, *J* = 10.8 Hz, 1.2 Hz, 1H, CH=CH₂), 4.30 (s, 2H, NCH₂), 3.86 (s, 1H, NH).

The procedure for preparation of Ru complex **4c** is the same as in Example 1 in 1.0 mmol scale. 35 mg of green solid product **4c** was obtained (yield: 5%).

Ru complex **(4c)** ¹HNMR (400 MHz): δ 19.09 (s,1H, Ru=CH), 7.50-6.69 (m, 12H), 5.27 (m, 1H), 4.33 (d, *J* = 12.9 Hz, 1H), 4.04 (s, 4H, NCH₂CH₂N), 3.59 (d, *J* = 12.9 Hz, 1H), 2.45 (s, 12H), 2.37 (s, 6H).

### Example 4 (Not a part of the present application)

### Synthesis of Ru complex 4d

The synthetic procedure for preparation of ligand **3d** is the same as in Example 1 in 5.0 mmol scale. 0.74g of yellow oil product **3d** was obtained (yield: 62%).

Ligand **3d** ¹H-NMR (400 MHz, CDCl₃): δ 7.32-7.23 (m, 2H), 7.04-6.91 (m, 2H), 6.82 (dd, *J* = 2.0 Hz, 6.6 Hz, 2H), 6.62 (dd, *J* = 2.4 Hz, 6.6 Hz, 2H), 5.73 (d, *J* = 17.1 Hz, 1H), 5.39 (d, *J* = 11.1 Hz, 1H), 4.25 (s, 2H), 3.77 (s, 3H).

The procedure for preparation of Ru complex **4d** is the same as in Example 1 in 1.0 mmol scale. 231 mg of green solid product **4d** was obtained (yield: 32%).

Ru complex **(4d)** ¹HNMR (400 MHz, CDCl₃): δ 18.68 (s, Ru=CH), 7.23-6.65 (m, 10H), 6.36 (dd, *J* = 2.8, 9.6 Hz, 1H), 6.03 (d, *J* = 12.8 Hz, 1H), 4.14-3.90 (m, 4H, NCH₂CH₂N), 3.85 (s, 3H), 3.47 (d, *J* = 12.8 Hz, 1H), 2.89-1.62 (m, 18H).

### Example 5 (Not a part of the present application)

### Synthesis of Ru complex 4e

The structure of ligand **3e** is the same as **3d** for preparation of Ru complex **4e,** just another Ru complex reagent **la** was used instead of Ru reagent **1b**.

The procedure for preparation of Ru complex **4e** is the same as in Example 2 in 1.0 mmol scale. 243 mg of green solid product **4e** was obtained (35% yield).

Ru complex **(4e)** ¹HNMR (400 MHz, CDCl₃): δ 19.28 (d, *J* = 8.4 Hz, Ru=CH), 7.45 (d, *J* = 8.8 Hz, 2H), 7.31-7.16 (m, 3H), 6.83 (d, *J* = 8.8 Hz, 2H), 5.13 (t, *J* = 12.4 Hz, 1H), 7.96 (d, *J* = 12.4 Hz, 1H), 3.85 (d, *J* = 12.4 Hz, 1H), 3.80 (s, 3H), 2.28-1.24 (m, 33H, PCy₃).

### Example 6 (Not a part of the present application)

### Synthesis of Ru complex 4f

The synthetic procedure for preparation of ligand **3f** is the same as in Example 1 in 5.0 mmol scale. 0.79g of yellow oil product **3f** was obtained (yield: 63%).

Ligand **3f** ¹H-NMR (400 MHz, CDCl₃): δ 7.21 (m, 2H), 6.94 (m, 2H), 6.85 (m, 2H), 6.73 (m, 2H), 5.68 (dd, *J* = 1.2 Hz, 16.8 Hz, 1H), 5.38 (dd, *J* = 1.5 Hz, 11.4 Hz, 1H), 4.40 (s, 2H), 3.77 (s, 3H), 2.89 (s, 3H).

The procedure for preparation of Ru complex **4f** is the same as in Example 1 in 1.0 mmol scale. 103 mg of green solid product **4f** was obtained (yield: 14%).

Ru complex **(4f)** ¹HNMR (400 MHz, CDCl₃): δ 18.99 (s, Ru=CH), 7.48-7.44 (m, 1H), 7.19-6.86 (m, 7H), 6.72-6.66 (m, 1H), 5.29 (t, *J* = 13.2 Hz, 1H), 4.19-3.58 (m, 8H), 2.52-2.37 (m, 18H).

### Example 7 (Not a part of the present application)

### Synthesis of Ru complex 4g

The synthetic procedure for preparation of ligand **3g** is the same as in Example 1 in 5.0 mmol scale. 0.70g of yellow oil product **3g** was obtained (yield: 56%). The product **3g** is confirmed by LC-MS (M+H⁺): m/z calculated: 285.1, found: 285.1, and directly used for preparation of Ru complex **4g.**

The procedure for preparation of Ru complex **4g** is the same as in Example 1 in 1.0 mmol scale. 61 mg of green solid product **4g** was obtained (yield: 8%).

Ru complex **(4g)** ¹HNMR (400 MHz, CDCl₃): δ 19.11 (s,1H, Ru=CH), 8.36 (dd, *J* = 2.0, 8.0 Hz, 1H), 7.29-6.65 (m, 10H), 5.30 (t, *J* = 13.6 Hz, 1H), 4.23 (d, *J* = 13.2 Hz, 1H), 4.10 (s, 3H), 3.80 (s, 4H, NCH₂CH₂N), 3.69 (d, *J* = 13.2 Hz, 1H), 2.65-2.08 (m, 18H).

### Example 8 (Not a part of the present application)

### Synthesis of Ru complex 4h

The synthetic procedure for preparation of ligand **3h** is the same as in Example 1 in 5.0 mmol scale. 0.47g of yellow solid product **3h** was obtained (yield: 32%).

Ligand **3h** ¹H-NMR (400 MHz, CDCl₃): δ 7.32 (dd, *J* = 5.6 Hz, 8.0 Hz, 1H), 7.25 (dd, *J* = 2.8 Hz, 10.4 Hz, 1H), 7.00-6.92 (m, 2H), 6.34 (s, 2H), 5.72 (d, *J* = 17.2 Hz, 1H), 5.38 (d, *J* = 11.2 Hz, 1H), 4.23 (s, 2H), 3.68 (s, 3H), 2.24 (s, 6H).

The procedure for preparation of Ru complex **4h** is the same as in Example 1 in 1.0 mmol scale. 315 mg of green solid product **4h** was obtained (yield: 42%).

Ru complex **(4h)** ¹HNMR (400 MHz, CDCl₃): 19.02 (s, 1H, Ru=CH), 7.21-6.82 (m, 8H), 6.40 (dd, *J* = 9.6 Hz, 1.6 Hz), 5.21 (m, 1H), 4.06-4.00 (m, 5H), 3.70 (s, 3H), 3.54 (d, *J* = 13.2 Hz, 1H), 2.48-2.18 (m, 24H).

### Example 9 (Not a part of the present application)

### Synthesis of Ru complex 4j

The synthetic procedure for preparation of ligand **3j** is the same as in Example 1 in 5.0 mmol scale. 0.91g of yellow liquid product **3j** was obtained (yield: 93%).

Ligand **3j** ¹H-NMR (400 MHz, CDCl₃): δ 7.34-7.26 (m, 2H), 7.22-7.13 (m 2H), 6.98-6.95 (m, 2H), 6.81 (m, 1H), 6.70-6.68 (m, 1H), 5.73 (d, *J* = 17.2 Hz, 1H), 5.36 (d, *J* = 11.2 Hz, 1H), 4.32 (s, 2H), 2.81 (m, 1H), 1.24 (d, *J* = 6.8 Hz, 6H).

The procedure for preparation of Ru complex **4j** is the same as in Example 1 in 1.0 mmol scale. 353 mg of green solid product **4j** was obtained (yield: 48%).

Ru complex **(4j)** ¹H-NMR (400 MHz, CDCl₃): δ 18.88 (s,1H, Ru=CH), 7.57-6.44 (m, 11H), 5.36 (t, *J* = 13.2 Hz, 1H), 4.16-4.02 (m, 5H), 4.01 (d, *J* = 13.2 Hz, 1H), 2.75-2.00 (m, 19H), 1.01-0.90 (m, 6H).

### Example 10 (Not a part of the present application)

### Synthesis of Ru complex 4k

The synthetic procedure for preparation of ligand **3k** is the same as in Example 1 in 5.0 mmol scale. 0.57g of yellow oil product **3k** was obtained (yield: 83%).

Ligand **3k** ¹H-NMR (400 MHz, CDCl₃): δ = 7.26 Hz) : 7.317 (dd, 1H, *J* = 6 Hz,8.4 Hz), 7.256 (dd, *J* = 2.8 Hz, 10.4 Hz, 1H), 7.094-7.017 (m, 3H), 6.961 (td, *J* = 2.8 Hz, 8.8 Hz, 1H), 6.873 (t, 1H, *J* = 6.8 Hz), 5.735 (d, *J* = 17.2 Hz 1H,), 5.412 (d, *J* = 10.8 Hz, 1H), 4.133(s, 2H), 2.276(s, 6H).

The procedure for preparation of Ru complex **4k** is the same as in Example 1 in 1.0 mmol scale. 490 mg of green solid product **4k** was obtained (yield: 68%).

Ru complex **(4k)** ¹HNMR (400 MHz, CDCl₃): δ 18.90 (s, 1H, Ru=CH), 7.27-6.77 (m, 9H), 6.41 (d, *J* = 8.0 Hz, 1H), 5.43 (t, *J* = 13.2 Hz, 1H), 4.18-4.00 (m, 5H), 3.25 (d, J = 13.6 Hz, 1H), 2.76-1.27 (m, 24H).

### Example 11 (Not a part of the present application)

### Synthesis of Ru complex 4m

The synthetic procedure for preparation of ligand **3m** is the same as in Example 1 in 5.0 mmol scale. 0.76g of yellow oil product **3m** was obtained (yield: 49%). The product **3m** is confirmed by LC-MS (M+H⁺): m/z calculated: 311.2, found: 311.2, and directly used for preparation of the Ru complex **4m.**

The procedure for preparation of Ru complex **4m** is the same as in Example 1 in 1.0 mmol scale. 404 mg of green solid product **4m** was obtained (yield: 52%).

Ru complex **(4m)** ¹HNMR (400 MHz, CDCl₃): δ 18.95 (s,1H, Ru=CH), 7.43-6.36 (m, 10H), 4.00 (m, 6H), 2.67-2.06 (m, 20H), 0.90-0.83 (m, 12H).

### Example 12 (Not a part of the present application)

### Synthesis of Ru complex 4n

The synthetic procedure for preparation of ligand **3n** is the same as in Example 1 in 5.0 mmol scale. 0.63g of yellow oil product **3n** was obtained (yield: 45%).

Ligand **3n** ¹H-NMR (400 MHz, CDCl₃): δ 7.33 (dd, *J* = 6.8 Hz, 6.8 Hz, 1H), 7.26 (d, *J* = 11.6 Hz, 1H), 7.08 (dd, *J* = 10.8 Hz, 17.6 Hz, 1H), 6.69 (t, *J* = 8.4 Hz, 1H), 6.86 (s, 2H), 5.74 (d, *J* = 17.6 Hz, 1H), 5.42 (d, *J* = 10.8 Hz, 1H), 4.08 (s, 2H), 2.25 (s, 9H).

The procedure for preparation of Ru complex **4n** is the same as in Example 1 in 1.0 mmol scale. 470 mg of green solid product **4n** was obtained (yield: 64%).

Ru complex **(4n):** ¹H-NMR (400 MHz, CDCl₃): δ 18.88 (s,1H, Ru=CH), 7.25-6.36 (m, 9H), 5.40 (t, *J* = 13.2 Hz, 1H), 4.14-4.00 (m, 6H), 2.77-1.90 (m, 27H).

### Example 13 (Not a part of the present application)

### Synthesis of Ru complex 4p

The synthetic procedure for preparation of ligand **3p** is the same as in Example 1 in 5.0 mmol scale. 0.85g of yellow oil product **3p** was obtained (yield: 67%).

Ligand **3p** ¹H-NMR (400 MHz, CDCl₃): δ=7.26Hz):7.368 (dd, 1H, *J*=6.00 Hz, 8.40 Hz), 7.258-7.126 (m, 4H), 7.019-6.922 (m, 3H), 5.632 (dd, 1H, *J* = 1.20 Hz, 17.60 Hz), 5.287 (dd, 1H, *J* = 1.20 Hz, 11.20 Hz), 4.072 (s, 2H), 2.537 (s, 3H), 2.290 (s, 3H)

The procedure for preparation of Ru complex **4p** is the same as in Example 1 in 1.0 mmol scale. 184 mg of green solid product **4p** was obtained (yield: 26%).

Ru complex **(4p)** ¹HNMR (400 MHz, CDCl₃): δ 18.91 (s,1H, Ru=CH), 7.63-6.42 (m, 10H), 5.27 (t, *J* = 13.2 Hz, 1H), 4.13-4.01 (m, 5H), 3.44 (d, *J* = 13.2 Hz, 1H), 2.46-2.00 (m, 21H).

### Example 14 (Not a part of the present application)

### Synthesis of Ru complex 4q

The synthetic procedure for preparation of ligand **3q** is the same as in Example 1 in 5.0 mmol scale. 0.69g of yellow oil product **3q** was obtained (yield: 46%).

Ligand **3q** ¹H-NMR (400 MHz, CDCl₃): δ 7.21 (dd, *J* = 2.8 Hz, 10.0 Hz, 1H), 7.15 (dd, *J* = 5.6 Hz, 7.6 Hz, 1H), 6.97-6.88 (m, 2H), 6.39 (s, 2H), 5.68 (d, *J* = 17.2 Hz, 1H), 5.36 (dd, *J* = 0.8 Hz, 11.2 Hz, 1H), 4.40 (s, 2H), 3.67 (s, 3H), 2.87 (s, 3H), 2.24 (s, 6H).

The procedure for preparation of Ru complex **4q** is the same as in Example 1 in 1.0 mmol scale. 291 mg of green solid product **4q** was obtained (yield: 38%).

Ru complex **(4q)** ¹HNMR (400 MHz, CDCl₃): δ 18.75 (s,1H, Ru=CH), 7.26-6.21 (m, 9H), 4.05-3.85 (m, 5H), 3.72 (s, 3H), 3.34 (d, *J* = 13.2 Hz, 1H), 2.82-0.95 (m, 30H).

### Example 15 (Not a part of the present application)

### Synthesis of Ru complex 4r

The synthetic procedure for preparation of ligand **3r** is the same as in Example 1 in 5.0 mmol scale. 0.55g of yellow oil product **3r** was obtained (yield: 44%).

Ligand **3r** ¹H-NMR (400 MHz, CDCl₃): δ 7.33-7.25 (m, 2H), 7.00-6.93 (m, 2H), 6.84 (bd, *J* = 8.4 Hz, 2H), 6.55 (dd, *J* = 4.4 Hz, 9.6 Hz, 1H), 5.74 (d, *J* = 17.2 Hz, 1H), 5.40 (d, *J* = 11.2 Hz, 1H), 4.29 (s,2H), 3.46 (bs, 1H), 2.12 (s, 3H).

The procedure for preparation of Ru complex **4r** is the same as in Example 1 in 1.0 mmol scale. 101 mg of green solid product **4r** was obtained (yield: 14%).

Ru complex **(4r)** ¹HNMR (400 MHz, CDCl₃): δ 18.89 (s,1H, Ru=CH), 7.69-6.43 (m, 10H), 5.23 (dd, *J* = 13.2, 11.3 Hz, 1H), 4.16-3.94 (m, 5H), 3.46 (d, *J* = 11.3 Hz, 1H), 2.62-1.00 (m, 21H).

### Example 16 (Not a part of the present application)

### Synthesis of Ru complex 4s

The synthetic procedure for preparation of ligand **3s** is the same as in Example 1 in 5.0 mmol scale. 0.83g of yellow oil product **3s** was obtained (yield: 51%).

Ligand **3s** ¹H-NMR (400 MHz, CDCl₃): δ 7.30 (dd, *J* = 6.0 Hz, 8.5 Hz, 1H), 7.23 (dd, *J* = 3.0 Hz, 10.0 Hz, 1H), 6.70-6.90 (m, 2H), 6.79 (d, *J* = 8.5 Hz, 2H), 6.58 (d, *J* = 8.5 Hz, 2H), 5.70 (d, *J* = 18.0 Hz, 1H), 5.37 (d, *J* = 11.0 Hz, 1H), 4.23 (s, 2H), 3.88 (t, *J* = 6.5 Hz, 2H), 1.73 (m, 2H), 1.44 (m, 2H), 1.35-1.31 (m, 4H), 0.90 (t, *J* = 6.0 Hz, 3H).

The procedure for preparation of Ru complex **4s** is the same as in Example 1 in 1.0 mmol scale. 679 mg of green solid product **4s** was obtained (yield: 85%).

Ru complex **(4s)** ¹HNMR (400 MHz, CDCl₃): δ 18.68 (s,1H, Ru=CH), 7.28-6.42 (m, 10H), 6.37 (d, *J* = 8.5 Hz, 1H), 5.05 (m, 1H), 4.06-3.93 (m, 7H,), 3.57 (d, *J* = 12.8 Hz, 1H), 2.89-1.29 (m, 29H).

### Example 17 (Not a part of the present application)

### Synthesis of Ru complex 4t

The synthetic procedure for preparation of ligand **3t** is the same as in Example 1 in 5.0 mmol scale. 0.67g of yellow product **3t** was obtained (yield: 38%). The product **3t** is confirmed by LC-MS (M+H⁺): m/z calculated: 339.2, found: 339.2, and directly used for preparation of the Ru complex **4t.**

The procedure for preparation of Ru complex **4t** is the same as in Example 1 in 1.0 mmol scale. 185 mg of green solid product **4t** was obtained (yield: 23%).

Ru complex **(4t)** ¹HNMR (300 MHz, CDCl₃): δ 18.97 (s,1H, Ru=CH), 8.54-8.45 (m, 2H), 6.66-6.96 (m, 8H), 4.16-4.10 (m, 1H), 4.03 (s, 4H, NCH₂CH₂N), 2.63-1.75 (m, 22H), 0.92 (d, *J* = 7.6Hz), 0.83 (d, *J* = 7.6Hz).

### Example 18 (Not a part of the present application)

### Synthesis of Ru complex 4u

The synthetic procedure for preparation of ligand **3u** is the same as in Example 1 in 5.0 mmol scale. 0.39g of yellow oil product **3u** was obtained (yield: 28%).

Ligand **3u** ¹H-NMR (400 MHz, CDCl₃): δ 7.53 (d, *J* = 2.0 Hz, 1H), 7.39 (d, *J* = 8.4 Hz, 1H), 7.28-7.26 (m, 1H), 7.21-7.12 (m, 3H), 7.03 (dd, *J* = 10.8 Hz, 17.6Hz, 1H), 5.73 (d, *J* = 17.6 Hz, 1H), 5.43 (d, *J* = 10.8 Hz, 1H), 4.07 (s, 2H), 3.26 (m, 2H), 1.25 (d, *J* = 6.4 Hz, 12 H).

The procedure for preparation of Ru complex **4u** is the same as in Example 1 in 1.0 mmol scale. 254 mg of green solid product **4u** was obtained (yield: 32%).

Ru complex **(4u)** ¹HNMR (300 MHz, CDCl3): δ 19.03 (s,1H, Ru=CH), 7.48-6.63 (m, 10H), 5.53 (m, 1H), 4.81-4.78 (m, 1H), 4.00 (s, 4H, NCH₂CH₂N), 2.51-2.49 (m, 1H), 2.51-2.32 (m, 18H), 1.12 (d, *J* = 7.6Hz), 1.04 (d, *J* = 7.6Hz).

### Example 19 (Not a part of the present application)

### Synthesis of Ru complex 4v

The synthetic procedure for preparation of ligand **3v** is the same as in Example 1 in 5.0 mmol scale. 1.08g of yellow oil product **3v** was obtained (yield: 81%).

Ligand **3v** ¹H-NMR (400 MHz, CDCl₃): δ 7.56 (d, *J* = 7.2 Hz, 1H), 7.34 (dd, *J* = 1.6 Hz, 7.6 Hz, 1H), 7.30-7.26 (m, 2H), 7.03 (dd, *J* = 11.2 Hz, 17.2 Hz, 1H), 6.86-6.80 (m, 2H), 6.68-6.62 (m, 2H), 5.72 (dd, *J* = 1.2 Hz, 17.2 Hz, 1H), 5.33 (dd, *J* = 1.2 Hz, 11.2 Hz, 1H), 4.56 (m, 1H), 4.36 (s, 2H), 1.33 (d, *J* = 6 Hz, 6H).

The procedure for preparation of Ru complex **4v** is the same as in Example 1 in 1.0 mmol scale. 73 mg of green solid product **4v** was obtained (yield: 10%).

Ru complex **(4v)** ¹HNMR (400 MHz, CDCl₃): δ 18.97 (s, Ru=CH), 7.50-6.58 (m, 11H), 5.26-3.52 (m, 8H), 3.48-2.07 (m, 18H), 1.23 (d, *J* = 6.4 Hz, 6H).

### Example 20 (Not a part of the present application)

### Synthesis of Ru complex 4w

The structure of ligand **3w** is the same as **3v** for preparation of Ru complex **4w,** just another Ru complex reagent **1a** was used instead of Ru reagent **1b**.

The procedure for preparation of Ru complex **4w** is the same as in Example 2 in 1.0 mmol scale. 219 mg of green solid product **4w** was obtained (yield: 31%).

Ru complex **(4w)** ¹HNMR (400 MHz, CDCl₃): δ 19.56 (d, *J* = 9.9 Hz, Ru=CH), 8.20 (d, *J* = 8.1 Hz, 1H), 7.66-6.84 (m, 6H), 5.46 (d, *J* = 12 Hz, 1H), 5.22 (t, *J* = 6 Hz, 1H), 4.56 (m, 1H), 3.95 (d, *J* = 12.0 Hz, 1H), 2.34-0.87 (m, 39H, PCy₃).

### Example 21 (Not a part of the present application)

### Synthesis of Ru complex 4x

The synthetic procedure for preparation of ligand **3x** is the same as in Example 1 in 5.0 mmol scale. 0.96g of yellow oil product **3x** was obtained (yield: 76%).

Ligand **3x** ¹H-NMR (400 MHz, CDCl₃): δ 7.27 (dd, *J* = 4.5 Hz, 6.15 Hz, 1H), 7.21-7.17 (m, 1H), 6.95-6.88 (m, 2H), 6.82-6.75 (m, 2H), 6.64-6.60 (m, 1H), 6.55 (d, *J* = 5.7 Hz, 1H), 5.66 (d, *J* = 12.9 Hz, 1H), 5.32 (d, *J* = 8.1 Hz, 1H), 4.48 (m, 1H), 4.26 (s, 2H), 1.27 (d, *J* = 4.5 Hz, 6H).

The procedure for preparation of Ru complex **4x** is the same as in Example 2 in 1.0 mmol scale. 420 mg of green solid product **4x** was obtained (yield: 58%).

Ru complex **(4x)** ¹HNMR (400 MHz, CDCl₃): δ 19.55 (d, *J* = 9.9 Hz, Ru=CH), 8.14 (d, *J* = 8.1 Hz, 1H), 7.36-6.83 (m, 6H), 5.46 (d, *J* = 12.0 Hz, 1H), 5.13 (t, *J* = 6.0 Hz, 1H), 4.56 (m, 1H), 3.90 (d, *J* = 12.0 Hz, 1H), 2.30-1.25 (m, 39H, PCy₃).

### Example 22 (Not a part of the present application)

### Synthesis of Ru complex 4y

The synthetic procedure for preparation of ligand **3y** is the same as in Example 1 in 5.0 mmol scale. 0.58g of yellow oil product **3y** was obtained (yield: 47%).

Ligand **3y** ¹H-NMR (400 MHz, CDCl₃): δ 7.33 (dd, *J* =5.6 Hz, 8.4Hz, 1H), 7.25 (dd, *J* = 2.8 Hz, 10 Hz, 1H), 7.05-6.82 (m, 3H), 6.81 (dd, *J* = 1.6 Hz, 8 Hz, 1H), 6.74-6.69 (m, 1H), 6.62 (dd, *J* = 1.6 Hz, 8Hz, 1H), 5.57 (d, *J* = 17.6 Hz, 1H), 5.40 (d, *J* = 11.2 Hz, 1H), 4.31 (s, 2H), 3.84 (s, 3H).

The procedure for preparation of Ru complex **4y** is the same as in Example 1 in 1.0 mmol scale. 267 mg of green solid product **4y** was obtained (yield: 37%).

Ru complex **(4y):** ¹HNMR (400 MHz, CDCl₃): δ 18.83 (s, Ru=CH), 7.50-6.39 (m, 11H), 5.21 (t, *J* = 12.4 Hz, 1H), 4.69-3.46 (m, 9H), 2.62-2.08 (m, 18H).

### Example 23 (Not a part of the present application)

### Synthesis of Ru complex 4z

The structure of ligand **3z** is the same as **3y** for preparation of Ru complex **4z,** just another Ru complex intermediate **1a** was used instead of Ru intermediate **1b**.

The procedure for preparation of Ru complex **4z** is the same as in Example 2 in 1.0 mmol scale. 362 mg of green solid product **4z** was obtained (yield: 52%).

Ru complex **(4z)** ¹HNMR (400 MHz, CDCl₃): δ 19.35 (d, *J* = 9.9 Hz, Ru=CH), 8.11 (d, *J* = 8.1 Hz, 1H), 7.34-6.85 (m, 6H), 5.48 (d, *J* = 12.0 Hz, 1H), 5.27 (t, *J* = 6 Hz, 1H), 3.93 (d, *J* = 12.0 Hz, 1H), 3.88 (s, 3H), 2.33-1.24 (m, 33H, PCy₃).

### Example 24 (Not a part of the present application)

### Synthesis of Ru complex 4aa

The structure of ligand **3aa** is the same as **3x** for preparation of Ru complex **4aa,** just another Ru complex intermediate **1b** was used instead of Ru intermediate **1a.**

The procedure for preparation of Ru complex **4aa** is the same as in Example 1 in 1.0 mmol scale. 631 mg of green solid product **4aa** was obtained (yield: 84%).

Ru complex **(4aa)** ¹HNMR (400 MHz, CDCl₃): δ 18.89 (s, Ru=CH), 7.60-6.45 (m, 11H), 5.13-3.52 (m, 8H), 2.95-2.10 (m, 18H), 0.95 (d, *J* = 6.4 Hz, 6H)

### Example 25 (Not a part of the present application)

### Synthesis of Ru complex 4ab

The synthetic procedure for preparation of ligand **3ab** is the same as in Example 1 in 5.0 mmol scale. 0.32g of yellow oil product **3ab** was obtained (yield: 26%).

Ligand **3ab** ¹H-NMR (400 MHz, CDCl₃): δ 8.36 (d, *J* = 2.8 Hz, 1H), 8.05 (dd, *J*= 2.8 Hz, 8.4 Hz, 1H), 7.54 (d, *J* = 8.4 Hz, 1H), 7.01 (dd, *J* = 10.5 Hz, 17.1 Hz, 1H), 6.85-6.69 (m, 3H), 6.44 (d, *J* = 7.5 Hz, 1H), 5.86 (dd, *J* = 0.9 Hz, 17.1 Hz, 1H,), 5.53 (dd, *J* = 0.9Hz, 10.5 Hz, 1H), 4.46 (s, 2H), 3.87 (s, 3H)

The procedure for preparation of Ru complex **4ab** is the same as in Example 1 in 1.0 mmol scale. 300 mg of green solid product **4ab** was obtained (yield: 40%).

Ru complex **(4ab)** ¹HNMR (400 MHz, CDCl₃): δ 16.52 (s, Ru=CH), 7.58 (m, 1H), 7.09 (s, 4H), 6.93-6.60 (m, 6H), 4.52 (m, 1H), 4.35 (s, 2H), 4.18 (s, 4H, NCH₂CH₂N), 3.89 (s, 6H), 2.49 (s, 12H), 2.40 (s, 6H).

### Example 26 (Not a part of the present application)

### Synthesis of Ru complex 4ac

The synthetic procedure for preparation of ligand **3ac** is the same as in Example 1 in 5.0 mmol scale. 1.09g of yellow oil product **3ac** was obtained (yield: 91%).

Ligand **3ac** ¹H-NMR (400 MHz, CDCl₃): δ 7.49 (s, 1H, NH), 7.27 (d, *J* = 7.5 Hz, 1H, aromatic H), 7.09-7.00 (m, 2H, aromatic H, CH=CH₂), 6.88-6.63 (m, 5H, aromatic H), 5.75 (d, *J* = 17.4 Hz, 1H, CH=CH₂), 5.38 (d, *J* = 10.8 Hz, 1H, CH=CH₂), 4.28 (s, 2H, NCH₂), 3.81 (s, 6H, OCH₃).

The procedure for preparation of Ru complex **4ac** is the same as in Example 1 in 1.0 mmol scale. 367 mg of green solid product **4ac** was obtained (yield: 50%).

Ru complex **(4ac)** ¹HNMR (400 MHz, CDCl₃): δ 19.03 (s, Ru=CH), 8.38 (d, *J* = 2.0 Hz, 1H), 7.69 (d, *J* = 16.0 Hz, 1H), 7.44 (d, *J* = 7.6 Hz, 1H), 7.21-7.03 (m, 5H), 6.83-6.59 (m, 3H), 5.24 (t, *J* =12.0 Hz, 1H), 4.66 (d, *J* =12.0 Hz, 1H), 4.45 (m, 1H), 4.20-4.05 (m, 4H, NCH₂CH₂N), 3.62 (d, *J* =120 Hz, 1H), 2.69-2.03 (m, 18H), 1.18 (d, *J* = 5.6 Hz, 6H).

### Example 27 (Not a part of the present application)

### Synthesis of Ru complex 4ad

The synthetic procedure for preparation of ligand **3ad** is the same as in Example 1 in 5.0 mmol scale. 1.01g of yellow oil product **3ad** was obtained (yield: 79%).

Ligand **3ad** ¹H-NMR (400 MHz, CDCl₃): δ 7.48 (d, *J* = 2.1 Hz, 1H, aromatic H), 7.27-7.24 (m, 1H, aromatic H), 7.04 (dd, *J* = 18Hz, 10.8 Hz, 1H, CH=CH₂), 6.85-6.79 (m, 3H, aromatic H), 6.67-6.61 (m, 2H, aromatic H), 5.74 (dd, *J* = 18 Hz, 1.2 Hz, 1H, CH=CH₂), 5.28 (dd, *J* = 10.8 Hz, 1.2 Hz, 1H, CH=CH₂), 4.59-4.53 (m, 2H, OCH, NH), 4.29 (s, 2H, NCH₂), 3.86 (s, 3H, OCH₃), 1.37 (d, *J* = 6.4 Hz, 6H, OCH(CH₃)₂).

The procedure for preparation of Ru complex **4ad** is the same as in Example 1 in 1.0 mmol scale. 374 mg of green solid product **4ad** was obtained (yield: 49%).

Ru complex **(4ad)** ¹HNMR (400 MHz, CDCl₃): δ 16.52 (s, Ru=CH), 7.59 (m, 1H), 7.09 (s, 4H), 6.92-6.84 (m, 4H), 6.75-6.66 (m, 2H), 4.59 (m, 1H), 4.35 (s, 2H), 4.18 (s, 4H, NCH₂CH₂N), 3.89 (s, 3H), 2.49 (s, 12H), 2.40 (s, 6H, 18H), 0.93 (m, 6H).

### Example 28 (Not a part of the present application)

### Synthesis of Ru complex 4ae

The synthetic procedure for preparation of ligand **3ae** is the same as in Example 1 in 5.0 mmol scale. 0.32g of yellow oil product **3ae** was obtained (yield: 27%).

Ligand **3ae** ¹HNMR (400 MHz, CDCl₃): δ 8.36 (d, *J* = 2.4 Hz, 1H), 8.05 (dd, *J* = 2.4 Hz, 8.4 Hz, 1H), 7.54 (d, *J* = 8.4 Hz, 1H), 7.01 (dd, *J* = 10.8 Hz, 17.1 Hz, 1H), 6.84-6.75 (m, 2H), 6.71-6.65 (m, 2H), 6.42 (dd, *J* = 1.8 Hz, 7.8 Hz, 1H), 5.85 (dd, *J* = 0.9 Hz, 17.1 Hz, 1H), 5.53 (dd, *J* = 0.9 Hz, 10.8 Hz, 1H), 4.58 (m, 1H), 4.47 (s, 1H), 1.36 (d, *J* = 6.0 Hz, 6H).

The procedure for preparation of Ru complex **4ae** is the same as in Example 1 in 1.0 mmol scale. 389 mg of green solid product **4ae** was obtained (yield: 50%).

Ru complex **(4ae)** ¹HNMR (400 MHz, CDCl₃): δ 19.03 (s,1H, Ru=CH), 8.38 (d, *J* = 2.0 Hz, 1H), 7.69 (d, *J* = 16.0 Hz, 1H), 7.44 (d, *J* = 7.6 Hz, 1H), 7.21-7.03 (m, 5H), 6.83-6.59 (m, 3H), 5.24 (t, *J* =12.0 Hz, 1H), 4.66 (d, *J* =12.0 Hz, 1H), 4.45 (m, 1H), 4.20-4.05 (m, 4H, NCH₂CH₂N), 3.62 (d, *J* =12.0 Hz, 1H), 2.69-2.03 (m, 18H), 1.18 (d, *J* = 5.6 Hz, 6H).

### Example 29 (Not a part of the present application)

### Synthesis of Ru complex 4af

The synthetic procedure for preparation of ligand **3af** is the same as in Example 1 in 5.0 mmol scale. 0.76g of yellow oil product **3af** was obtained (yield: 65%).

Ligand **3af** ¹H-NMR (400 MHz, CDCl₃): δ 7.38-7.34 (m, 2H, aromatic H), 7.22-7.10 (m, 2H, aromatic H, CH=CH₂), 7.01-6.88 (m, 4H, aromatic H), 5.63 (d, *J* = 17.1 Hz, 1H, CH=CH₂), 5.29 (d, *J* = 10.8 Hz, 1H, CH=CH₂), 4.20 (s, 2H, NCH₂), 3.88 (s, 3H, OCH₃), 2.63 (s, 3H, NCH₃).

The procedure for preparation of Ru complex **4af** is the same as in Example 1 in 1.0 mmol scale. 111 mg of green solid product **4af** was obtained (yield: 15%).

Ru complex **(4af)** ¹HNMR (400 MHz, CDCl₃): δ 18.54 (s,1H, Ru=CH), 7.45 (d, *J* = 8.0 Hz, 1H), 7.24-7.19 (m, 4H), 7.06-6.96 (m, 6H), 6.14 (d, *J* = 13.2 Hz, 1H), 5.39 (d, *J* = 13.2 Hz, 1H), 4.07-3.77 (m, 7H), 3.52 (s, 3H), 2.65-2.30 (m, 18H).

### Example 30 (Not a part of the present application)

### Synthesis of Ru complex 4ag

The synthetic procedure for preparation of ligand **3ag** is the same as in Example 1 in 5.0 mmol scale. 0.84g of yellow oil product **3ag** was obtained (yield: 76%).

Ligand **3ag** ¹H-NMR (400 MHz, CDCl₃): δ = 7.26 Hz): 7.32 (dd, *J* = 5.70 Hz, 8.40 Hz, 1H), 7.24 (dd, *J* = 9.9 Hz, 2.4 Hz, 1H), 7.03-6.90 (m, 2H), 6.54-6.39 (m, 3H), 5.71 (dd, *J* = 1.2 Hz, 17.4 Hz, 1H), 5.37 (dd, *J* = 1.2 Hz, 10.8 Hz, 1H), 4.25 (s, 2H), 4.07 (bs, 1H), 3.81 (s, 3H), 3.76 (s, 3H).

The procedure for preparation of Ru complex **4ag** is the same as in Example 1 in 1.0 mmol scale. 302 mg of green solid product **4ag** was obtained (40% yield).

Ru complex **(4ag)** ¹HNMR (400 MHz, CDCl₃): δ 18.83 (s,1H, Ru=CH), 7.36-6.14 (m, 10H), 5.12 (t, *J* = 12.4 Hz, 1H), 4.50-3.42 (m, 12H), 2.62-2.05 (m, 18H).

### Example 31 (Not a part of the present application)

### Synthesis of Ru complex 4ah

The synthetic procedure for preparation of ligand **3ah** is the same as in Example 1 in 5.0 mmol scale. 0.46g of yellow oil product **3ah** was obtained (yield: 38%).

Ligand **3ah** ¹H-NMR (400 MHz, CDCl₃): δ 7.34-7.23 (m, 2H), 7.03-6.91 (m, 2H), 6.69(dd, *J* = 1.2 Hz, 8.10 Hz , 1H), 7.52-6.45 (m, 2H), 5.72 (d, *J* = 17.4 Hz, 1H), 5.38 (d, *J* = 11.4 Hz, 1H), 4.32 (bs, 1H), 4.28 (s, 2H), 2.27 (s, 3H).

The procedure for preparation of Ru complex **4ah** is the same as in Example 1 in 1.0 mmol scale. 376 mg of green solid product **4ah** was obtained (yield: 51%).

Ru complex **(4ah)** ¹HNMR (400 MHz, CDCl₃): δ 18.90 (s,1H, Ru=CH), 7.60-6.36 (m, 10H), 5.25 (t, *J* = 12.0 Hz, 1H), 4.78 (d, *J* = 12.0 Hz, 1H), 4.05 (s, 4H, NCH₂CH₂N), 3.53 (s, 3H), 3.43 (d, *J* = 12.0 Hz, 1H), 2.56-2.13 (m, 21H).

### Example 32 (Not a part of the present application)

### Synthesis of Ru complex 4aj

The synthetic procedure for preparation of ligand **3aj** is the same as in Example 1 in 5.0 mmol scale. 1.22g of yellow oil product **3aj** was obtained (yield: 90%).

Ligand **3aj** ¹H-NMR (400 MHz, CDCl₃): δ 7.35-7.21 (m, 2H), 7.03-6.77 (m, 4H), 6.71-6.58 (m, 2H), 5.71 (d, *J* = 17.7 Hz, 1H), 5.38 (d, *J* = 11.1 Hz, 1H), 4.31 (s, 2H), 4.06 (q, *J* = 11.1 Hz, 2H), 1.40 (t, *J* = 11.1Hz, 3H).

The procedure for preparation of Ru complex **4aj** is the same as in Example 2 in 1.0 mmol scale. 390 mg of green solid product **4aj** was obtained (yield: 55%).

Ru complex **(4aj)** ¹HNMR (400 MHz, CDCl₃): δ19.45 (d, J = 9.6 Hz, Ru=CH), 8.18 (d, *J* = 7.6 Hz, 1H), 7.40-7.33 (m, 2H), 7.21-7.11 (m, 2H), 6.95-6.88 (m, 2H), 5.52 (m, 1H), 5.23 (m, 1H), 4.16-3.94 (m, 3H), 2.36-0.81 (m, 36H, PCy₃).

### Example 33 (Not a part of the present application)

### Synthesis of Ru complex 4ak

The synthetic procedure for preparation of ligand **3ak** is the same as in Example 1 in 5.0 mmol scale. 0.65g of yellow oil product **3ak** was obtained (yield: 52%).

Ligand **3ak** ¹H-NMR (400 MHz, CDCl₃): δ 7.47 (dd, *J* = 6.0 Hz, 8.4 Hz, 1H), 7.21 (dd, *J* = 10.4 Hz, 2.4 Hz, 1H), 7.13 (dd, *J* = 11.2 Hz, 17.2 Hz, 1H), 6.97-6.92 (m, 3H), 6.79 (d, *J* = 8.4 Hz, 1H), 5.63 (d, *J* = 17.2 Hz, 1H), 5.28 (d, *J* = 11.2 Hz, 1H), 4.57 (m, 1H), 4.21 (s, 2H), 2.66 (s, 3H), 1.29-1.27 (m, 15H).

The procedure for preparation of Ru complex **4ak** is the same as in Example 1 in 1.0 mmol scale. 299 mg of green solid product **4ak** was obtained (yield: 37%).

Ru complex **(4ak)** ¹HNMR (400 MHz, CDCl₃): δ 19.08 (s,1H, Ru=CH), 7.97-6.33 (m, 10H), 5.08 (m, 2H), 4.34 (m, 1H), 4.02 (s, 4H, NCH₂CH₂N), 3.41 (m, 1H), 2.53-2.31 (m, 18H), 1.29 (s, 9H), 0.89-0.87 (m, 6H).

### Example 34 (Not a part of the present application)

### Synthesis of Ru complex 4am

The synthetic procedure for preparation of ligand **3am** is the same as in Example 1 in 5.0 mmol scale. 1.10g of yellow oil product **3am** was obtained (yield: 86%).

Ligand **3am** ¹H-NMR (400 MHz, CDCl₃): δ 7.32 (m, 1H), 7.26-7.21 (m, 1H), 7.00-6.93 (m, 2H), 6.52-6.42 (m, 3H), 5.71 (d, *J* = 17.4 Hz, 1H), 5.37 (d, *J* = 11.1 Hz, 1H), 4.50 (m, 1H), 4.38 (m, 1H), 4.26 (s, 2H), 1.31 (m, 12H).

The procedure for preparation of Ru complex **4am** is the same as in Example 1 in 1.0 mmol scale. 437 mg of green solid product **4am** was obtained (yield: 54%).

Ru complex **(4am)** ¹HNMR (400 MHz, CDCl₃): δ 18.85 (s,1H, Ru=CH), 7.26-6.07 (m, 10H), 5.04 (t, *J* = 13.2 Hz, 1H), 4.48 (m, 1H), 4.39-4.33 (m, 2H), 4.15-4.02 (m, 4H, NCH₂CH₂N), 3.65 (m, 1H), 2.66-2.05 (m, 18H), 1.55 (m, 6H), 1.38 (m, 6H).

### Example 35 (Not a part of the present application)

### Synthesis of Ru complex 4an

The synthetic procedure for preparation of ligand **3an** is the same as in Example 1 in 5.0 mmol scale. 0.66g of yellow oil product **3an** was obtained (yield: 41%).

Ligand **3an** ¹H-NMR (400 MHz, CDCl₃): δ 7.34-7.22 (m, 2H), 7.02-6.93 (m, 2H), 6.70 (d, *J* = 7.8 Hz, 1H), 6.48-6.43 (m, 1H), 5.71 (d*, J* = 17.4 Hz, 1H), 5.37 (d, *J* = 10.8 Hz, 1H), 4.46 (m, 1H), 4.40 (bs, 1H), 4.28 (s, 2H), 2.25 (s, 3H), 1.30 (d, *J* = 6.0 Hz, 6H).

The procedure for preparation of Ru complex **4an** is the same as in Example 1 in 1.0 mmol scale. 359 mg of green solid product **4an** was obtained (yield: 46%).

Ru complex **(4an)** ¹HNMR (400 MHz, CDCl₃): δ 18.98 (s,1H, Ru=CH), 7.66-6.39 (m, 10H), 5.17 (t, *J* = 13.2 Hz, 1H), 4.71 (d, *J* = 13.2 Hz, 1H), 4.36 (m, 1H), 4.06 (brs, 4H, NCH₂CH₂N), 3.42 (d, *J* = 13.2 Hz, 1H), 2.63-2.09 (m, 21H), 1.09 (m, 6H).

### Example 36 (Not a part of the present application)

### Synthesis of Ru complex 4ap

The synthetic procedure for preparation of ligand **3ap** is the same as in Example 1 in 5.0 mmol scale. 0.70g of yellow oil product **3ap** was obtained (yield: 57%).

Ligand **3ap** ¹H-NMR (400 MHz, CDCl₃): δ 7.33-7.25 (m, 2H), 7.04 (dd, *J* = 10.8 Hz, 17.2 Hz, 1H), 6.96-6.92 (m, 1H), 6.79 (d, *J* = 2.4 Hz, 1H), 6.54 (d, *J* = 2.4 Hz, 1H), 5.72 (d, *J* = 17.2 Hz, 1H), 5.37(d, *J* = 10.8 Hz, 1H), 4.55 (m, 1H), 4.23 (s, 2H), 3.99 (bs, 1H), 1.40 (s, 9H), 1.29 (s, 9H), 1.20 (d, *J* = 6.0 Hz, 6H).

The procedure for preparation of Ru complex **4ap** is the same as in Example 1 in 1.0 mmol scale. 380 mg of green solid product **4ap** was obtained (yield: 44%).

Ru complex **(4ap)** ¹HNMR (400 MHz, CDCl₃): δ 18.99 (s,1H, Ru=CH), 7.45-6.36 (m, 9H), 5.05 (m, 2H), 3.98-3.91 (m, 5H), 3.72 (d, *J* = 13.2 Hz, 1H), 2.48-2.34 (m, 19H), 1.45-0.95 (m, 21H).

### Example 37 (Not a part of the present application)

### Synthesis of Ru complex 4aq

The synthetic procedure for preparation of ligand **3aq** is the same as in Example 1 in 5.0 mmol scale. 0.63g of yellow oil product **3aq** was obtained (yield: 52%).

Ligand **3aq** ¹H-NMR (400 MHz, CDCl₃): δ 7.54 (d, *J* = 2.0 Hz, 1H), 7.32 (d, *J* = 8.4 Hz, 1H), 7.23 (dd, *J* = 2.0 Hz, 8.4 Hz, 1H), 6.98 (dd, *J* = 11.2 Hz, 17.2 Hz, 1H), 6.89 (td, *J* = 1.6 Hz, 7.6 Hz, 1H), 6.83 (td, *J* = 1.6 Hz, 8.0 Hz, 1H), 6.73 (td, *J* = 1.6 Hz, 8.0Hz, 1H), 6.59 (dd, *J* = 1.6 Hz, 7.6 Hz, 1H), 5.74 (dd, *J* = 0.80 Hz, 17.2 Hz, 1H), 5.40 (dd, *J* = 0.80 Hz, 11.2 Hz, 1H), 4.33 (s, 2H), 3.86 (s, 3H).

The procedure for preparation of Ru complex **4aq** is the same as in Example 1 in 1.0 mmol scale. 665 mg of green solid product **4aq** was obtained (yield: 90%).

Ru complex **(4aq)** ¹H-NMR (400 MHz, CDCl₃): δ 18.75 (s,1H, Ru=CH), 7.50-7.44 (m, 2H), 7.04-6.36 (m, 9H), 5.32-5.21 (m, 1H), 4.65 (d, *J* = 13.2 Hz, 1H), 4.16-4.04 (m, 4H, NCH₂CH₂N), 3.59 (s, 3H), 3.48 (d, *J* = 13.2 Hz, 1H), 2.62-2.32 (m, 18H).

### Example 38 (Not a part of the present application)

### Synthesis of Ru complex 4ar

The synthetic procedure for preparation of ligand **3ar** is the same as in Example 1 in 5.0 mmol scale. 0.56g of yellow oil product **3ar** was obtained (yield: 44%).

Ligand **3ar** ¹H-NMR (400 MHz, CDCl₃): δ 7.52 (d, *J* = 2.0 Hz, 1H), 7.31 (d, *J* = 8.4 Hz, 1H), 7.22 (dd, *J* = 2.0 Hz, 8.4 Hz, 1H), 6.96 (dd, *J* = 11.2 Hz, 17.2 Hz, 1H), 6.86-6.81 (m, 2H), 6.68 (td, *J* = 1.2 Hz, 7.6Hz 1H,), 6.56 (dd, *J* = 1.6 Hz, 7.6 Hz, 1H), 5.73 (dd, *J* = 0.8 Hz, 17.2 Hz, 1H,), 5.39 (dd, *J* = 0.8 Hz, 11.2 Hz, 1H), 4.56 (m, 1H), 4.33 (s, 3H), 1.35 (d, *J* = 6.0 Hz, 6H).

The procedure for preparation of Ru complex **4ar** is the same as in Example 1 in 1.0 mmol scale. 499 mg of green solid product **4ar** was obtained (yield: 65%).

Ru complex **(4ar)** ¹HNMR (300 MHz, CDCl₃): δ 18.82 (s,1H, Ru=CH), 7.47-7.43 (m, 2H), 7.01-6.56 (m, 9H), 5.12-5.09 (m, 1H), 4.56-4.45 (m, 2H), 4.40-4.15 (m, 4H, NCH₂CH₂N), 3.48-3.45 (m, 1H), 2.64-2.04 (m, 18H), 1.10 (d, *J* = 6.4 Hz, 6H).

### Example 39 (Not a part of the present application)

### Synthesis of Ru complex 4as

The synthetic procedure for preparation of ligand **3as** is the same as in Example 1 in 5.0 mmol scale. 0.45g of yellow oil product **3as** was obtained (yield: 34%).

Ligand **3as** ¹H-NMR (400 MHz, CDCl₃): δ 8.20 (d, *J* = 1.5 Hz, 1H), 7.90 (dd, *J* = 1.5 Hz, 8.0 Hz, 1H), 7.45 (d, *J* = 8.0 Hz 1H,), 7.01 (dd, *J* = 11.5 Hz, 17.0 Hz, 1H), 6.83-6.80 (m, 2H), 6.67 (td, *J* = 2.0 Hz, 7.0 Hz, 1H), 6.52 (dd, *J* = 2.0 Hz, 7.5 Hz, 1H), 5.80 (d, *J* = 17.0 Hz, 1H), 5.42 (d, *J* = 11.5 Hz, 1H), 4.56 (m, 1H), 4.42 (s, 2H), 3.93 (s, 3H), 1.34 (d, *J* = 6.5 Hz, 6H).

The procedure for preparation of Ru complex **4as** is the same as in Example 1 in 1.0 mmol scale. 467 mg of green solid product **4as** was obtained (yield: 59%).

Ru complex **(4as)** ¹HNMR (400 MHz, CDCl₃): δ 18.82 (s,1H, Ru=CH), 8.15 (dd, *J* = 6.4, 1.2 Hz, 2H), 7.51 (d, *J* = 1.2 Hz, 1H), 7.44 (d, *J* = 1.2 Hz, 1H), 7.05-6.99 (m, 5H), 8.15 (d, *J* = 6.4Hz, 2H), 6.59-6.56 (m, 1H), 5.22 (m, 1H), 4.63 (m, 1H), 4.41(m,1H), 3.96 (m, 4H, NCH₂CH₂N), 3.55-3.52 (m, 1H), 2.66-2.33 (m, 18H), 1.14 (d, *J* = 6.4 Hz, 6H).

### Example 40 (Not a part of the present application)

### Synthesis of Ru complex 4at

The synthetic procedure for preparation of ligand **3at** is the same as in Example 1 in 5.0 mmol scale. 0.53g of yellow oil product **3at** was obtained (yield: 33%).

Ligand **3at** ¹H-NMR (400 MHz, CDCl₃): δ 7.91 (s, 1H), 7.63 (d, *J* = 8.0 Hz, 1H), 7.56 (d, *J* = 8.0 Hz, 1H), 7.03 (dd, *J* = 11.2 Hz, 17.2 Hz, 1H), 6.88-6.82 (m, 2H), 6.74 (t, *J* = 8.0 Hz, 1H), 6.53 (d, *J* = 7.6 Hz, 1H), 5.81 (d, *J* = 17.2 Hz, 1H), 5.49 (d, *J* = 11.2 Hz, 1H), 4.44 (s, 2H), 3.88(s, 3H), 2.74 (s, 6H).

The procedure for preparation of Ru complex **4at** is the same as in Example 1 in 1.0 mmol scale. 341 mg of green solid product **4at** was obtained (yield: 42%).

Ru complex **(4at)** ¹HNMR (400 MHz, CDCl₃): δ 19.02 (s,1H, Ru=CH), 7.87 (dd, *J* = 8.0, 1.2 Hz, 1H), 7.44 (dd, *J* = 7.2, 1.2 Hz, 1H), 7.25-7.03 (m, 9H), 5.37-5.30 (m, 1H), 4.76-4.74 (m, 1H), 4.16-4.01 (m, 4H, NCH₂CH₂N), 3.58-3.54 (m, 4H), 2.75 (s, 6H), 2.73-1.98 (m, 18H).

### Example 41 (Not a part of the present application)

### Synthesis of Ru complex 4au

The synthetic procedure for preparation of ligand **3au** is the same as in Example 1 in 5.0 mmol scale. 0.58g of yellow oil product **3au** was obtained (yield: 39%).

Ligand **3au** ¹H-NMR (400 MHz, CDCl₃): δ 7.93 (s, 1H), 7.64 (d, *J* = 8.0 Hz, 1H), 7.50 (d, *J* = 8.0 Hz, 1H), 7.01 (dd, *J* = 10.8 Hz, 16.8 Hz, 1H), 6.84-6.69 (m, 3H), 6.49 (d, *J* = 7.6 Hz, 1H), 5.74 (d, *J* = 16.8 Hz, 1H), 5.47 (d, *J* = 10.8 Hz, 1H), 4.59-4.53 (m, 1H), 4.43 (s, 2H), 3.14 (t, *J* = 8 Hz, 4H), 1.51 (m, 4H), 1.36 (d, *J* = 5.6 Hz, 6H), 1.33-1.27 (m, 4H), 0.90 (t, *J* = 7.2 Hz, 6H).

The procedure for preparation of Ru complex **4au** is the same as in Example 1 in 1.0 mmol scale. 471 mg of green solid product **4au** was obtained (yield: 51%).

Ru complex **(4au)** ¹HNMR (300 MHz, CDCl₃): δ 19.06 (s,1H, Ru=CH), 7.87 (d, *J* = 7.6Hz, 1H), 7.42 (d, *J* = 7.6Hz, 1H), 7.29 (d, *J* = 12.0 Hz, 1H), 7.11-6.56 (m, 8H), 5.22-5.19 (m, 1H), 4.63-4.64 (m, 1H), 4.45-4.42 (m, 1H), 4.14-4.01 (m, 4H, NCH₂CH₂N), 3.56-3.53 (m, 1H), 3.12-3.07 (m, 4H), 2.67-2.36 (m, 18H), 1.99-1.00 (m, 24H).

### Example 42 (Not a part of the present application)

### Synthesis of Ru complex 4av

The synthetic procedure for preparation of ligand **3av** is the same as in Example 1 in 5.0 mmol scale. 0.65g of white solid product **3av** was obtained (yield: 39%).

Ligand **3av** ¹H-NMR (400 MHz, CDCl₃): δ = 7.26 Hz) : 7.894 (s, 1H), 7.624 (d,1H, *J* = 8 Hz), 7.553 (d, *J* = 8 Hz, 1H), 7.017 (dd, *J* = 10.8 Hz, 17.2 Hz, 1H), 6.844-6.789 (m, 2H), 6.711 (d, *J* = 8 Hz, 1H), 6.698 (t, *J* = 8 Hz, 1H), 5.800 (d, *J* = 17.2 Hz, 1H), 5.493 (d, *J* = 10.8 Hz, 1H), 4.584 (m, 1H), 4.453 (s, 2H), 2.737 (s, 6H), 1.365 (d, *J* = 6 Hz, 6H).

The procedure for preparation of Ru complex **4av** is the same as in Example 1 in 1.0 mmol scale. 622 mg of green solid product **4av** was obtained (yield: 74%).

Ru complex **(4av)** ¹HNMR (300 MHz, CDCl₃): δ 19.06 (s,1H, Ru=CH), 7.87 (d, *J* = 7.6Hz, 1H), 7.42 (d, *J* = 7.6Hz, 1H), 7.11-6.56 (m, 9H), 5.27-5.20 (m, 1H), 4.64-4.61 (m, 1H), 4.46-4.44 (m, 1H), 4.14-4.01 (m, 4H, NCH₂CH₂N), 3.59-3.56 (m, 1H), 3.12-3.07 (m, 4H), 2.75 (s, 6H), 2.67-2.36 (m, 18H), 1.13 (d, *J* = 6.0Hz, 6H).

### Example 43 (Not a part of the present application)

### Synthesis of Ru complex 4aw

The structure of ligand **3aw** is the same as **3av** for preparation of Ru complex **4aw,** just another Ru complex reagent **1a** was used instead of Ru reagent **1b**.

The procedure for preparation of Ru complex **4aw** is the same as in Example 2 in 1.0 mmol scale. 626 mg of green solid product **4aw** was obtained (yield: 77%).

Ru complex **(4aw)** ¹HNMR (400 MHz, CDCl₃): δ 19.56 (d, *J* = 9.6 Hz, Ru=CH), 8.21 (d, *J* = 8.0 Hz, 1H), 8.09 (d, *J* = 2.0 Hz, 1H), 8.10 (dd, *J* = 7.6, 2 Hz, 1H), 7.34-6.87 (m, 4H), 5.47-5.44 (m, 1H), 5.33-5.27 (m, 1H), 4.62-4.56 (m, 1H), 3.99-3.96 (m, 1H), 2.80 (s, 6H), 2.30-1.24 (m, 39H, PCy₃).

### Example 44 (Not a part of the present application)

### Synthesis of Ru complex 4ax

The synthetic procedure for preparation of ligand **3ax** is the same as in Example 1 in 5.0 mmol scale. 0.77g of yellow product **3ax** was obtained (yield: 55%). The product **3t** is confirmed by LC-MS (M+H⁺): m/z calculated: 431.2, found: 431.2, and directly used for preparation of the Ru complex **4ax.**

The procedure for preparation of Ru complex **4ax** is the same as in Example 1 in 1.0 mmol scale. 421 mg of green solid product **4ax** was obtained (yield: 47%).

Ru complex **(4ax)** ¹HNMR (400 MHz, CDCl₃): δ 18.99 (s,1H, Ru=CH), 7.88 (dd, *J* = 8.0, 2.0 Hz, 1H), 7.44 (dd, *J* = 7.2, 1.2 Hz, 1H), 7.28-6.63 (m, 9H), 5.35-5.28 (m, 1H), 4.75-4.72 (m, 1H), 4.16-4.12 (m, 4H, NCH₂CH₂N), 3.61 (s, 3H), 3.56-3.52 (m, 4H), 3.10-3.06 (m, 4H), 2.63-2.05 (m, 18H), 1.37-0.98 (m, 14H).

### Example 45 (Not a part of the present application)

### Synthesis of Ru complex 4ay

The synthetic procedure for preparation of ligand **3ay** is the same as in Example 1 in 5.0 mmol scale. 0.56g of yellow oil product **3ay** was obtained (yield: 31%).

Ligand **3ay** ¹H-NMR (400 MHz, CDCl₃): δ 8.02 (d, *J* = 1.6 Hz, 1H), 7.72 (dd, *J* = 1.6 Hz, 8.4 Hz, 1H), 7.51 (d, *J* = 8.4 Hz, 1H), 7.01 (dd, *J* = 10.8 Hz, 17.6 Hz, 1H), 6.84-6.80 (m, 2H), 6.70 (td, *J* = 1.2 Hz, 7.6 Hz, 1H), 6.48 (dd, *J* = 1.2 Hz, 8.0 Hz, 1H), 5.80 (d, *J* = 17.6 Hz, 1H), 5.48 (d, *J* = 10.8 Hz, 1H), 4.67 (bs, 1H), 4.58 (m, 1H), 4.44 (s, 2H), 3.22-3.15 (bm, 1H), 1.81-1.77 (bm, 2H), 1.68-1.63 (bm, 2H), 1.36 (d, *J* = 6 Hz, 6H), 1.32-1.12 (m, 6H).

The procedure for preparation of Ru complex **4ay** is the same as in Example 1 in 1.0 mmol scale. 241 mg of green solid product **4ay** was obtained (yield: 27%).

Ru complex **(4ay)** ¹HNMR (400 MHz, CDCl₃): δ 19.03 (s, 1H, Ru=CH), 7.60 (d, *J* = 7.6 Hz, 1H), 7.43 (d, *J* = 3.6 Hz, 1H), 7.14 (s, 1H), 7.09-7.00 (m, 5H), 6.81-6.57(m, 3H), 5.22 (m, 1H), 4.64-4.61 (m, 1H), 4.64-4.42 (m, 2H), 4.15-4.02 (m, 4H, NCH₂CH₂N), 3.16 (m, 1H), 3.17 (m, 1H), 2.67-2.00 (m, 18H), 1.85-1.00 (m, 16H).

### Example 46

### Synthesis of Ru complex 4ba

The synthetic procedure for preparation of ligand **3ba** is the same as in Example 1 in 5.0 mmol scale. 0.96g of yellow oil product **3ba** was obtained (yield: 67%).

Ligand **3ba** ¹H NMR (CDCl₃, 400 MHz): δ 7.23 (m, 4H), 6.92 (m, 2H), 6.80 (m, 1H), 6.67 (m, 2H), 5.68 (d, 1H), 5.39 (d, 1H), 4.64 (s, 2H), 4.06 (s, 2H), 3.75 (s, 3H).

The procedure for preparation of Ru complex **4ba** is the same as in Example 1 in 1.0 mmol scale. 176 mg of green solid product **4ba** was obtained (yield: 22%).

Ru complex **(4ba)** ¹HNMR (400 MHz, CDCl₃): δ 18.74 (s, 1H, Ru=CH), 7.25-7.24 (m, 1H), 7.19(s, 1H), 7.14-7.04 (m, 7H), 6.93 (s, 1H), 6.71 (s, 1H), 6.41-6.40 (d, *J*=9.0 Hz, 1H), 6.10-6.07 (d, *J*=12.0 Hz, 1H), 4.52-4.49 (d, *J*=13.5Hz, 1H), 4.33-4.29 (d, *J*=18.5 Hz, 1H), 4.09 (s, 2H), 3.92 (s, 2H), 3.31 (s, 3H), 2.96-2.92 (d, *J*=19.0Hz, 1H), 2.83 (s, 3H), 2.71(s, 3H), 2.47 (s, 3H), 2.39 (s, 3H), 2.06 (s, 3H), 2.02 (s, 3H).

### Example 47

### Synthesis of Ru complex 4bb

The synthetic procedure for preparation of ligand **3bb** is the same as in Example 1 in 5.0 mmol scale. 1.13g of yellow oil product **3bb** was obtained (yield: 71%).

Ligand **3bb** ¹H NMR (CDCl₃, 400 MHz): δ 7.21 (m, 4H), 6.90 (m, 2H), 6.78 (m, 1H), 6.67 (d, 2H), 5.68 (d, 1H), 5.38 (d, 1H), 5.06 (m, 1H), 4.64 (s, 2H), 3.99 (s, 2H), 1.23 (d, 6H).

The procedure for preparation of Ru complex **4bb** is the same as in Example 1 in 1.0 mmol scale. 237 mg of green solid product **4bb** was obtained (yield: 30%).

Ru complex **(4bb)** ¹HNMR (400 MHz, CDCl₃): δ 18.74 (s, 1H, Ru=CH), 7.27-7.25 (dd, *J*=8.0, 3.0 Hz, 1H), 7.19 (s, 1H), 7.14-7.05 (m, 7H), 6.93 (s, 1H), 6.71 (s, 1H), 6.42-6.40 (d, *J*=9.0 Hz, 1H), 6.07-6.05 (d, *J*=12.5 Hz, 1H), 4.65-4.61 (m, 1H), 4.51-4.49 (d, *J*=12.5 Hz, 1H), 4.24-4.20 (d, *J*=18.0 Hz, 1H), 4.10 (s, 2H), 3.92 (s, 2H), 2.90-2.86 (d, *J*=18 Hz, 1H), 2.83 (s, 3H), 2.71 (s, 3H), 2.47 (s, 3H), 2.39 (s, 3H), 2.07 (s, 3H), 2.03 (s, 3H), 0.90-0.82 (d, *J*=33.0, 6.5 Hz, 6H).

### Example 48

### Synthesis of Ru complex 4bc

The synthetic procedure for preparation of ligand **3bc** is the same as in Example 1 in 5.0 mmol scale. 0.74g of yellow oil product **3bc** was obtained (yield: 43%).

Ligand **3bc** ¹H NMR (CDCl₃, 400 MHz): δ 7.23 (m, 2H), 6.92 (m, 2H), 6.81 (m, 2H), 6.67 (m, 2H), 5.67 (d, 1H), 5.37 (d, 1H), 5.05 (m, 1H), 4.57 (s, 2H), 3.98 (s, 2H), 3.77 (s, 3H), 1.22 (d, 6H).

The procedure for preparation of Ru complex **4bc** is the same as in Example 1 in 1.0 mmol scale. 578 mg of green solid product **4bc** was obtained (yield: 73%).

Ru complex **(4bc)** ¹HNMR (400 MHz, CDCl₃): δ 18.72 (s, 1H, Ru=CH), 7.24-7.22 (dd, *J*=8.5, 2.5 Hz, 1H), 7.16 (s, 1H), 7.07-7.04 (m, 4H), 6.91 (s, 1H), 6.75 (s, 1H), 6.66 (s, 1H), 6.64(s, 1H), 6.39-6.38 (d, *J*=8.0 Hz, 1H), 6.02-6.00 (d, *J*=12.0 Hz, 1H), 4.64-4.59 (m, 1H), 4.50-4.47 (d, *J*=13.0 Hz, 1H), 4.13-4.09 (d, *J*=18 Hz, 1H), 4.08 (s, 2H), 3.90 (s, 2H), 3.83(s, 3H), 2.81 (s, 3H), 2.81-2.79 (d, *J*=11.5 Hz, 1H), 2.69 (s, 3H), 2.45 (s, 3H), 2.39 (s, 3H), 2.08 (s, 3H), 2.01 (s, 3H), 0.89-0.81 (dd, *J*=34.0, 6.0 Hz, 6H).

### Example 49

### Synthesis of Ru complex 4bd

The synthetic procedure for preparation of ligand **3bd** is the same as in Example 1 in 5.0 mmol scale. 0.96g of yellow oil product **3bd** was obtained (yield: 52%).

Ligand **3bd** ¹H NMR (CDCl₃, 400 MHz): δ 7.21 (m, 2H), 7.16 (m, 2H), 6.86 (m, 2H), 6.58 (m, 2H), 5.68 (d, 1H), 5.39 (d, 1H), 5.06 (m, 1H), 4.60 (s, 2H), 3.97 (s, 2H), 1.23 (d, 6H).

The procedure for preparation of Ru complex **4bd** is the same as in Example 1 in 1.0 mmol scale. 236 mg of green solid product **4bd** was obtained (yield: 29%).

Ru complex **(4bd)** ¹HNMR (400 MHz, CDCl₃): δ 18.72 (s, 1H, Ru=CH), 7.28-7.26 (m, 1H), 7.19 (s, 1H), 7.10-7.05 (m, 6H), 6.94 (s, 1H), 6.82 (s, 1H), 6.41-6.39 (d, *J*=9.5 Hz, 1H), 6.07-6.04 (d, *J*=12.0 Hz, 1H), 4.68-4.64 (m, 1H), 4.45-4.43 (d, *J*=12.5 Hz, 1H), 4.24-4.20 (d, *J*=18.0 Hz, 1H), 4.09 (s, 2H), 3.93(s, 2H), 2.91-2.87 (d, *J*=18.5 Hz, 1H), 2.81 (s, 3H), 2.70 (s, 3H), 2.47 (s, 6H), 2.10 (s, 3H), 2.03 (s, 3H), 0.93-0.87 (dd, *J*=24.0, 7.0 Hz, 6H).

### Example 50

### Synthesis of Ru complex 4be

The synthetic procedure for preparation of ligand **3be** is the same as in Example 1 in 5.0 mmol scale. 1.46g of yellow oil product **3be** was obtained (yield: 84%).

Ligand **3be** H NMR (CDCl₃, 400 MHz): δ 7.23 (m, 2H), 6.91 (m, 4H), 6.61 (m, 2H), 5.68 (d, 1H), 5.38 (d, 1H), 5.05 (m, 1H), 4.58 (s, 2H), 3.95 (s, 2H), 1.23 (d, 6H).

The procedure for preparation of Ru complex **4be** is the same as in Example 1 in 1.0 mmol scale. 396 mg of green solid product **4be** was obtained (yield: 49%).

Ru complex **(4be)** ¹HNMR (400 MHz, CDCl₃): δ 18.71 (s, 1H, Ru=CH), 7.29-7.25 (dd, *J*=8.5, 2.5 Hz, 1H), 7.19 (s, 1H), 7.13-7.06 (m, 4H), 6.94 (s, 1H), 6.82-6.77 (m, 3H), 6.42-6.39 (dd, *J*=9.5, 2.5 Hz, 1H), 6.08-6.05 (d, *J*=13.0 Hz, 1H), 4.66-4.64 (m, 1H), 4.47-4.45 (d, *J*=12.5 Hz, 1H), 4.21-4.18 (d, *J*=18 Hz, 1H), 4.10 (s, 2H), 3.93 (s, 2H), 3.89-3.86 (d, *J*=18 Hz, 1H), 2.83 (s, 3H), 2.70 (s, 3H), 2.48 (s, 3H), 2.42 (s, 3H), 2.11 (s, 3H), 2.02 (s, 3H), 0.92-0.85 (dd, *J*=26.5, 7.0 Hz, 3H).

### Example 51

### Synthesis of Ru complex 4bf

The synthetic procedure for preparation of ligand **3bf** is the same as in Example 1 in 5.0 mmol scale. 0.68g of yellow oil product **3bf** was obtained (yield: 51%).

Ligand **3bf** ¹H NMR (CDCl₃, 400 MHz): δ 7.26 (m, 1H), 7.22 (m, 2H), 6.92 (m, 1H), 5.68 (d, 1H), 5.37 (d, 1H), 5.09 (m, 1H), 3.74 (s, 2H), 3.26 (s, 2H), 2.30 (m, 3H), 1.26 (d, 6H).

The procedure for preparation of Ru complex **4bf** is the same as in Example 1 in 1.0 mmol scale. 76 mg of green solid product **4bf** was obtained (yield: 10%).

Ru complex **(4bf)** ¹HNMR (400 MHz, CDCl₃): δ 18.54 (s, 1H, Ru=CH), 7.16-6.87 (m, 7H), 6.15-6.13 (dd, *J*=10.0, 2.0 Hz, 1H), 5.44-5.41 (d, *J*=13.5 Hz, 1H), 4.76-4.71 (m, 1H), 4.37-4.34 (d, *J*=15.5 Hz, 1H), 3.96 (s, 4H, NCH₂CH₂N), 3.07-3.05 (d, *J*=13 Hz, 1H), 2.75-2.40 (m, 18H), 1.66 (s, 3H), 1.21-1.17 (dd, *J*=13.0, 6.5 Hz, 6H).

### Example 52 (Not a part of the present application)

### Synthesis of Ru complex 4bg

The synthetic procedure for preparation of ligand **3bg** is the same as in Example 1 in 5.0 mmol scale. 0.83g of yellow oil product **3bg** was obtained (yield: 59%).

Ligand **3bg** ¹H NMR (CDCl₃, 400 MHz): δ 7. 24 (m, 2H), 6.93 (m, 2H), 6.70 (m, 1H), 6.30 (m, 2H), 5.71 (d, 1H), 5.40 (d, 1H), 4.58 (s, 1H), 4.44 (m, 1H), 4.29 (s, 2H), 1.28 (d, 6H).

The procedure for preparation of Ru complex **4bg** is the same as in Example 1 in 1.0 mmol scale. 302mg of green solid product **4bg** was obtained (yield: 39%).

Ru complex **(4bg)** ¹HNMR (400 MHz, CDCl₃): δ 18.91 (s, 1H, Ru=CH), 7.60-7.58 (dd, *J* = 9.5, 2.5 Hz, 1H), 7.24-7.20 (m, 1H), 7.13-7.05 (m, 3H), 6.94-6.92 (dd, *J* = 8.0, 6.0 Hz, 1H), 6.80 (brs, 1H), 6.74-6.70 (m, 1H), 6.64-6.61 (dd, *J* = 9.0, 5.0 Hz, 1H), 6.45-6.43 (dd, *J* = 10.5, 3.0 Hz, 1H), 5.20-5.15 (t, *J* = 13.5, 1H, NCH₂), 4.69-4.67 (d, *J* = 12.5 Hz, 1H, NCH₂), 4.38-4.33 (m, 1H, OCH(CH₃)₂), 4.12-4.08(m, 4H, NCH₂CH₂N), 3.47-3.45 (d, *J* = 12.5 Hz, 1H, NH), 2.65 (s, 6H), 2.56 (s, 6H), 2.26 (s, 3H), 2.09 (s, 3H), 1.14-1.12 (dd, *J* = 6.0, 4.0 Hz, 6H, OCH(CH₃)₂).

### Example 53 (Not a part of the present application)

### Synthesis of Ru complex 4bh

The synthetic procedure for preparation of ligand **3bh** is the same as in Example 1 in 5.0 mmol scale. 0.94g of yellow oil product **3bh** was obtained (yield: 78%).

Ligand **3bh** ¹H NMR (CDCl₃, 400 MHz): δ 7.99 (s, 1H), 7.95-7.93 (m, 1H), 7.55-7.53 (d, 1H), 7.36-7.32 (m, 2H), 7.30-7.23 (m, 2H), 7.03-6.98 (m, 1H), 6.66-6.61 (m, 2H), 5.72-5.68 (m, 1H), 5.36-5.34 (m, 1H), 4.46-4.45 (d, 2H), 3.85 (s, 3H).

The procedure for preparation of Ru complex **4bh** is the same as in Example 1 in 1.0 mmol scale. 542mg of green solid product **4bh** was obtained (yield: 74%).
Ru complex **(4bh)** ¹HNMR (400 MHz, CDCl₃): δ 18.89 (s, 1H, Ru=CH), 7.91-7.89 (d, *J* = 8.0 Hz, 1H), 7.76-7.74 (dd, *J* = 8.0, 1.5 Hz, 1H), 7.51-7.48 (td, *J* = 8.5, 7.0, 1.5 Hz, 1H), 7.25-7.21(td, *J* = 13.5, 11.0, 2.0 Hz, 1H), 7.19-7.16 (t, *J* = 8.0 Hz, 1H), 7.12-7.09 (t, *J* = 7.5 Hz, 2H), 7.04-7.03 (d, *J* = 7.0 Hz, 1H), 7.00-6.88 (m, 3H), 6.78-6.76 (d, *J* = 7.0 Hz, 1H), 6.65 (brs, 1H, NH), 6.64-6.59 (t, *J* = 12.5 Hz, 1H, NCH₂), 4.08(brs, 2H, NCH₂CH₂N), 3.99 (brs, 2H, NCH₂CH₂N), 3.72-3.69 (dd, *J* = 13.5, 2.0 Hz, 1H, NCH₂), 3.67 (s, 3H, COOCH₃), 2.62-2.03 (m, 18H).

### Example 54 (Not a part of the present application)

### Synthesis of Ru complex 4bj

The synthetic procedure for preparation of ligand **3bj** is the same as in Example 1 in 5.0 mmol scale. 0.99g of yellow oil product **3bj** was obtained (yield: 82%).

Ligand **3bj** ¹H NMR (CDCl₃, 400 MHz): δ 7.58-7.57 (d, 1H), 7.38-7.36 (d, 1H), 7.32-7.25 (m, 2H), 7.08-7.00 (m, 3H), 6.74-6.70 (m, 1H), 6.65-6.63 (d, 1H), 5.73-5.69 (m, 1H), 5.33-5.30 (m, 1H), 4.90 (s, 1H), 4.35 (s, 2H), 2.63 (s, 6H).

The procedure for preparation of Ru complex **4bj** is the same as in Example 1 in 1.0 mmol scale. 508mg of green solid product **4bj** was obtained (yield: 69%).

Ru complex **(4bj)** ¹HNMR (400 MHz, CDCl₃): δ 18.90 (s, 1H, Ru=CH), 7.63-7.61 (d, *J* = 7.5 Hz, 1H), 7.49-7.46 (t, *J* = 7.0 Hz, 1H), 7.19-7.16 (t, *J* = 8.0 Hz, 1H), 7.11-6.95 (m, 6H), 6.87-6.84 (t, *J* = 8.0 Hz, 1H), 6.80-6.79 (d, *J* = 7.5 Hz, 1H), 6.72 (brs, 1H), 6.68-6.65 (d, *J* = 11.5 Hz, 1H, NCH₂), 5.50-5.45 (t, *J* = 13.0 Hz, 1H, NCH₂), 4.15-3.96 (m ,4H, NCH₂CH₂N), 3.51-3.48 (d, *J* = 13.5 Hz, 1H, NH), 2.66-2.30 (m, 21H, aromatic CH₃, NCH₃), 2.05 (brs, 3H, NCH₃).

### Example 55 (Not a part of the present application)

### Synthesis of Ru complex 6a

**SM1-5a** (5.0mmol) and **SM2-5a** (5.0mmol) was added into a 50 mL of three-neck round-bottom flask filled with inert gas (Ar), and followed by adding anhydrous DCM (10 mL) and Na₂SO₄ (5 eq) was added. The reaction was stirred until completed overnight (monitored by TLC). The reaction mixture was filtered, and the crude imine product **5a** (1.25g, 97%) was obtained by removing all DCM solvent under vacuum. The crude imine product **5a** was directly used for next step to prepare Ru complex **6a.**

To a 50 mL two-necked round bottom flask, after filling with Ar atmosphere, were added ligand **5a** (1.0mmol) and CuCl (3.0mmol, 3eq) and 30 mL dry DCM, followed by refilling with Ar three times and protected with Ar balloon in close system. Ru complex **1b** (1.0mmol) was added under Ar protection, and the mixture was stirred for 0.5 hr at room temperature.

After the reaction was complete, the solution was filtered and the filtrate was concentrated and slurred with silica gel. The crude was obtained by silica gel column chromatography and washed with methanol or pentane-DCM to obtain 453mg of yellow-green solid product **6a,** yield: 79%.

Ru complex **(6a)** ¹HNMR (400 MHz, CDCl₃): δ 18.53 (s, 1H, Ru=CH), 8.59 (s, 1H), 7.28-6.49 (m, 11H), 4.160 (s, 4H, NCH₂CH₂N), 2.50 (s, 12H), 2.42 (s, 6H).

### Example 56 (Not a part of the present application)

### Synthesis of Ru complex 6b

The synthetic procedure for preparation of ligand **5b** is the same as in Example 52 in 5.0 mmol scale. 1.21g of crude imine product **5b** was obtained (yield: 95%), and it was directly used for next step to prepare Ru complex **6b.**

To a 50 mL two-necked round bottom flask, after filling with Ar atmosphere, were added ligand **5b** (1.0mmol) and CuCl (3.0mmol, 3eq) and 30 mL dry DCM, followed by refilling with Ar three times and protected with Ar balloon in close system. Ru complex **1a** (1.0mmol) was added under Ar protection, and the mixture was stirred for 0.5 hr at room temperature.

After the reaction was complete, the solution was filtered and the filtrate was concentrated and slurred with silica gel. The crude was obtained by silica gel column chromatography and washed with methanol or pentane-DCM to obtain 414mg yello-green solid product **6b,** yield: 77%.

Ru complex **(6b)** ¹HNMR (400 MHz, CDCl₃): δ 19.20 (d, *J* = 10.8 Hz, Ru=CH), 8.82 (d, *J* = 9.2 Hz, 1H), 7.84 (m, 1H), 7.80 (d, *J* = 8.4 Hz, 1H), 7.45 (m, 4H), 2.46-1.29 (m, 33H, PCy₃).

### Example 57 (Not a part of the present application)

### Synthesis of Ru complex 6c

The synthetic procedure for preparation of ligand **5c** is the same as in Example 52 in 5.0 mmol scale. 1.16g of crude imine product **5c** was obtained (yield: 92%), and it was directly used for next step to prepare Ru complex **6c.**

The synthetic procedure is the same as in Example 52 in 1.0 mmol scale. 664 mg of yellow-green solid product **6c** was obtained (96% yield).

Ru complex **(6c)** ¹HNMR (400 MHz, CDCl₃): δ 18.52 (s, 1H, Ru=CH), 8.60(s, 1H), 7.28-7.13 (m, 7H), 7.02 (d, *J* = 8.8 Hz, 1H), 6.80 (m, 1H), 6.09 (d, *J* = 8.8 Hz, 1H), 4.16 (s, 4H, NCH₂CH₂N), 3.84 (s, 3H), 2.51 (m, 18H).

### Example 58 (Not a part of the present application)

### Synthesis of Ru complex 6d

The synthetic procedure for preparation of ligand **5d** is the same as in Example 52 in 5.0 mmol scale. 1.18g of crude imine product **5d** was obtained (yield: 94%), and it was directly used for next step to prepare Ru complex **6d.**

The synthetic procedure is the same as in Example 52 in 1.0 mmol scale. 68 mg of yellow-green solid product **6d** was obtained (31% yield).

Ru complex **(6d):** ¹HNMR (400 MHz, CDCl₃): δ 18.73 (s, 1H, Ru=CH), 8.62 (s, 1H), 7.67-7.46 (m, 3H), 7.11 (s, 4H), 6.78-6.65 (m, 5H), 4.13 (s, 4H, NCH₂CH₂N), 3.81 (s, 3H), 2.49 (m, 18H).

### Example 59 (Not a part of the present application)

### Synthesis of Ru complex 6e

The synthetic procedure for preparation of ligand **5e** is the same as in Example 52 in 5.0 mmol scale. 1.13g of crude imine product **5e** was obtained (yield: 93%), and it was directly used for next step to prepare Ru complex **6e.**

The synthetic procedure is the same as in Example 52 in 1.0 mmol scale. 41 mg of yellow-green solid product **6e** was obtained (24% yield).

Ru complex **(6e)** ¹HNMR (400 MHz, CDCl₃): δ 18.74 (s, 1H, Ru=CH), 8.60 (s, 1H), 7.69-7.49 (m, 3H), 7.12-7.04 (m, 8H), 6.80 (d, *J* = 8.7 Hz, 1H), 4.13 (s, 4H, NCH₂CH₂N), 2.50 (m, 18H).

### Example 60 (Not a part of the present application)

### Synthesis of Ru complex 6f

The synthetic procedure for preparation of ligand **5f** is the same as in Example 52 in 5.0 mmol scale. 1.28g of crude imine product **5f** was obtained (yield: 94%), and it was directly used for next step to prepare Ru complex **6f.**

The synthetic procedure is the same as in Example 52 in 1.0 mmol scale. 664 mg of yellow-green solid product **6f** was obtained (17% yield).

Ru complex **(6f)** ¹HNMR (400 MHz, CDCl₃): δ 18.60 (s, 1H, Ru=CH), 8.58 (s, 1H), 7.48-7.29 (m, 2H), 7.02 (d, *J* = 8.8 Hz, 2H), 6.74-6.69 (m, 3H), 4.17 (s, 4H, NCH₂CH₂N), 3.85 (s, 3H), 2.52 (m, 18H).

### Example 61 (Not a part of the present application)

### Synthesis of Ru complex 6g

The synthetic procedure for preparation of ligand **5g** is the same as in Example 52 in 5.0 mmol scale. 1.23g of crude imine product **5g** was obtained (yield: 96%), and it was directly used for next step to prepare Ru complex **6g.**

The synthetic procedure is the same as in Example 52 in 1.0 mmol scale. 35 mg of green solid product **6g** was obtained (22% yield).

Ru complex **(6g)** ¹H-NMR (400 MHz, CDCl₃): δ 18.66 (s, 1H, Ru=CH), 8.56 (s, 1H), 7.50-7.34 (m, 2H), 7.26 (s, 4H), 7.00-6.40 (m, 5H), 4.14 (s, 4H, NCH₂CH₂N), 3.81 (s, 3H), 2.49 (m, 18H).

### Example 62 (Not a part of the present application)

### Synthesis of Ru complex 6h

The synthetic procedure for preparation of ligand **5h** is the same as in Example 52 in 5.0 mmol scale. 1.29g of crude imine product **5h** was obtained (yield: 96%), and it was directly used for next step to prepare Ru complex **6h.**

The synthetic procedure is the same as in Example 52 in 1.0 mmol scale. 106 mg of yellow-green solid product **6h** was obtained (37% yield).

Ru complex **(6h)** ¹HNMR (400 MHz, CDCl₃): δ 16.52 (s, 1H, Ru=CH), 8.43 (s, 1H, N=CH), 8.10 (s, 1H), 7.46-7.22 (m, 2H), 7.73-6.96 (m, 8H), 4.19 (s, 4H, NCH₂CH₂N), 3.95 (s, 3H), 3.87 (s, 3H), 2.49 (s, 12H), 2.48 (s, 6H).

### Example 63 (Not a part of the present application)

### Synthesis of Ru complex 6j

The synthetic procedure for preparation of ligand **5j** is the same as in Example 52 in 5.0 mmol scale. 1.31g of crude imine product **5j** was obtained (yield: 97%), and it was directly used for next step to prepare Ru complex **6j.**

The synthetic procedure is the same as in Example 52 in 1.0 mmol scale. 190 mg of red solid product **6j** was obtained, and the product **6j** is unstable and difficult to detect the structure by ¹HNMR. But the crude Ru complex **6j** could be directly used for metathesis reaction.

### Example 64

### Synthesis of Ru complex 8a

The synthetic procedure for preparation of ligand **7a** is the same as in Example 1 in 5.0 mmol scale. 0.26g of oily product **7a** was obtained (yield: 28%).

Ligand **7a** ¹H-NMR (400 MHz, CDCl₃): δ 7.21 (dd*, J* = 18.0, 11.20 Hz, 1H), 7.00 (td, *J* = 9.2, 2.8, 1.6 Hz, 1H), 6.73-6.67 (m, 1H), 5.67 (dd, *J* = 18.0, 1.2 Hz, 1H), 5.34 (d, *J* = 11.2 Hz, 1H), 2.77 (d, *J* = 2.4 Hz, 6H).

The procedure for preparation of Ru complex **8a** is the same as in Example 1 in 1.0 mmol scale. 208 mg of green solid product **8a** was obtained, yield: 32%.

Ru complex **(8a)** ¹HNMR (400 MHz, CDCl₃): δ16.80 (s, 1H, Ru=CH), 7.07 (s, 4H, aromatic H), 6.94 (m, 1H), 6.30 (d, *J* = 6.4 Hz, 1H), 4.11 (s, 4H, NCH₂CH₂N), 2.69 (s, 6H), 2.49 (s, (s, 12H), 2.42 (s, 6H).

### Example 65

### Synthesis of Ru complex 8b

The synthetic procedure for preparation of ligand **7b** is the same as in Example 1 in 5.0 mmol scale. 0.89g of solid product **7b** was obtained (yield: 92%).

Ligand **7b** ¹H-NMR (400 MHz, CDCl₃): δ 8.25 (d, *J* = 2.7 Hz, 1H), 8.05 (dd, *J* = 8.7 Hz, 2.4 Hz, 1H), 6.90 (d, *J* = 9.0 Hz, 1H), 6.82 (dd, *J* = 17.4, 11.1 Hz, 1H), 5.77 (dd*, J* = 17.7, 0.9 Hz, 1H), 5.37 (dd, *J* = 10.8, 0.6 Hz, 1H), 2.92 (s, 6H).

The procedure for preparation of Ru complex **8b** is the same as in Example 1 in 1.0 mmol scale. 59 mg of green solid product **8b** was obtained (yield: 9%).

Ru complex **(8b)** ¹HNMR (400 MHz, CDCl₃): δ 16.97 (s, 1H, Ru=CH), 8.40 (dd, *J* = 8.8, 2.4 Hz, 1H), 7.65 (d, *J* = 2.4 Hz, 1H), 7.29 (d, *J* = 8.8 Hz, 1H), 7.07 (s, 4H), 4.20 (s, 4H, NCH₂CH₂N), 2.57 (s, 6H), 2.47 (s, 12H), 2.39 (s, 6H).

### Example 66

### Synthesis of Ru complex 8c

The synthetic procedure for preparation of ligand **7c** is the same as in Example 1 in 5.0 mmol scale. 0.96g of yellow oil product **7c** was obtained (yield: 96%).

Ligand **7c** ¹H-NMR (400 MHz, CDCl₃): δ 8.19 (d, *J* = 2.8 Hz, 1H), 7.98 (dd, *J* =9.0, 2.8 Hz, 1H), 6.86 (d, *J* = 9.0 Hz, 1H), 6.73 (dd*, J* = 17.6 Hz, 11.2 Hz, 1H), 5.69 (d, *J* = 17.6 Hz, 1H), 5.29 (d, *J* = 11.2 Hz, 1H), 3.12 (q, 2H, *J* = 6.8 Hz), 2.78 (s, 3H), 1.09 (t, *J* = 6.8 Hz, 3H).

The procedure for preparation of Ru complex **8c** is the same as in Example 1 in 1.0 mmol scale. 161 mg of green solid product **8c** was obtained (24% yield).

Ru complex **(8c)** ¹HNMR (400 MHz, CDCl₃): δ 16.69 (s, 1H, Ru=CH), 8.36 (dd, *J* = 8.8, 2.4 Hz, 1H), 7.62 (d, *J* = 2.4 Hz, 1H), 7.18 (d, *J* = 8.8 Hz, 1H), 7.17-7.00 (m, 4H), 4.16-3.80 (m, 6H), 2.84-2.08 (m, 21H), 0.57 (t, *J* = 6.8 Hz, 3H).

### Example 67

### Synthesis of Ru complex 8d

The synthetic procedure for preparation of ligand **7d** is the same as in Example 1 in 5.0 mmol scale. 1.02g of yellow oil product **7d** was obtained (yield: 92%).

Ligand **7d** ¹H NMR(400 MHz, CDCl₃): 8.27(d, *J* = 2.7 Hz, 1H), 8.05 (dd, *J* = 9.0, 3.0 Hz, 1H),6.92 (d, *J* = 9.0 Hz, 1H), 6.75 (dd, *J* = 18.0, 10.8 Hz, 1H), 5.77 (dd, *J* = 17.7, 0.9 Hz, 1H), 5.34 (dd, *J* = 1.2 Hz, 10.8 Hz, 1H),3.71 (m, 1H), 2.74 (s, 3H), 1.13 (d, *J* = 6.6 Hz, 6H).

The procedure for preparation of Ru complex **8d** is the same as in Example 1 in 1.0 mmol scale. 103mg green solid product **8d** was obtained, yield: 15%.

The product is unstable, so it is difficult to detect the structure by ¹HNMR. But the crude Ru complex **8d** could be directly used for metathesis reaction.

### Example 68

### Synthesis of Ru complex 8e

The synthetic procedure for preparation of ligand **7e** is the same as in Example 1 in 5.0 mmol scale. 0.63g of yellow oil product **7e** was obtained (yield: 37%).

Ligand **7e** ¹H-NMR (400 MHz, CDCl₃): δ 8.11-8.06 (m, 2H, aromatic H), 6.65-6.55 (m, 2H, aromatic H, CH=CH₂), 5.61 (d, *J* = 17.1 Hz, CH=CH₂), 5.47 (d, *J* = 10.8 Hz, CH=CH₂), 4.43 (s, 1H, NH), 3.78-3.74 (m, 1H, NCH), 1.28 (d, *J* = 7.8 Hz, NCH(CH₃)₂).

The procedure for preparation of Ru complex **8e** is the same as in Example 1 in 1.0 mmol scale. 74mg green solid product **8e** was obtained, yield: 15%.

The product is unstable, so it is difficult to detect the structure by ¹HNMR. But the crude Ru complex **8e** could be directly used for metathesis reaction.

### Example 69

### Synthesis of Ru complex 8f

The synthetic procedure for preparation of ligand **7f** is the same as in Example 1 in 5.0 mmol scale. 0.68g of yellow oil product **7f** was obtained (yield: 66%).

Ligand **7f** ¹HNMR (400 MHz, CDCl₃): δ 8.11 (d, *J* = 2.0 Hz, 1H), 7.89 (dd, *J* = 8.8, 2.0 Hz, 1H), 6.96 (d, *J* = 8.8 Hz, 1H), 6.91 (dd, *J* = 12.0, 18.4 Hz, 1H), 5.76 (dd, *J* = 18.40, 1.20 Hz, 1H), 5.31 (dd, *J* = 12.0, 1.2 Hz, 1H), 3.90 (s, 3H), 2.83 (s, 6H).

The procedure for preparation of Ru complex **8f** is the same as in Example 1 in 1.0 mmol scale. 396 mg of green solid product **8f** was obtained (yield: 59%).

Ru complex **(8f)** ¹HNMR (400 MHz, CDCl₃): δ16.80 (s, 1H, Ru=CH), 8.18 (dd, *J* = 8.8, 2.4 Hz, 1H), 7.46 (d, *J* = 2.4 Hz, 1H), 7.23 (d, *J* = 8.8 Hz, 1H), 7.07 (s, 4H), 4.11 (s, 4H, NCH₂CH₂N), 3.91 (s, 3H), 2.58 (s, 6H), 2.47 (s, 12H), 2.43 (s, 6H).

### Example 70

### Synthesis of Ru complex 8g

The synthetic procedure for preparation of ligand **7g** is the same as in Example 1 in 5.0 mmol scale. 1.03g of yellow oil product **7g** was obtained (yield: 96%).

Ligand **7g** ¹H NMR (CDCl₃, 400Hz): δ 7.45-7.44 (m, 1H), 7.25-7.21 (m, 1H), 7.12-7.10 (m, 1H), 7.05-6.99 (m, 2H), 5.69-5.65 (m, 1H), 5.27-5.25 (m, 1H),3.80 (s, 2H), 3.70 (s, 3H), 2.90 (s, 3H).

The procedure for preparation of Ru complex **8g** is the same as in Example 1 in 1.0 mmol scale. 530 mg of green solid product **8g** was obtained (yield: 79%).

Ru complex **(8g)** ¹HNMR (400 MHz, CDCl₃): δ 16.70 (s, 1H, Ru=CH), 7.37 (m, 1H), 7.04-6.91 (m, 6H), 6.72 (d, *J* = 7.6 Hz, 1H), 5.05 (d, *J* = 11.6 Hz, 1H), 3.88-3.85 (m, 4H, NCH₂CH₂N), 3.52 (s, 3H), 3.44 (d, *J* = 11.6 Hz, 1H), 2.85-1.50 (m, 21H).

### Example 71

### Synthesis of Ru complex 8h

The synthetic procedure for preparation of ligand **7h** is the same as in Example 1 in 5.0 mmol scale. 0.64g of product **7h** was obtained (yield: 51%). The product **7h** is confirmed by LC-MS (M+H⁺): m/z calculated: 251.2, found: 251.2, and directly used for preparation of the Ru complex **8h.**

The procedure for preparation of Ru complex **8h** is the same as in Example 1 in 1.0 mmol scale. 530 mg of green solid product **8h** was obtained (yield: 74%).

Ru complex **(8h)** ¹HNMR (400 MHz, CDCl₃): δ 16.56 (s, 1H, Ru=CH), 8.33 (dd, *J* = 8.4, 2.4 Hz, 1H), 7.56 (d, *J* = 2.4 Hz), 7.20-6.94 (m, 5H), 5.22 (d, *J* = 11.2 Hz, 1H), 4.21-3.96 (m, 4H, NCH₂CH₂N), 3.56 (s, 3H), 3.54 (d, *J* = 11.2 Hz, 1H), 2.94-0.92 (m, 21H).

### Example 72

### Synthesis of Ru complex 8j

The synthetic procedure for preparation of ligand **7j** is the same as in Example 1 in 5.0 mmol scale. 0.58g of yellow oil product **7j** was obtained (yield: 46%).

Ligand **7j** ¹HNMR (400 MHz, CDCl₃): δ 8.24 (d, *J* = 2.8 Hz, 1H), 8.08 (dd, *J* = 8.8, 2.8 Hz, 1H), 7.03 (d, *J* = 8.8 Hz, 1H), 6.79 (dd, J = 17.6, 10.8 Hz, 1H), 5.79 (dd, J = 17.6, 1.2 Hz, 1H), 5.4 (dd, J = 10.8, 1.2 Hz, 1H), 5.05 (m, 1H), 3.94 (s, 2H), 3.024 (s, 3H), 1.24 (d, J = 6.4 Hz, 6H).

The procedure for preparation of Ru complex **8j** is the same as in Example 1 in 1.0 mmol scale. 320 mg of green solid product **8j** was obtained (yield: 43%).

Ru complex **(8j)** ¹HNMR (400 MHz, CDCl₃): δ 16.64 (s, 1H, Ru=CH), 8.34 (dd, *J* = 8.4, 2.4 Hz, 1H), 7.54 (d, *J* = 2.4 Hz, 1H), 7.25-6.93 (m, 5H), 5.17 (d, *J* = 11.2 Hz, 1H), 4.84-4.83 (m, 1H), 4.14-3.93 (m, 4H, NCH₂CH₂N), 3.45 (d, *J* = 11.2 Hz, 1H), 2.89-1.19 (m, 27 H).

### Example 73

### Synthesis of Ru complex 8k

The synthetic procedure for preparation of ligand **7k** is the same as in Example 1 in 5.0 mmol scale. 0.53g of product **7k** was obtained (yield: 44%). The product **7k** is confirmed by LC-MS (M+H⁺): m/z calculated: 251.2, found: 251.2, and directly used for preparation of the Ru complex **8k.**

The procedure for preparation of Ru complex **8k** is the same as in Example 1 in 1.0 mmol scale. 530 mg of green solid product **8k** was obtained (yield: 74%).

Ru complex **(8k)** ¹HNMR (400MHz, CDCl₃): δ 16.70 (s, 1H, Ru=CH), 7.18-7.13 (m, 3H), 7.05 (s, 1H), 6.96-6.94 (m, 2H), 6.48-6.45 (dd, *J*=8.0, 2.0 Hz, 1H), 5.19-5.16 (d, *J*=15.5 Hz, 1H), 4.17 (s, 2H), 3.94 (s, 2H), 3.62 (s, 3H), 3.50-3.47 (d, *J*=15.5 Hz, 1H), 2.94 (s, 3H), 2.80 (s, 3H), 2.49 (s, 3H), 2.32 (s, 6H), 2.00 (s, 6H).

### Example 74

### Synthesis of Ru complex 8m

The synthetic procedure for preparation of ligand **7m** is the same as in Example 1 in 5.0 mmol scale. 0.76g of yellow oil product **7m** was obtained (yield: 68%). Ligand **7m** ¹H-NMR (CDCl₃, 400Hz): δ 7.15-7.13 (m, 1H), 7.11-7.08 (m, 1H), 7.05-6.99 (m, 1H), 6.93-6.89 (m, 1H), 5.68-5.65 (m, 1H), 5.32-5.30 (d, 1H), 3.74 (s, 2H), 3.69 (s, 3H), 2.86 (s, 3H).

The procedure for preparation of Ru complex **8m** is the same as in Example 1 in 1.0 mmol scale. 430 mg of green solid product **8m** was obtained (yield: 41%).

Ru complex **(8m)** ¹HNMR (400MHz, CDCl₃): δ 16.67 (s, 1H, Ru=CH), 7.10-7.16 (m, 3H), 7.02 (s, 1H), 6.91-6.94 (m, 2H), 6.43-6.45 (dd, *J*=8.75,2.5 Hz, 1H), 5.13-5.16 (d, *J*=15.5 Hz, 1H), 4.15 (s, 2H), 3.91(s, 2H), 3.59 (s, 3H), 3.44-3.47 (d, *J*=15.0 Hz, 1H), 2.92 (s, 3H), 2.77(s, 3H), 2.47 (s, 3H), 2.29 (s, 6H), 1.97 (s, 6H)..

### Example 75

### Synthesis of Ru complex 8n

The synthetic procedure for preparation of ligand **7n** is the same as in Example 1 in 5.0 mmol scale. 0.79g of yellow oil product **7n** was obtained (yield: 71%).

Ligand **7n** ¹H-NMR (400 MHz, CDCl₃): δ 7.02 (dd, J = 9.6, 3.2 Hz, 1H), 6.87 (td, *J* = 8.8, 3.2 Hz, 1H), 6.79 (dd, , *J* = 17.2, 11.2 Hz, 1H), 6.43 (dd, *J* = 8.8, 4.8 Hz, 1H), 5.65 (dd, *J* = 17.2, 1.6 Hz, 1H), 5.39 (dd, *J* = 11.2, 1.6 Hz, 1H), 5.11 (m, 1H), 3.85 (s, 2H), 1.27 (d, *J* = 6.4 Hz, 6H).

The procedure for preparation of Ru complex **8n** is the same as in Example 1 in 1.0 mmol scale. 599 mg of green solid product **8n** was obtained (yield: 87%).

Ru complex **(8n)** ¹HNMR (400 MHz, CDCl₃): δ16.82 (s, 1H, Ru=CH), 7.12-7.02 (m, 5H), 6.64 (m, 1H), 6.51-6.48 (m, 1H), 4.15 (s, 4H, NCH₂CH₂N), 3.95-3.92 (m, 1H), 3.74 (s, 3H), 2.50-2.37 (m, 18H), 0.96 (d, *J* = 6.4 Hz, 1H).

### Example 76

### Synthesis of Ru complex 8p

The synthetic procedure for preparation of ligand **7p** is the same as in Example 1 in 5.0 mmol scale. 0.365g of product **7p** was obtained (yield: 27%).

Ligand **7p** ¹H-NMR (400 MHz, CDCl₃): δ 7.42 (m, 1H), 7.17-7.15 (m, 1H), 7.09-7.06 (m, 1H), 7.04-6.98 (m, 1H), 5.69-5.65 (m, 1H), 5.30-5.27 (m, 1H), 5.0-4.95 (m, 1H), 3.75 (s, 2H), 3.23-3.19 (m, 2H), 1.19-1.18 (d, 6H), 1.07-1.04 (m, 3H).

The procedure for preparation of Ru complex **8p** is the same as in Example 1 in 1.0 mmol scale. 167 mg of green solid product **8p** was obtained (yield: 23%).

Ru complex **(8p)** ¹HNMR (400 MHz, CDCl₃): δ16.52 (s, 1H, Ru=CH), 7.34-32 (dd, *J* = 8.5, 2.0 Hz, 1H), 7.17 (s, 1H), 7.08 (s, 1H), 7.03 (s, 1H), 6.93 (s, 1H), 6.79-6.77 (d, *J* = 8.0 Hz, 1H), 6.66 (s, 1H), 5.08-5.05 (d, *J* = 14.5 Hz, 1H), 4.81-4.76 (m, 1H), 4.16 (s, 2H, NCH₂CH₂N), 3.90 (s, 2H, NCH₂CH₂N), 3.62-3.59 (d, *J=* 16.0 Hz, 1H, NCH₂), 2.91 (s, 3H), 2.81 (s, 3H), 2.48 (s, 3H), 2.32 (s, 3H), 2.30 (s, 3H), 2.16-2.09 (m, 2H, NCH₂CH₃), 1.95 (s, 3H), 1.24-1.19 (dd*, J* = 17.5, 6.0 Hz, 6H, OCH(CH₃)₂), 0.53-0.50 (t, *J* = 5.5 Hz, 3H, NCH₂CH₃).

### Example 77

### Synthesis of Ru complex 8q

The synthetic procedure for preparation of ligand **7q** is the same as in Example 1 in 5.0 mmol scale. 487mg of yellow oil product **7q** was obtained (yield: 38%).

Ligand **7q** ¹H-NMR (400 MHz, CDCl₃): δ 7.29-7.26 (m, 1H), 7.17-7.14 (m, 1H), 6.85-6.14 (m, 2H), 6.56-6.55 (d, 1H), 5.65-5.62 (m, 1H), 5..37-5.30 (m, 1H), 4.19-4.15 (m, 1H), 3.74 (s, 3H), 1.57-1.50 (d, 3H).

The procedure for preparation of Ru complex **8q** is the same as in Example 1 in 1.0 mmol scale. 147mg of brown solid product **8q** was obtained (yield: 21%).

Ru complex **(8q)** ¹HNMR (400 MHz, CDCl₃): δ16.91 (s, 1H, Ru=CH), 7.43-7.40 (m, 1H), 7.08-7.03 (m, 5H), 6.85-6.84 (d, *J* = 6.5 Hz, 1H), 6.72-6.70 (d, *J* = 7.5 Hz, 1H), 4.12 (s, 4H, NCH₂CH₂N), 4.07 (s, 1H, NH), 4.02-3.98 (m, 1H, NCH), 3.76 (s, 3H, COOCH₃), 2.52 (s, 9H), 2.39 (brs, 9H), 1.02-1.01 (d, *J* = 6.0 Hz, 3H)

### Example 78

### Synthesis of Ru complex 8r

The synthetic procedure for preparation of ligand **7r** is the same as in Example 1 in 5.0 mmol scale. 1.06g of yellow oil product **7r** was obtained (yield: 83%). The product **7r** is confirmed by LC-MS (M+H⁺): m/z calculated: 285.1, found: 285.1, and directly used for preparation of Ru complex **8r..**

The synthetic procedure for preparation of Ru complex **8r** is the same as in Example 1 in 1.0 mmol scale. 386 mg of brown solid product **8r** was obtained by precipitation in hexane and MeOH, and the crude product **8r** is unstable and difficult to detect the structure by ¹HNMR. But the crude Ru complex **6j** could be directly used for metathesis reaction.

### Example 79

### Synthesis of Ru complex 8s

The synthetic procedure for preparation of ligand **7s** is the same as in Example 1 in 5.0 mmol scale. 1.18g of yellow oil product **7s** was obtained (yield: 67%).

Ligand **7s** ¹H-NMR (400 MHz, CDCl₃): δ 7.44 (d, J = 2.7Hz, 1H, aromatic H), 7.64 (d, J = 3.0 Hz, 1H, aromatic H), 7.16-7.12 (m, 2H, aromatic H), 7.08-6.92 (m, 2H, aromatic H, CH=CH₂), 6.76 (d, J = 8.7 Hz, 1H, aromatic H), 5.66 (dd, J = 17.7, 1.5Hz, 1H, CH=CH₂), 5.25 (dd, J = 10.8, 0.9 Hz, 1H, CH=CH₂), 4.46 (t, J = 6.0 Hz, 1H, OCH), 4.06 (s, 2H, NCH₂), 2.63 (d, J = 8.4 Hz, 3H, NCH₃), 1.31-1.26 (m, 6H, OCH(CH₃)₂).

The procedure for preparation of Ru complex **8s** is the same as in Example 1 in 1.0 mmol scale. 379 mg of green solid product **8s** was obtained (yield: 48%).

Ru complex **(8s)** ¹HNMR (400 MHz, CDCl₃): δ17.58 (d, *J* = 6.0 Hz, 1H, Ru=CH), 7.59-7.55 (m, 2H), 7.48 (d, *J* = 8.4 Hz, 1H), 7.22 (dd, *J* = 2.4, 8.8 Hz, 1H), 7.14 (d, *J* = 8.4 Hz, 1H), 6.78 (d, *J* = 8.8 Hz, 1H), 4.80 (d, *J* = 12.8 Hz, 1H), 4.50-4.47 (m, 1H), 4.05 (d, *J* = 12.8 Hz, 1H), 2.704 (s, 3H), 2.38-0.78 (m, 39H, PCy₃).

### Example 80

### Synthesis of Ru complex 8t

The synthetic procedure for preparation of ligand **7t** is the same as in Example 1 in 5.0 mmol scale. 0.83g of product **7t** was obtained (yield: 51%). The product **7q** is confirmed by LC-MS (M+H⁺): m/z calculated: 316.1, found: 316.1, and directly used for preparation of the Ru complex **8t.**

The procedure for preparation of Ru complex **8t** is the same as in Example 1 in 1.0 mmol scale. 602 mg of green solid product **8t** was obtained (yield: 77%).

Ru complex **(8t)** ¹HNMR (400 MHz, CDCl₃): δ16.87 (s, 1H, Ru=CH), 7.41 (dd, *J* = 2, 8.4 Hz, 1H), 7.19-7.13 (m, 5H), 7.031 (d, *J* = 8.4 Hz, 1H), 6.93 (d, *J* = 7.2 Hz, 1H), 6.77-6.76 (m, 2H), 6.65 (t, *J* = 7.2 Hz, 1H), 4.66 (d, *J* = 12.4 Hz, 1H), 4.48-4.43 (m, 1H), 4.02-3.98 (m, 5H), 2.54-2.30 (m, 18H), 2.25 (s, 3H), 1.29 (d, *J* = 6 Hz, 6H).

### Example 81

### Synthesis of Ru complex 8u

The synthetic procedure for preparation of ligand **7u** is the same as in Example 1 in 5.0 mmol scale. 1.21g of yellow oil product **7u** was obtained (yield: 71%).

Ligand **7u** ¹H-NMR (400 MHz, CDCl₃): δ 7.44 (d, J = 2.7Hz, 1H, aromatic H), 7.64 (d, J = 3.0 Hz, 1H, aromatic H), 7.16-7.12 (m, 2H, aromatic H), 7.08-6.92 (m, 2H, aromatic H, CH=CH₂), 6.76 (d, J = 8.7 Hz, 1H, aromatic H), 5.66 (dd, J = 17.7, 1.5Hz, 1H, CH=CH₂), 5.25 (dd, J = 10.8, 0.9 Hz, 1H, CH=CH₂), 4.46 (t, J = 6.0 Hz, 1H, OCH), 4.06 (s, 2H, NCH₂), 2.63 (d, J = 8.4 Hz, 3H, NCH₃), 1.31-1.26 (m, 6H, OCH(CH₃)₂).

The procedure for preparation of Ru complex **8u** is the same as in Example 1 in 1.0 mmol scale. 302 mg of green solid product **8u** was obtained (yield: 37%).

Ru complex **(8u)** ¹HNMR (400 MHz, CDCl₃): δ16.84 (s, 1H, Ru=CH), 7.18 (d, *J* = 8.4 Hz, 1H), 7.81 (m, 5H), 6.75 (m, 1H), 6.62 (d, *J* = 8.8 Hz, 1H), 6.32 (d, *J* = 8.4 Hz, 1H), 4.29-4.24 (m, 1H), 4.11 (s, 4H, NCH₂CH₂N), 3.85 (d, *J* = 14.0 Hz, 1H), 3.09 (d, *J* = 14.0 Hz, 1H), 2.74 (s, 3H), 2.43-2.28 (m, 18H), 1.10 (d, *J* = 6.0 Hz, 6H).

### Example 82 (Not a part of the present application)

### Synthesis of Ru complex 10a

The synthetic procedure for preparation of ligand **9a** is the same as in Example 1 in 5.0 mmol scale. 0.97g of yellow oil product **9a** was obtained (yield: 93%).

Ligand **9a** ¹H-NMR (400 MHz, CDCl₃): δ 7.80 (dd, *J* = 8.4, 5.7 Hz, 1H), 7.38 (dd, *J* = 17.7, 11.1 Hz, 1H), 7.14 (dd*, J* = 10.5, 2.7 Hz, 1H), 6.90 (td, *J* = 8.4, 2.1 Hz,1H), 5.55 (d, *J* = 17.7 Hz, 1H), 5.30 (d, *J* = 11.1 Hz, 1H), 5.17-5.09 (m,1H),1.27 (d, *J* = 6.3 Hz, 6H).

The procedure for preparation of Ru complex **10a** is the same as in Example 1 in 1.0 mmol scale. 128 mg of green solid product **10a** was obtained (yield: 19%). The product is unstable, so it is difficult to detect the structure by ¹HNMR. But the crude Ru complex **10a** could be directly used for metathesis reaction.

### Example 83 (Not a part of the present application)

### Synthesis of Ru complex 10b

The synthetic procedure for preparation of ligand **9b** is the same as in Example 1 in 5.0 mmol scale. 0.89g of yellow oil product **9b** was obtained (yield: 87%).

Ligand **9b** ¹H-NMR (400 MHz, CDCl₃): δ 7.80 (dd, *J* = 8.4, 5.7 Hz, 1H), 7.38 (dd, *J* = 17.7, 11.1 Hz, 1H), 7.14 (dd, *J* = 10.5, 2.7 Hz, 1H), 6.90 (td, *J* = 8.4, 2.1 Hz,1H), 5.55 (d, *J* = 17.7 Hz, 1H), 5.30 (d, *J* = 11.1 Hz, 1H), 5.17-5.09 (m,1H),1.27 (d, *J* = 6.3 Hz, 6H).

The procedure for preparation of Ru complex **10b** is the same as in Example 1 in 1.0 mmol scale. 97 mg of green solid product **10b** was obtained (yield: 15%). The product is unstable, so it is difficult to detect the structure by ¹HNMR. But the crude Ru complex **10b** could be directly used for metathesis reaction.

### Example 84 (Not a part of the present application)

### Synthesis of Ru complex 10c

The synthetic procedure for preparation of ligand **9c** is the same as in Example 1 in 5.0 mmol scale. 0.82g of product **9c** was obtained (yield: 76%). The product **9c** is confirmed by LC-MS (M+H⁺): m/z calculated: 208.1, found: 208.0, and directly used for preparation of the Ru complex **10c.**

The procedure for preparation of Ru complex **10c** is the same as in Example 1 in 1.0 mmol scale. 29 mg of green solid product **10c** was obtained (yield: 5%).

Ru complex **(10c)** ¹HNMR (400 MHz, CDCl₃): δ 18.68 (s, 1H, Ru=CH), 8.44 (dd, *J* = 8.4, 2.4 Hz, 1H), 8.20 (d, *J* = 8.4 Hz, 1H), 7.60 (d, *J* = 2.4 Hz, 1H), 7.13 (s, 4H), 4.14 (s, 4H, NCH₂CH₂N), 3.97 (s, 3H), 2.48 (s, 12H), 2.459 (s, 6H).

### Example 85 (Not a part of the present application)

### Synthesis of Ru complex 10d

The synthetic procedure for preparation of ligand **9d** is the same as in Example 1 in 5.0 mmol scale. 0.78g of product **9d** was obtained (yield: 72%). The product **9d** is confirmed by LC-MS (M+H⁺): m/z calculated: 236.1, found: 236.1, and directly used for preparation of the Ru complex **10d.**

The procedure for preparation of Ru complex **10d** is the same as in Example 1 in 1.0 mmol scale. 238 mg of green solid product **10d** was obtained (yield: 34%).

Ru complex **(10d)** ¹HNMR (400 MHz, CDCl₃): δ 18.71 (s, 1H, Ru=CH), 8.42 (dd, *J* = 9.0, 2.4 Hz, 1H), 8.18 (d, *J* = 9.0 Hz, 1H), 7.60 (d, *J* = 2.4 Hz, 1H), 7.13 (s, 4H), 5.25 (m, 1H), 4.13 (s, 4H, NCH₂CH₂N), 2.46 (m, 18H), 1.24 (d, *J* = 6.0 Hz, 6H,).

### Example 86 (Not a part of the present application)

### Synthesis of Ru complex 10e

The synthetic procedure for preparation of ligand **9e** is the same as in Example 1 in 5.0 mmol scale. 0.92g of product **9e** was obtained (yield: 82%). The product **9e** is confirmed by LC-MS (M+H⁺): m/z calculated: 225.1, found: 225.1, and directly used for preparation of the Ru complex **10e.**

The procedure for preparation of Ru complex **10e** is the same as in Example 1 in 1.0 mmol scale. 235 mg of green solid product **10e** was obtained (yield: 34%).

Ru complex **(10e)** ¹HNMR (400 MHz, CDCl₃): δ 18.56 (s, 1H, Ru=CH), 7.98 (d, *J* =8.8 Hz, 1H), 8.18 (dd, *J* = 8.8, 2.4 Hz, 1H), 7.11 (s, 4H), 7.06 (d, *J* = 2.4 Hz, 1H), 5.23 (m, 1H), 4.11 (s, 4H, NCH₂CH₂N), 2.45 (m, 18H), 1.28 (d, *J* = 6.0 Hz, 6H).

### Example 87 (Not a part of the present application)

### Synthesis of Ru complex 10f

The synthetic procedure for preparation of ligand **9f** is the same as in Example 1 in 5.0 mmol scale. 1.13g of yellow oil product **9f** was obtained (yield: 81%).

Ligand **9f** ¹H-NMR (400 MHz, CDCl₃): δ 7.52 (d, *J* = 3.0 Hz, 1H), 7.26 (dd, *J* = 8.7Hz, 3.0 Hz, 1H), 6.89 (d, *J* = 8.7 Hz, 1H), 5.75 (m, 1H), 5.21 (m, 1H), 5.07-4.97 (m, 2H), 3.17-3.16 (m, 2H), 2.82 (s, 3H), 2.35-2.28 (m, 2H), 1.35 (d, *J* = 6.0Hz, 6H).

The procedure for preparation of Ru complex **10f** is the same as in Example 1 in 1.0 mmol scale. 274 mg of green solid product **10f** was obtained (yield: 37%).

Ru complex **(10f)** ¹HNMR (400 MHz, CDCl₃): ¹H-NMR (400 MHz, CDCl₃): δ 18.74 (s, 1H, Ru=CH), 8.21 (dd, *J* = 8.0, 2.4 Hz, 1H), 8.08 (d, *J* = 8.0 Hz, 1H), 7.54 (d, *J* = 2.4 Hz, 1H), 7.12 (s, 4H), 5.32 (m, 1H), 5.25 (m, 1H), 4.13 (s, 4H, NCH₂CH₂N), 2.47 (m, 18H), 1.43 (d, *J* = 6.0 Hz), 1.24 (d, *J* = 6.0 Hz, 6H).

### Example 88 (Not a part of the present application)

### Synthesis of Ru complex 10g

The synthetic procedure for preparation of ligand **9g** is the same as in Example 1 in 5.0 mmol scale. 1.43g of yellow oil product **9g** was obtained (yield: 79%).

Ligand **9g** ¹H-NMR (400 MHz, CDCl₃): δ 7.88 (d, *J* = 9.6 Hz, 1H, aromatic H), 7.86-7.21 (m, 5H, aromatic H, CH=CH₂), 6.83 (d, *J* = 9.3 Hz, 1H, aromatic H), 5.68 (d, *J* = 16.8 Hz, 1H, CH=CH₂), 5.40 (d, *J* = 11.1 Hz, 1H, CH=CH₂), 5.32 (s, 2H, OCH₂), 4.55 (m, 1H, OCH(CH₃)₂), 1.31 (d, *J* = 8.1 Hz, 6H, OCH(CH₃)₂).

The procedure for preparation of Ru complex **10g** is the same as in Example 1 in 1.0 mmol scale. 440 mg of green solid product **10g** was obtained (yield: 53%).

Ru complex **(10g)** ¹HNMR (400 MHz, CDCl₃): δ 18.60 (s, 1H, Ru=CH), 8.01 (d, *J* =8.4 Hz, 1H), 7.59 (dd, *J* = 1.6, 8.4 Hz, 1H), 7.31-7.23 (m, 1H), 7.24 (dd, *J* = 2.8, 8.8 Hz, 1H), 6.81 (d, *J* =8.8 Hz, 1H), 6.71 (d, *J* = 2.0 Hz, 1H), 5.33 (s, 2H), 4.52 (m, 1H), 4.16 (s, 4H, NCH₂CH₂N), 2.51 (s, 12H), 2.48 (s, 6H), 1.28 (d, 6H, *J* = 6.0 Hz).

### Example 89 (Not a part of the present application)

### Synthesis of Ru complex 10h

The synthetic procedure for preparation of ligand **9h** is the same as in Example 1 in 5.0 mmol scale. 1.38g of yellow oil product **9h** was obtained (yield: 83%).

Ligand **9h** ¹H-NMR (400 MHz, CDCl₃): δ 7.87 (d, *J* = 8.4 Hz, 1H, aromatic H), 7.55-7.24 (m, 6H, aromatic H, CH=CH₂), 6.95-6.90 (m, 1H, aromatic H), 5.66 (d, *J* = 21.6 Hz, 1H, CH=CH₂), 5.42-5.32 (m, 3H, CH=CH₂, OCH₂), 4.60 (m, 1H, OCH(CH₃)₂), 1.25 (d, *J* = 8.1 Hz, 6H, OCH(CH₃)₂).

The procedure for preparation of Ru complex **10h** is the same as in Example 1 in 1.0 mmol scale. 183 mg of green solid product **10h** was obtained (yield: 23%).

Ru complex **(10h)** ¹HNMR (400 MHz, CDCl₃): δ 18.60 (s, 1H, Ru=CH), 8.00 (d, *J* =8.8 Hz, 1H), 7.55 (d, *J* = 8.4 Hz, 1H), 7.32-7.29 (m, 1H), 7.14 (s, 4H), 7.01-6.70 (m, 4H), 5.38 (s, 2H), 4.56 (m, 1H), 4.16 (s, 4H, NCH₂CH₂N), 2.71 (s, 12H), 2.52 (s, 6H), 1.32 (d, *J* = 6.0 Hz, 6H).

### Example 90 (Not a part of the present application)

### Synthesis of Ru complex 10j

The synthetic procedure for preparation of ligand **9j** is the same as in Example 1 in 5.0 mmol scale. 1.19g of yellow oil product **9j** was obtained (yield: 63%).

Ligand **9j** ¹H-NMR (300 MHz, CDCl₃): δ 8.42 (d, *J* = 2.1 Hz, 1H, aromatic H), 8.14 (d, *J* = 2.1 Hz, 1H, aromatic H), 8.11 (d, *J* = 2.4 Hz, 1H, aromatic H), 7.48-7.24 (m, 3H, aromatic H, CH=CH₂), 6.84 (d, *J* = 9.0 Hz, 1H, aromatic H), 5.84 (d, *J* = 17.7 Hz, 1H, CH=CH₂), 5.53 (d, *J* = 10.8 Hz, 1H, CH=CH₂), 5.37 (s, 2H, OCH₂), 4.57 (m, 1H, OCH(CH₃)₂), 1.32 (d, *J* = 8.1 Hz, 6H, OCH(CH₃)₂).

The procedure for preparation of Ru complex **10j** is the same as in Example 1 in 1.0 mmol scale. 345 mg of yellow solid product **10j** was obtained (yield: 41%).

It is confirmed by Ru complex **(10j)** ¹HNMR (400 MHz, CDCl₃): δ 18.75 (s, 1H, Ru=CH), 8.45 (dd, *J* =8.8, 1.6 Hz, 1H), 8.21 (d, *J* =8.8 Hz, 1H), 7.64 (d, *J* =1.6 Hz, 1H), 7.39-7.25 (m, 2H), 7.17 (s, 4H), 6.83 (d, *J* =8.8 Hz, 1H), 5.37 (s, 2H), 4.53 (m, 1H), 4.15 (s, 4H, NCH₂CH₂N), 2.51 (m, 18H), 1.40 (d, *J* = 6.0 Hz, 6H).

### Example 91 (Not a part of the present application)

### Synthesis of Ru complex 11a

The Ru complex (Grela catalyst **2f**, 1.0mmol) and 4-chlorin pyridine ligand (10mmol) were reacted directly to form another Ru complex **11a** in 20 mL of anhydrous DCM in a 100 mL of three-neck flask filled with inert gas (Ar), and the reaction mixture was stirred for 0.5 hr at room temperature. After complete, 20mL of pentane (-10°C) was added into reaction solution, then filtered and washed with MeOH to obtain 747 mg of yellow-green solid product **11a,** yield: 95%.

Ru complex **(11a)** ¹HNMR (400 MHz, CDCl₃): δ 17.00 (s, 1H), 8.47-6.83 (m, 11H), 4.91 (m, 1H), 4.17 (s, 4H), 2.48-2.41 (m, 18H), 1.26 (d, *J* = 4.4 Hz, 6H).

### Example 92 (Not a part of the present application)

### Synthesis of Ru complex 11b

The synthetic procedure is the same as in Example 85. 394 mg of yellow-green solid product **11b** was obtained (yield: 48%).

Ru complex **(11b)** ¹HNMR (400 MHz, CDCl₃): δ 16.49 (s, 1H), 8.90-8.50 (m, 2H), 7.86 (d, *J* = 7.2 Hz, 1H), 7.47 (dd, *J* = 2.0, 7.2Hz, 1H), 7.33 (m, 1H), 7.27 (m, 1H), 7.08 (s, 3H), 6.90 (d, *J* = 1.6 Hz, 1H), 6.74-6.72 (m, 1H), 4.87-4.84 (m, 1H), 4.19 (s, 4H), 2.48-2.42 (m, 18H), 1.27 (d, *J* = 4.0 Hz, 6H).

### Example 93 (Not a part of the present application)

### Synthesis of Ru complex 11c

The synthetic procedure is the same as in Example 85. 733 mg of yellow-green solid product **11c** was obtained (yield: 95%).

Ru complex **(11c)** ¹HNMR (400 MHz, CDCl₃): δ 16.56 (s, 1H), 7.47 (dd, *J* = 2.0, 7.2 Hz, 1H), 7.31-7.27 (m, 5H), 7.20-7.19 (m, 3H), 7.08-6.94 (m, 1H),6.72 (d, *J* = 6.4Hz, 1H), 4.85-4.81 (m, 1H), 4.18 (s, 3H), 3.85 (s, 4H), 2.48-2.31 (m, 18H), 1.26 (d, *J* = 6.0 Hz, 6H).

### Example 94 (Not a part of the present application)

### Synthesis of Ru complex 11d

The synthetic procedure is the same as in Example 85. 403 mg of yellow-green solid product **11d** was obtained (yield: 52%).

Ru complex **(11d)** ¹HNMR (400 MHz, CDCl₃): δ 16.49 (s, 1H), 8.67 (m, 2H), 7.47 (d, *J* = 5.6 Hz, 1H), 7.37 (m, 3H), 7.08 (s, 3H),6.73 (d, *J* = 6.8Hz, 1H), 4.85-4.83 (m, 1H), 4.19 (s, 4H), 2.48-2.41 (m, 18H), 1.26 (d, *J* = 4.4 Hz, 6H).

### Example 95 (Not a part of the present application)

### Synthesis of Ru complex 11e

The synthetic procedure is the same as in Example 85. 458 mg of yellow-green solid product **11e** was obtained (yield: 59%).

It is confirmed by Ru complex **(11e)** ¹HNMR (400 MHz, CDCl₃): δ 16.52 (s, 1H), 8.60-8.51 (m, 2H), 7.67 (d, *J* = 8.0 Hz, 2H), 7.46 (d, *J* = 2.4 Hz, 1H), 7.06 (s, 4H), 6.88 (d, *J* = 2.4 Hz, 1H), 6.71 (d, *J* = 8.0 Hz, 2H), 4.84-4.81 (m, 1H), 4.16 (s, 4H), 2.45-2.39 (m, 18H), 1.24 (d, *J* = 4.0 Hz, 6H).

### Example 96 (Not a part of the present application)

### Synthesis of Ru complex 11f

The synthetic procedure is the same as in Example 85. 733 mg of yellow-green solid product **11f** was obtained (yield: 97%).

Ru complex **(11f)** ¹HNMR (400 MHz, CDCl₃): δ 16.57 (s, 1H), 7.63-6.69 (m, 11H), 4.83-4.81 (m, 1H), 4.16 (s, 4H), 2.45-2.39 (m, 21H), 1.24 (d, *J* = 4.0 Hz, 6H).

### Example 97 (Not a part of the present application)

### Synthesis of Ru complex 11g

The synthetic procedure is the same as in Example 85. 330 mg of yellow-green solid product **11g** was obtained (yield: 37%).

Ru complex **(11g)** ¹HNMR (400 MHz, CDCl₃): δ 18.67 (s, 1H), 8.40 (m, 1H), 7.47-6.91 (m, 13H), 6.58 (m, 1H), 4.12 (m, 6H), 2.63-2.27 (m, 19H), 1.00 (d, *J* = 4.0 Hz, 6H).

### Example 98 (Not a part of the present application)

### Synthesis of Ru complex 11h

The synthetic procedure is the same as in Example 85. 619 mg of yellow-green solid product **11h** was obtained (yield: 73%).

Ru complex **(11h)** ¹HNMR (400 MHz, CDCl₃): δ 18.67 (s, 1H), 8.43 (s, 1H), 7.45-7.35 (m, 3H), 7.19-6.93 (m, 10H), 6.60 (d, *J* = 7.6 Hz, 1H), 4.15 (m, 6H), 2.52-2.28 (m, 19H), 1.08-0.89 (m, 6H).

### Example 99 (Not a part of the present application)

### Synthesis of Ru complex 11j

The synthetic procedure is the same as in Example 85. 416 mg of yellow-green solid product **11j** was obtained (yield: 49%).

Ru complex **(11j)** ¹HNMR (400 MHz, CDCl₃): δ 18.67 (s, 1H), 8.40 (m, 1H), 7.69-6.90 (m, 13H), 6.60 (m, 1H), 4.12 (m, 6H), 2.62-2.17 (m, 19H), 1.00 (d, *J* = 4.0 Hz, 6H).

### Example 100 (Not a part of the present application)

### Synthesis of Ru complex 11k

The synthetic procedure is the same as in Example 85. 561 mg of yellow-green solid product **11k** was obtained (yield: 63%).

Ru complex **(11k)** ¹HNMR (400 MHz, CDCl₃): δ18.69 (s, 1H), 8.42 (s, 2H), 7.62-6.93 (m, 16H), 6.60 (dd, *J* = 2.0, 7.6 Hz, 2H), 4.14 (s, 6H), 2.52-2.27 (m, 18H), 0.98 (d, *J* = 4.4 Hz, 6H).

### Example 101 (Not a part of the present application)

### Synthesis of Ru complex 11m

The synthetic procedure is the same as in Example 85. 685 mg of yellow-green solid product **11m** was obtained (yield: 78%).

Ru complex **(11m):** ¹H-NMR (400 MHz, CDCl₃): δ 16.85 (s, 1H), 8.42-7.07 (m, 15H), 4.95 (m, 1H), 4.19 (s, 4H), 2.45-2.29 (m, 18H), 1.29 (d, *J* = 4.4 Hz, 6H).

### Example 102 (Not a part of the present application)

### Synthesis of Ru complex 11n

The synthetic procedure is the same as in Example 85. 704 mg of yellow-green solid product **11n** was obtained (yield: 85%).

Ru complex **(11n)** ¹HNMR (400 MHz, CDCl₃): δ16.85 (s, 1H), 8.47-6.85 (m, 16H), 4.94 (m, 1H), 4.19 (s, 4H), 2.40-2.29 (m, 18H), 1.29 (d, *J* = 4.4 Hz, 6H).

### Example 103 (Not a part of the present application)

### Synthesis of Ru complex 11p

The synthetic procedure is the same as in Example 85. 797 mg of yellow-green solid product **11p** was obtained (yield: 96%).

Ru complex **(11p)** ¹HNMR (400 MHz, CDCl₃): δ17.00 (s, 1H), 8.47-6.82 (m, 11H), 4.90 (m, 1H), 4.17 (s, 4H), 2.48-2.41 (m, 18H), 1.26 (d, *J* = 4.4 Hz, 6H).

### Example 104 (Not a part of the present application)

### Synthesis of Ru complex 11q

The synthetic procedure is the same as in Example 85. 365 mg of yellow-green solid product **11q** was obtained (yield: 47%).

Ru complex **(11q)** ¹HNMR (400 MHz, CDCl₃): δ 17.33 (s, 1H), 8.71 (s, 1H), 8.56 (d, *J* = 3.2 Hz, 1H), 7.84 (d, *J* = 6.0Hz, 1H), 7.41-7.34 (m, 1H), 7.23-7.21 (m, 1H), 7.01 (dd, *J* = 3.2, 9.6 Hz), 5.23-5.21 (m, 1H), 2.37-0.90 (m, 33H).

### Example 105 (Not a part of the present application)

### Synthesis of Ru complex 11r

The synthetic procedure is the same as in Example 85. 604 mg of yellow-green solid product **11r** was obtained (yield: 69%).

Ru complex **(11r)** ¹HNMR (400 MHz, CDCl₃): δ 18.65 (s, 1H), 8.56 (s, 1H), 7.50-6.39 (m, 20H), 4.14 (s, 4H), 3.80 (s, 3H), 2.42-2.29 (m, 18H).

### Example 106 (Not a part of the present application)

### Synthesis of Ru complex 4i

Starting material **4-SM** (44g, 100mmol) and anhydrous ethanol (250mL) were added into a 500 mL three-necked flask filled with inert gas (Ar), followed by adding NaOEt (400mmol, 4.0eq) was quickly added with agitation. The reaction mixture was heated to 60 °C. After the reaction was completed in 0.5-1.0 hr, 120 mL of water was added into flask, and the aqueous layer was extracted with pentane (200 mL×3), and the combined organic layers were washed with brine (150 mL×2) solution, then dried over NaSO₄ and concentrated to obtain about 50 mL of crude carbine intermediate **4-1** directly for next step at 0-5 °C.

RuCl₂(PPh₃)₃ (29g, 30mmol) was dissolved in 250 mL of anhydrous DCM in a 500 mL three-neck flask filled with inert gas (Ar), and the DCM solution was cooled to -70°C, then the previously prepared crude carbine intermediate **4-1** (50 mL) was added into the DCM solution at -70 °C. After 10 min, the solution was heated to room temperature, and CuCl (100mmol) was added. After completed in 30 min, the reaction solution was filtered and purified by silica gel column chromatography (eluting solution: n-hexane:DCM = 2:1 to pure DCM). The product was concentrated and washed by anhydrous n-hexane. After dried by vacuum, the Ru complex intermediate **4-2** (19.3g) was obtained.

The intermediate **4-2** (10.0mmol) and tricyclohexylphosphine (PCy₃, 20mmol, 2.0eq.) were dissolved in DCM (30mL) in a 250 mL three-neck flask filled with inert gas (Ar), then stirred at 20°C for about 30 min. After completed, the crude product was purified by flash column to obtain dark-green solid. The solid product was washed with anhydrous methanol and n-hexane to obtain green solid product **4i** (crude yield: 60-70%). The product **4i** is not stable and difficult to analyze the structure by ¹HNMR. But the crud Ru complex **4i** can be used directly to prepare **4j** in next step.

### Example 107 (Not a part of the present application)

### Synthesis of Ru complex 4j

The Ru complex **4i** (5.0mmol) and a ligand H₂IMes(H)(CCl₃) (**4-4**, 10.0mmol, 2.0eq.) were dissolved in anhydrous toluene (30mL) in a 100 mL two-necked flask filled with Ar gas. The reaction mixture was heated to 80°C for 1.5hr. After the reaction was completed, the solution was cooled and filtered, then purified by flash column to obtain dark-green product. The crude product was washed by methanol and pentane-DCM to offer 2.3g of stable green solid product **4j** (yield: 59%).

Ru complex **4j** is confirmed by ¹HNMR (400 MHz, CDCl₃): δ 18.88 (s,1H, Ru=CH), 7.57-6.44 (m, 11H, aromatic H), 5.36 (t, *J* = 13.2 Hz, 1H, NH), 4.16-4.02 (m, 5H, NCH₂, NCH₂CH₂N), 4.01 (d, *J* = 13.2 Hz, 1H, NCH₂), 2.75-2.00 (m, 19H, CH(CH₃)₂, aromatic CH₃), 1.01-0.90 (m, 6H, CH(CH3)₂).

### Example 108 (Not a part of the present application)

### Synthesis of Ru complex 11h

The Ru complex **4j** (0.2mmol) and 4-chlorin pyridine 4-chlorin pyridine ligand (**4-5,** 2.0mmol) were reacted directly to form another Ru complex **11h** in 10 mL of anhydrous DCM in a 100 mL three-neck flask filled with inert gas (Ar). Preparation method and result of the Ru complex **11h** was the same as described in Example **92.** 619 mg of yellow-green solid product **11h** was obtained (yield: 73%).

Ru complex **11h** is confirmed by ¹HNMR (400 MHz, CDCl₃): δ 18.67 (s, 1H), 8.43 (s, 1H), 7.45-7.35 (m, 3H), 7.19-6.93 (m, 10H), 6.60 (d, *J* = 7.6 Hz, 1H), 4.15 (m, 6H), 2.52-2.28 (m, 19H), 1.08-0.89 (m, 6H).

### Example 109 (Not a part of the present application)

### Synthesis of Ru complex 2j

**SM-2b** (10.4g, 50 mmol) and RuCl₂(PPh₃)₃ (48g, 50 mmol) were dissolved in 250 mL of anhydrous THF in a 500mL three-neck round-bottom flask filled with inert gas (Ar) and reacted to form the Ru complex **2h**. The reaction mixture was stirred at room temperature until completed (monitored by TLC), and the reaction product **2h** was worked out by precipitation in hexane and dried over 42g (yield: 95%).

**2h** (8.9g, 10 mmol) and a new ligand **3x** (3.1g, 11 mmol) with CuCl (12 mmol) were dissolved in 100 mL of anhydrous DCM in a 500mL three-neck round-bottom flask filled with inert gas (Ar) and reacted to form another Ru complex **2j.** The reaction mixture was stirred until complete (monitored by TLC), and the reaction product **2j** was worked out and dried over (6.2g, yield: 89%). The product **2j** is not very stable and directly used for next step to prepare new developed Ru complexes **IIa** and **IIb.**

### Example 110 (Not a part of the present application)

### Synthesis of Ru complex 4x

**2j** (0.71g, 1.0 mmol) and a phosphine ligand PCy₃ (**4-3**, 1.5 mmol) were dissolved in 10 mL of anhydrous DCM in a 50mL three-neck flask filled with inert gas (Ar) and reacted to form the Ru complex **4x.** The reaction mixture was stirred until completed (monitored by TLC), the reaction product was precipitated in MeOH and filtered and purified by flask column. 0.56g of green solid product **4x** was obtained, yield: 78%.

The Ru complex **4x** prepared in this Example 110 is confirmed by ¹HNMR as the same as in Example 21.

### Example 111 (Not a part of the present application)

### Synthesis of Ru complex 4aa

Ru complex **4x** (0.72g, 1.0 mmol) and heterocyclic ligand H₂IMes(H)(CCl₃) (**4-4**, 48g, 50 mmol) were dissolved in 10 mL of anhydrous Toluene in a 50mL three-neck flask filled with inert gas (Ar) and reacted to form the Ru complex **4x.** The reaction mixture was stirred until complete (monitored by TLC), the reaction solution was filtered and purified by flask column. 0.55g of green solid product **4x** was obtained (yield: 73%).

The Ru complex **4aa** prepared in this Example 111 is confirmed by ¹HNMR as the same as in Example 24.

### Example 112

### RCM reaction

RCM test by selecting the Ru Complexes of Examples **1-108** as Catalyst **General Procedure** for RCM Catalyzed by Ru Complex in DCM: Olefin substrate (**15** or **17,** 50mg/each, respectivrly) was dissolved in 1.0 mL of freshly distilled DCM in a 15mL two-neck round-bottom flask under Ar at 20-25 °C, then Ru catalyst (2 mol% of Ru complex selected from Examples **1-103,** respectively) was added into the DCM solution. The kinetic data for conversion of RCM reactions in Equations 1-2 were determined by HPLC at 10 min., 30 min. 1.5 hr, 3.0 hr and until completed overnight. The RCM product (**16** and **18,** respectivrly) was determined and the conversion results of RCM reactions were listed in Tables 1-1, 1-2, 1-3, 1-4, 1-5, and 2 above, respectively.

The RCM product **16** is confirmed by ¹HNMR (400 MHz, CDCl₃): δ 7.72 (d, *J* = 8.2 Hz,, 1H), 7.32 (d, *J* = 8.0 Hz, 1H), 5.66 (d, *J* = 4.4 Hz, 1H), 4.11 (d, *J* = 4.4 Hz, 1H), 2.42 (s, 3H). *m*/*z* calculated: 222.1; found: 222.2.

The RCM product **18** is confirmed by ¹HNMR (400 MHz, CDCl₃): δ 7.78 (d, 2H, *J* = 8.21Hz), 7.31 (m, 7H), 6.01 (m, 1H), 4.47 (m, 2H), 4.30 (m, 2H), 2.41 (s, 3H). (M+H⁺): *m*/*z* calculated: 300.1, found: 300.2.

### Example 113

### Catalyst Screening for Cross Metathesis Reaction

CM test by selecting Ru Complexes of Examples **1-108** as Catalyst(s) **General Procedure** for CM Catalyzed by Ru Complex in DCM: Olefin substrate (**19,** 200mg/each, respectivrly) was dissolved in 3.0 mL of freshly distilled DCM in a 15mL two-neck round-bottom flask under Ar at 20-25 °C, then Ru catalyst (0.1 mol% of Ru complex selected from Examples **1-103,** respectively) was added into the DCM solution. The CM reaction results are described in section of Equation 3 above.

### Example 114

### Catalyst Screening for ROMP reaction without solvent

ROMP test by selecting Ru Complexes of Examples **1-108** as Catalyst(s) **General Procedure** for ROMP Catalyzed by Ru Complex without solvent for some liquid olefin substrates: Olefin substrate (**21, 23** or **25,** 5mL/each, respectivrly) was added into a 25mL flat-bottom bottle under Ar at 40-50°C, then Ru catalyst (0.1 mol% of Ru complex selected from Examples **1-103,** respectively) was added with agitation. The kinetic data and ROMP results for products **22, 24** and **26** are described in each section of Equation 4-6 above, respectively.

### Example 115

### Catalyst Screening for ROMP reaction with solvent

ROMP test by selecting Ru Complexes of Examples **1-108** as Catalyst(s) **General Procedure** for ROMP Catalyzed by Ru Complex in solution: 0.5g of cyclo-olefin substrate (**21, 23, 25, 27, 29,** or **31,** respectivrly) was dissolved in 10 mL of freshly distilled DCM in a 25mL two-neck round-bottom flask under Ar at 20-25 °C, then Ru catalyst (0.1 mol% of Ru complex selected from Examples **1-103,** respectively) was added into the DCM solution. The ROMP results for products **22, 24, 26 , 28, 30** and **32** are described in each section of Equation 4-9 above, respectively.

### Example 116

### Catalyst Screening for Depolymerization of nitrile butadiene rubber by Metathesis

### Metathesis Depolymerization test by selecting Ru Complexes from Examples 1-108 as Catalyst(s)

**General Procedure** for depolymerization Catalyzed by Ru Complex: 60g of nitrile butadiene rubber (NBR) was dissolved in 500 mL of anhydrous chlorobenzene in a 1.0L well-sealed steel reactor under Ar at 30 °C, then the Ru catalyst (**4ab,** 0.04 wt%, one of Ru complexes selected from Examples **1-108**) was added into chlorobenzene solution. The depolymerization by Ru catalyst was conducted overnight to produce lower molecular weight rubber as shown in Equation 10. The depolymerized butyl rubber product was precipitated in MeOH, and dried over 97% of yield. The final rubber product has a Mw of **2.78E+05, a Mn of 1.586E+5, and a Mooney viscosity of 60.3.**

### Example 117

### Catalyst Screening for Metathesis and Hydrogenation reactions of nitrile butadiene rubber

### Metathesis and Hydrogenation test by selecting Ru Complexes from Examples 1-108 as Catalyst(s)

**General Procedure** for Metathesis and Hydrogenation Catalyzed by Ru Complex in solution: 60g of nitrile butadiene rubber (NBR, Raw Material) substrate was dissolved in 500 mL of anhydrous chlorobenzene in a 1.0L steel well-sealed reactor under Ar, then Ru catalyst (**4aa,** 0.07 wt% of Ru complex selected from Examples **1-108,** respectively) was added into chlorobenzene solution, followed by adding hydrogen under high pressure 5MPa, and finally heated upto 130°C overnight. The hydrogenated nitrile butadiene rubber product (HNBR) by Ru catalyst was prepared with lower molecular weight and higher hydrognation degree as shown in Equation 11. The depolymerized and hydrogenated butyl rubber product was precipitated in MeOH, and dried over 98% of yield. The final product has a Mw of 1.60E+05, a Mn of 1.12E+05, an Iodine value of 12.6, and a hydrogenation degree of greater than 95%.

### Example 118

### Catalyst Screening for Hydrogenation and Metathesis reactions of nitrile butadiene rubber

### Metathesis and Hydrogenation test by selecting selecting Ru Complexes from Examples 1-108 as Catalyst(s)

**General Procedure** for Metathesis and Hydrogenation Catalyzed by Ru Complex in solution: 60g of nitrile butadiene rubber (NBR) substrate was dissolved in 500 mL of anhydrous chlorobenzene in a 1.0L steel well-sealed reactor under Ar, then hydrogen was added under high pressure 5MPa, followed by adding Ru catalyst (**4aa,** 0.1 wt% of Ru complex selected from Examples **1-108**) into chlorobenzene solution, then heated upto 130°C overnight. The hydrogenated nitrile butadiene rubber product (HNBR) by Ru catalyst was prepared with higher hydrognation degree and lower molecular weight as shown in Equation 12. The hydrogenated butyl rubber product was precipitated in MeOH, and dried over 98% of yield. The final product has a Mw of 1.80E+05, a Mn of 1.07E+05, an Iodine value of 3.1, and a hydrogenation degree of greater than 99%.

## Claims

1. A transition metal complex having the following structure **IIb,** 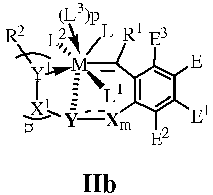 wherein:
m = 0 or 1, n = 1;
p = 0;
M is ruthenium;
L¹ and L² are the same or different and each selected from halogen anion (Cl⁻, Br⁻ or I⁻) anion;
L is an electron-donating ligand having the following structure **IIIa** or **IIId:** 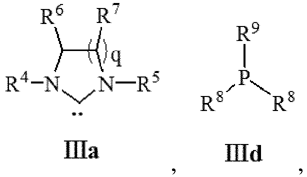
and in **IIIa,** q = 1, R⁴ and R⁵ each is 2,4,6-trimethylphenyl, R⁶ and R⁷ each is H; or in **IIId,** R⁸ and R⁹ each is cyclohexyl (Cy);
when m = 1, X is CH₂; Y is NH, C₁-C₂₀ alkylimino or or C₆-C₂₀ arylamino; "Y---X" is single bond;
when m = 0, Y is NH C₁-C₂₀ alkylimino, or C₆-C₂₀ arylamino;
X¹ and Y¹ are each oxygen, carbonyl, C₆-C₂₀ aryl or CH₂;
R¹ is H;
R² is H, or C₁-C₂₀ alkyl;
E is H, halogen, nitro, C₁-C₄ alkoxy, C₁-C₄ alkoxycarbonyl, or C₁-C₈ alkylaminosulfonyl;
E¹ and E² are each H, or halogen;
E³ is H;
E⁴ is H or C₁-C₄ alkyl;
E⁵ and E⁶ is H, halogen, C₁-C₄ alkyl or C₁-C₆ alkoxy;
E⁷ is H or C₁-C₄ alkyl.

2. The transition metal complex according to claim 1, wherein
R² is methyl, ethyl, or isopropyl.

3. The transition metal complex according to claim 1, wherein the transition metal complex is represented by any one of the following structures,

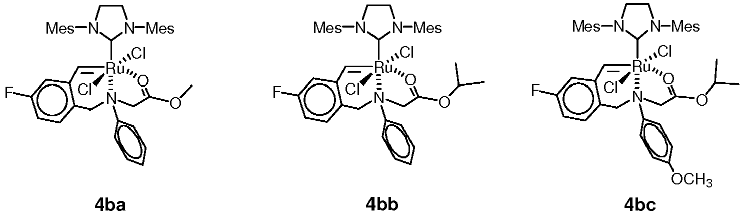

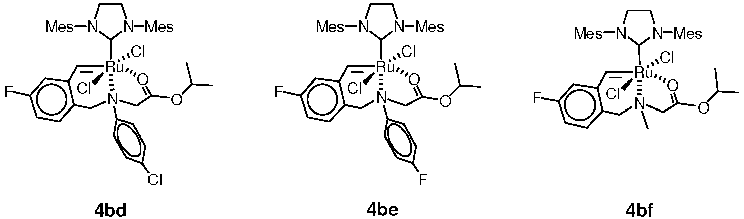

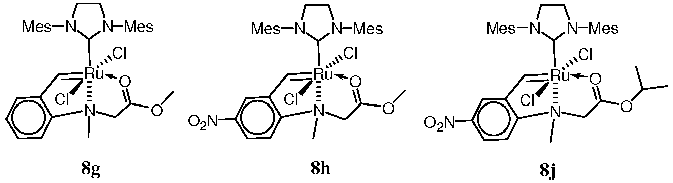

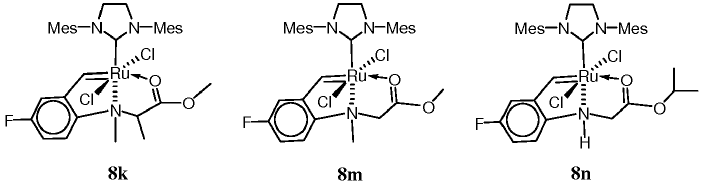

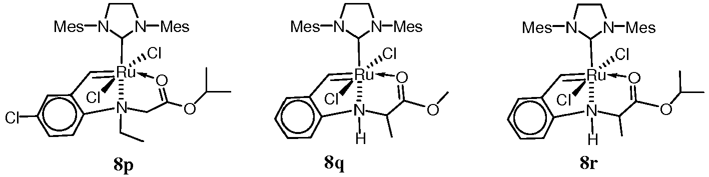

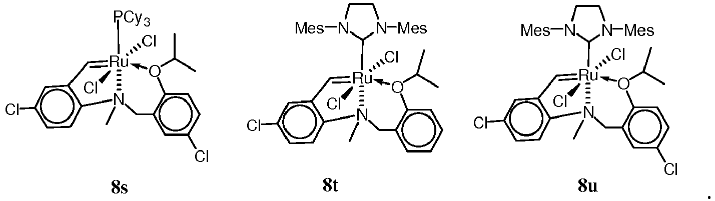

4. A method of carrying out a metathesis reaction with olefin substrate, comprising intramolecular ring-closing metathesis (RCM), intermolecular cross metathesis (CM), acyclic diene metathesis (ADMET) or ring-opening metathesis polymerization (ROMP) of cyclo-olefin substrate in the presence of one or more transition metal complexes of claim 1.

5. The method according to claim 4, wherein cyclo-olefin substrate for ROMP is selected from dicyclopentadiene (DCPD), norbornene, cyclooctene, or a kind of tensional cycloolefin; each is optionally substituted or unsubstituted with one or more of F, Cl, Br, C₁-C₁₅ alkyl, C₁-C₁₅ alkoxy, C₁-C₁₅ alkylthio, C₂-C₁₅ alkenyloxy, C₁-C₁₅ silanyl, C₁-C₁₅ alkylsilyloxy, C₆-C₁₅ aryl, C₆-C₁₅ aryloxy, C₁-C₁₅ alkylcarbonyl, C₆-C₁₅ arylcarbonyl, C₁-C₁₅ alkoxycarbonyl, C₆-C₁₅ aryloxycarbonyl, C₁-C₁₅ alkylaminocarbonyl, C₆-C₁₅ arylaminocarbonyl, C₁-C₁₅ alkylamido, C₆-C₁₅ arylamido, C₁-C₁₅ alkylaminosulfonyl, C₆-C₁₅ arylaminosulfonyl, C₁-C₁₅ sulfonylamido, C₃-C₁₅ heteroaryl or C₂-C₁₅ heterocyclic group.

6. The method according to claim 4, wherein the tensional cycloolefin is a cycloolefin substrate having the following structure **VIa-VIc;** 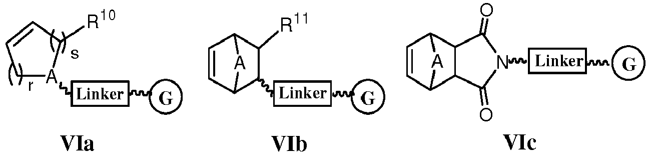
Wherein, r = 1, 2, 3 or 4; s = 1, 2, 3 or 4;
**A** is O, S, C₁-C₁₅ alkyl, C₁-C₁₅ alkoxy, C₁-C₁₅ aryloxy, C₁-C₁₅ alkylthio, C₁-C₁₅ alkoxycarbonyl, C₁-C₁₅ alkylamino, C₆-C₁₅ arylamino, C₁-C₁₅ alkylaminocarbonyl, C₆-C₁₅ arylaminocarbonyl, C₁-C₁₅ alkylamido, C₆-C₁₅ arylamido, or C₁-C₁₅ heterocyclic amido group;
**G** is a group of compounds with specific properties and uses; each is optionally selected from commercial drugs or liquid crystal monomers;
R¹⁰ and R¹¹ are each H, halogen, C₁-C₁₅ alkyl, C₁-C₁₅ alkoxy, C₁-C₁₅ alkylthio, C₁-C₁₅ alkylsilyoxy, C₆-C₁₅ aryloxy, C₆-C₁₅ aryl, C₂-C₁₅ heterocyclic, C₃-C₁₅ heterocyclic aryl, C₁-C₁₅ alkylcarbonyl, C₁-C₁₅ alkyloxycarbonyl, C₆-C₁₅ aryloxycarbonyl, C₁-C₁₅ alkylaminocarbonyl, C₆-C₁₅ arylaminocarbonyl, C₁-C₁₅ alkylamido, C₁-C₁₅ alkylsulfonyl, C₁-C₁₅ alkylsulfonamido, liquid crystal monomer or modified pro-drug;
"Linker" is C₁-C₁₅ alkyl, C₁-C₁₅ alkoxy, C₁-C₁₅ alkylthio, C₁-C₁₅ alkylsilyoxy, C₆-C₁₅ aryloxy, C₆-C₁₅ aryl, C₁-C₁₅ alkoxycarbonyl, C₆-C₁₅ aryloxycarbonyl, C₁-C₁₅ alkylaminocarbonyl, C₆-C₁₅ arylaminocarbonyl, C₁-C₁₅ alkylamido, C₆-C₁₅ arylamido, C₁-C₁₅ alkylsulfonamido, C₆-C₁₅ arylsulfonamido, C₃-C₁₅ heteroaryl or C₂-C₁₅ heterocyclic group.

7. The method according to claim 4, wherein r =1, 2, 3 or 4; s = 1, 2, 3 or 4;:
**A** is O, S, C₁-C₈ alkyl, C₁-C₈ alkoxy, C₁-C₈ aryloxy, C₁-C₁₅ alkylthio, C₁-C₈ alkoxycarbonyl, C₁-C₈ alkylamino, C₆-C₁₂ arylamino, C₁-C₈ alkylaminocarbonyl, C₆-C₁₂ arylaminocarbonyl, C₁-C₈ alkylamido, C₆-C₁₂ arylamido, or C₁-C₈ heterocyclic amido group;
**G** is a kind of compounds with specific properties and uses; each is optionally selected from commercial liquid crystal monomers or modified prodrugs;
R¹⁰ and R¹¹ are each H, halogen, C₁-C₈ alkyl, C₁-C₈ alkoxy, C₁-C₈ alkylthio, C₁-C₈ alkylsilyoxy, C₆-C₁₂ aryloxy, C₆-C₁₂ aryl, C₂-C₈ heterocyclic, C₃-C₁₂ heterocyclic aryl, C₁-C₈ alkylcarbonyl, C₁-C₈ alkyloxycarbonyl, C₆-C₁₂ aryloxycarbonyl, C₁-C₈ alkylaminocarbonyl, C₆-C₁₂ arylaminocarbonyl, C₁-C₈ alkylamido, C₁-C₈ alkylsulfonyl, C₁-C₈ alkylsulfonamido, liquid crystal monomer or modified pro-drug;
"Linker" is C₁-C₈ alkyl, C₁-C₈ alkoxy, C₁-C₈ alkylthio, C₁-C₈ alkylsilyoxy, C₆-C₁₂ aryloxy, C₆-C₁₂ aryl, C₁-C₈ alkoxycarbonyl, C₆-C₁₂ aryloxycarbonyl, C₁-C₈ alkylaminocarbonyl, C₆-C₁₂ arylaminocarbonyl, C₁-C₈ alkylamido, C₆-C₁₂ arylamido, C₁-C₈ alkylsulfonamido, C₆-C₁₂ arylsulfonamido, C₃-C₁₂ heteroaryl or C₂-C₈ heterocyclic group.

8. The method according to claim 4, wherein in structure **VIa-VIc,** r = 1 or 2, and s = 1 or 2;
**A** is O, CH₂, C₁-C₅ alkyl-amino, C₁-C₅ alkoxy, C₁-C₅ alkylaminocarbonyl or C₁-C₅ heterocyclic amido group;
"Linker" is C₁-C₆ alkyl, C₁-C₅ alkoxy, C₁-C₅ alkylthio, C₁-C₅ alkoxycarbonyl, C₁-C₅ alkylaminocarbonyl, C₆-C₁₂ arylaminocarbonyl, C₁-C₅ alkylamido or C₆-C₁₂ arylamido group;
R¹⁰ and R¹¹ are each H, C₁-C₅ alkoxy, C₆-C₁₂ aryloxy, C₁-C₅ alkoxycarbonyl, C₆-C₁₂ aryloxycarbonyl, C₁-C₅ alkylaminocarbonyl, C₆-C₁₂ arylaminocarbonyl, C₁-C₅ alkylamido, C₆-C₁₂ arylamido, liquid crystal monomer or modified prodrugs.

9. The method according to claim 4, wherein **G** is a kind of optionally modified prodrug of commercial drug Lipitor having the following structure **VIIa-VIId:** 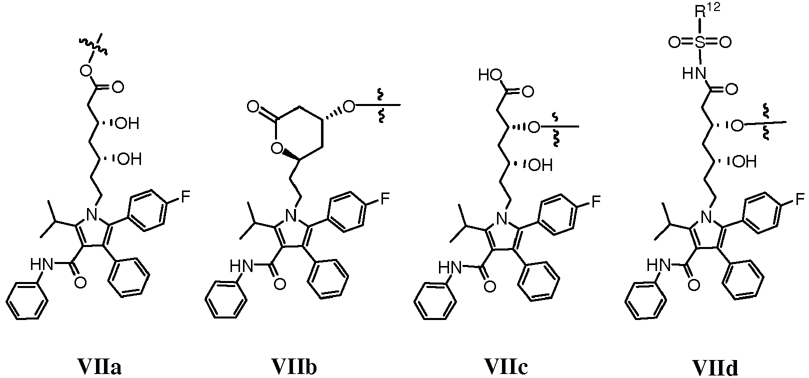 wherein R¹² is cyclopropyl, C₁-C₁₅ alkly, C₃-C₁₅ cycloalkyl, C₁-C₁₅ alkoxy, C₆-C₁₅ aryl, C₆-C₁₅ aryloxy, C₁-C₁₅ alkylamino, C₆-C₁₅ arylamino, C₁-C₁₅ alkylsulfonamido, C₆-C₁₅ arylsulfonamido, C₃-C₁₅ heterocyclic aryl or C₂-C₁₅ heterocyclic group.

10. The method according to claim 4, wherein R¹² is cyclopropyl, C₁-C₆ alkly, C₃-C₆ cycloalkyl, C₁-C₆ alkoxy, C₆-C₁₂ aryl, C₆-C₁₂ aryloxy, C₁-C₆ alkylamino, C₆-C₁₂ arylamino, C₁-C₆ alkylsulfonamido, C₆-C₁₂ arylsulfonamido, C₃-C₁₂ heterocyclic aryl or C₂-C₆ heterocyclic group.

11. A method of making a modified nitrile butadiene rubber (NBR) or styrene-butadiene rubber (SBR) by depolymerization in the presence of one or more transition metal complexes of claim 1 at 30-100°C.

12. A method of making a depolymerized HNBR (hydrogenated nitrile butadiene rubber) or styrene-butadiene rubber (SBR) by adding one or more transition metal complexes of claim 1 first to carry out depolymerization of NBR, followed by adding hydrogen into the reaction under high pressure for hydrogenation at 60-150°C.

13. A method of making a hydrogenated nitrile butadiene rubber (HNBR) or styrene-butadiene rubber by adding hydrogen under high pressure first, followed by adding one or more transition metal complexes of claim 1 at 60-150°C.

14. A use of transition metal complexes of claim 1 in depolymerization of a rubber comprising at least one carbon-carbon double bond.

15. A use of transition metal complexes of claim 1 in hydrogenation of a rubber comprising at least one carbon-carbon double bond.

## Patentansprüche

1. Übergangsmetallkomplex mit der folgenden Struktur IIb, 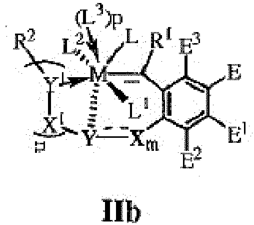 wobei:
m = 0 oder 1, n = 1;
p = 0;
M Ruthenium ist;
L¹ und L² gleich oder verschieden und jeweils ausgewählt aus Halogenanion (Cl⁻, Br⁻ oder I⁻) Anion sind;
L ein elektronenabgebender Ligand mit der folgenden Struktur IIIa oder IIId ist: 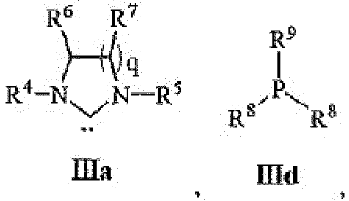
und in IIIa, q = 1, R⁴ und R⁵ jeweils 2,4,6-Trimethylphenyl sind, R⁶ und R⁷ jeweils H sind; oder in IIId sind R⁸ und R⁹ jeweils Cyclohexyl(Cy);
wenn m = 1, ist X CH₂; Y ist NH, C₁-C₂₀ Alkylimino oder C₆-C₂₀ Arylamino; "Y---X" ist eine Einfachbindung;
wenn m = 0, ist Y NH C₁-C₂₀ Alkylimino, oder C₆-C₂₀ Arylamino;
X¹ und Y¹ sind jeweils Sauerstoff, Carbonyl, C₆-C₂₀ Aryl oder CH₂;
R¹ ist H;
R² ist H, oder C₁-C₂₀ Alkyl;
E ist H, Halogen, Nitro, C₁-C₄ Alkoxy, C₁-C₄ Alkoxycarbonyl, oder C₁-C₈ Alkylaminosulfonyl;
E¹ und E² sind jeweils H oder Halogen;
E³ ist H;
E⁴ ist H oder C₁-C₄ alkyl;
E⁵ und E⁶ ist H, halogen, C₁-C₄ Alkyl oder C₁-C₆ Alkoxy;
E⁷ ist H oder C₁-C₄ Alkyl.

2. Übergangsmetallkomplex nach Anspruch 1, wobei
R² Methyl, Ethyl oder Isopropyl ist.

3. Übergangsmetallkomplex nach Anspruch 1, wobei der Übergangsmetallkomplex durch eine der folgenden Strukturen dargestellt wird,

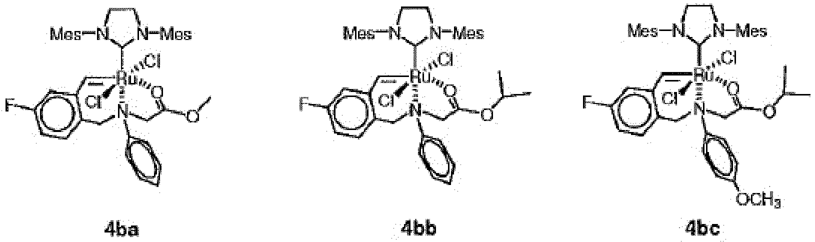

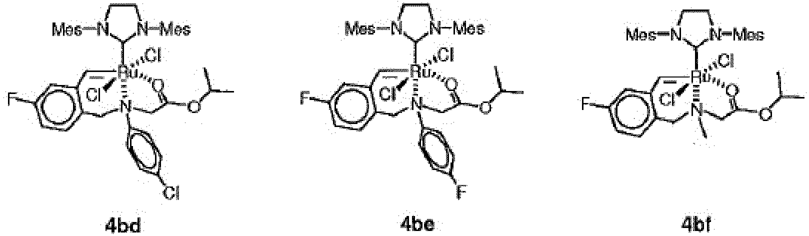

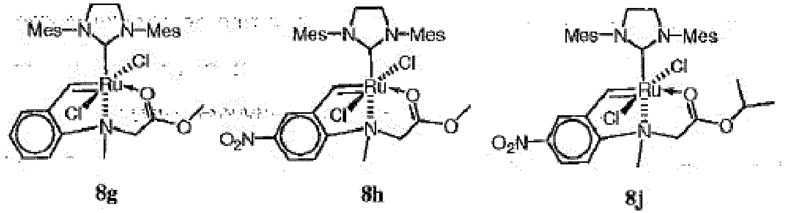

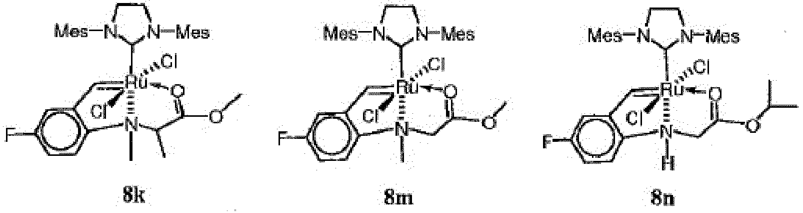

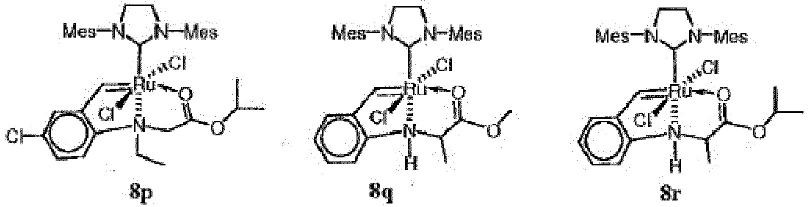

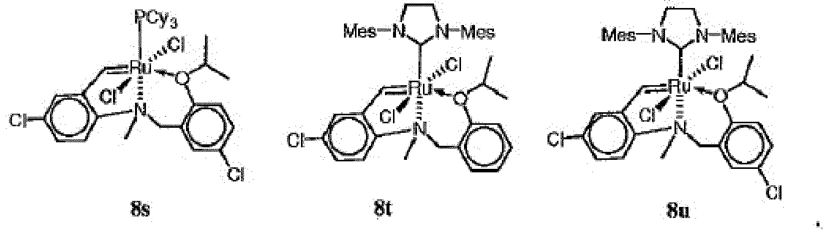

4. Verfahren zur Durchführung einer Metathesereaktion mit einem Olefinsubstrat, umfassend intramolekulare Ringschluß-Metathese (RCM), intermolekulare Kreuz-Metathese (CM), acyclische Dien-Metathese (ADMET) oder ringöffnende Metathesepolymerisation (ROMP) von Cycloolefin-Substrat in Gegenwart eines oder mehrerer Übergangsmetallkomplexe nach Anspruch 1.

5. Verfahren nach Anspruch 4, wobei das Cycloolefin-Substrat für ROMP aus Dicyclopentadien (DCPD), Norbornen, Cycloocten oder einer Art Zug-Cycloolefin ausgewählt wird; jedes ist optional substituiert oder unsubstituiert mit einem oder mehreren von F, Cl, Br, C₁-C₁₅ Alkyl, C₁-C₁₅ Alkoxy, C₁-C₁₅ Alkylthio, C₂-C₁₅ Alkenyloxy, C₁-C₁₅ Silanyl, C₁-C₁₅ Alkylsilyloxy, C₆-C₁₅ Aryl, C₆-C₁₅ Aryloxy, C₁-C₁₅ Alkylcarbonyl, C₆-C₁₅ Arylcarbonyl, C₁-C₁₅ Alkoxycarbonyl, C₆-C₁₅ Aryloxycarbonyl, C₁-C₁₅ Alkylaminocarbonyl, C₆-C₁₅ Arylaminocarbonyl, C₁-C₁₅ Alkylamido, C₆-C₁₅ Arylamido, C₁-C₁₅ Alkylaminosulfonyl, C₆-C₁₅ Arylaminosulfonyl, C₁-C₁₅ Sulfonylamido, C₃-C₁₅ Heteroaryl oder C₂-C₅ heterocyclischer Gruppe.

6. Verfahren nach Anspruch 4, wobei das Zug-Cycloolefin ein Cycloolefinsubstrat mit der folgenden Struktur VIa-VIc ist; 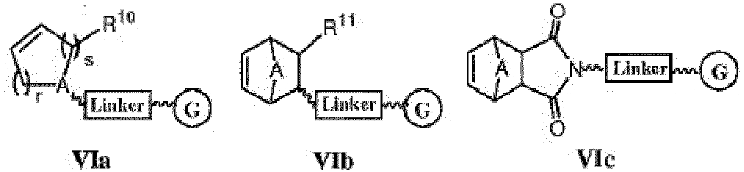
Wobei, r = 1, 2, 3 oder 4; s = 1, 2, 3 oder 4;
A ist 0, S, C₁-C₁₅ Alkyl, C₁-C₁₅ Alkoxy, C₁-C₁₅ Aryloxy, C₁-C₁₅ Alkylthio, C₁-C₁₅ Alkoxycarbonyl, C₁-C₁₅ Alkylamino, C₆-C₁₅ Arylamino, C₁-C₁₅ Alkylaminocarbonyl, C₆-C₁₅ Arylaminocarbonyl, C₁-C₁₅ Alkylamido, C₆-C₁₅ Arylamido, oder C₁-C₁₅ heterocyclische Amidogruppe;
G ist eine Gruppe von Verbindungen mit spezifischen Eigenschaften und Anwendungen; jede davon ist optional aus kommerziellen Arzneimitteln oder Flüssigkristallmonomeren ausgewählt;
R¹⁰ und R¹¹ sind jeweils H, Halogen, C₁-C₁₅ Alkyl, C₁-C₁₅ Alkoxy, C₁-C₅ Alkylthio, C₁-C₅ Alkylsilyoxy, C₆-C₁₅ Aryloxy, C₆-C₁₅ Aryl, C₂-C₁₅ heterocyclisch, C₃-C₁₅ heterocyclisches Aryl, C₁-C₁₅ Alkylcarbonyl, C₁-C₁₅ Alkyloxycarbonyl, C₆-C₁₅ Aryloxycarbonyl, C₁-C₁₅ Alkylaminocarbonyl, C₆-C₁₅ Arylaminocarbonyl, C₁-C₁₅ Alkylamido, C₁-C₁₅ Alkylsulfonyl, C₁-C₁₅ Alkylsulfonamid, Flüssigkristallmonomer oder modifiziertes Prodrug; "Linker" ist C₁-C₁₅ Alkyl, C₁-C₁₅ Alkoxy, C₁-C₁₅ Alkylthio, C₁-C₁₅ Allcylsilyoxy, C₆-C₁₅ Aryloxy, C₆-C₁₅ Aryl, C₁-C₁₅ Alkoxycarbonyl, C₆-C₁₅ Aryloxycarbonyl, C₁-C₁₅ Alkylaminocarbonyl, C₆-C₁₅ Arylaminocarbonyl, C₁-C₁₅ Alkylamido, C₆-C₁₅ Arylamido, C₁-C₁₅ Alkylsulfonamido, C₆-C₁₅ Arylsulfonamido, C₃-C₁₅ Heteroaryl oder C₂-C₁₅ heterocyclische Gruppe.

7. Verfahren nach Anspruch 4, wobei r =1, 2, 3 oder 4; s = 1, 2, 3 oder 4; :
A ist 0, S, C₁-C₈ Alkyl, C₁-C₈ Alkoxy, C₁-C₈ Aryloxy, C₁-C₁₅ Alkylthio, C₁-C₈ Alkoxycarbonyl, C₁-C₈ Alkylamino, C₆-C₁₂ Arylamino, C₁-C₈ Alkylaminocarbonyl, C₆-C₁₂ Arylaminocarbonyl, C₁-C₈ Alkylamido, C₆-C₁₂ Arylamido, oder C₁-C₈ heterocyclische Amidogruppe;
G ist eine Art von Verbindungen mit spezifischen Eigenschaften und Anwendungen; jede davon ist optional aus kommerziellen Flüssigkristallmonomeren oder modifizierten Prodrug ausgewählt;
R¹⁰ und R¹¹ sind jeweils H, Halogen, C₁-C₈ Alkyl, C₁-C₈ Alkoxy, C₁-C₈ Alkylthio, C₁-C₈ Alkylsilyoxy, C₆-C₁₂ Aryloxy, C₆-C₁₂ Aryl, C₂-C₈ heterocyclisch, C₃-C₁₂ heterocyclisches Aryl, C₁-C₈ Alkylcarbonyl, C₁-C₈ Alkyloxycarbonyl, C₆-C₁₂ Aryloxycarbonyl, C₁-C₈ Alkylaminocarbonyl, C₆-C₁₂ Arylaminocarbonyl, C₁-C₈ Alkylamido, C₁-C₈ Alkylsulfonyl, C₁-C₈ Alkylsulfonamid, Flüssigkristallmonomer oder modifiziertes Prodrug;
"Linker" ist C₁-C₈ Alkyl, C₁-C₈ Alkoxy, C₁-C₈ Alkylthio, C₁-C₈ Alkylsilyoxy, C₆-C₁₂ Aryloxy, C₆-C₁₂ Aryl, C₁-C₈ Alkoxycarbonyl, C₆-C₁₂ Aryloxycarbonyl, C₁-C₈ Alkylaminocarbonyl, C₆-C₁₂ Arylaminocarbonyl, C₁-C₈ Alkylamido, C₆-C₁₂ Arylamido, C₁-C₈ Alkylsulfonamido, C₆-C₁₂ Arylsulfonamido, C₃-C₁₂ Heteroaryl oder C₂-C₈ heterocyclische Gruppe.

8. Verfahren nach Anspruch 4, wobei in der Struktur VIa-VIc, r = 1 oder 2, und s = 1 oder 2;
A ist 0, CH₂, C₁-C₅ Alkyl-amino, C₁-C₅ Alkoxy, C₁-C₅ Alkylaminocarbonyl oder C₁-C₅ heterocyclische Amidogruppe;
"Linker" ist C₁-C₆ Alkyl, C₁-C₅ Alkoxy, C₁-C₅ Alkylthio, C₁-C₅ Alkoxycarbonyl, C₁-C₅ Alkylaminocarbonyl, C₆-C₁₂ Arylaminocarbonyl, C₁-C₅ Alkylamido oder C₆-C₁₂ Arylamido-Gruppe;
R¹⁰ und R¹¹ sind jeweils H, C₁-C₅ Alkoxy, C₆-C₁₂ Aryloxy, C₁-C₅ Alkoxycarbonyl, C₆-C₁₂ Aryloxycarbonyl, C₁-C₅ Alkylaminocarbonyl, C₆-C₁₂ Arylaminocarbonyl, C₁-C₅ Alkylamido, C₆-C₁₂ Arylamido, Flüssigkristallmonomer oder modifizierte Prodrugs.

9. Verfahren nach Anspruch 4, wobei G eine Art optional modifizierten Prodrugs des kommerziellen Wirkstoffs Lipitor mit der folgenden Struktur VIIa-VIId ist: 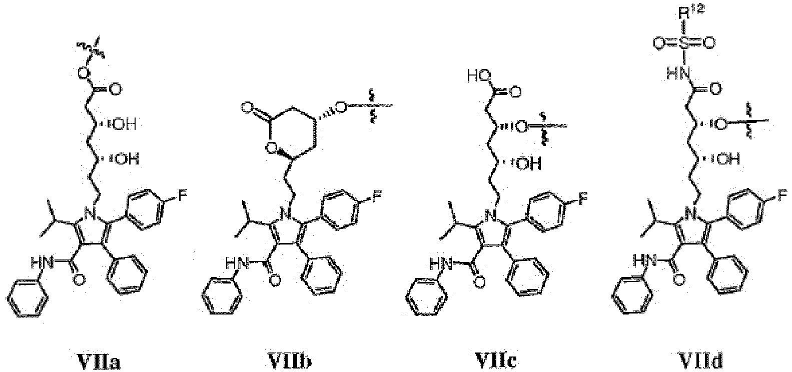 wobei R¹² Cyclopropyl, C₁-C₁₅ Alkyl, C₃-C₁₅ Cycloalkyl, C₁-C₁₅ Alkoxy, C₆-C₁₅ Aryl, C₆-C₁₅ Aryloxy, C₁-C₁₅ Alkylamino, C₆-C₁₅ Arylamino, C₁-C₁₅ Alkylsulfonamido, C₆-C₁₅ Arylsulfonamido, C₃-C₁₅ heterocyclisches Aryl oder C₂-C₁₅ heterocyclische Gruppe ist.

10. Verfahren nach Anspruch 4, wobei R¹² Cyclopropyl, C₁-C₆ Alkyl, C₃-C₆ Cycloalkyl, C₁-C₆ Alkoxy, C₆-C₁₂ Aryl, C₆-C₁₂ Aryloxy, C₁-C₆ Alkylamino, C₆-C₁₂ Arylamino, C₁-C₆ Alkylsulfonamido, C₆-C₁₂ Arylsulfonamido, C₃-C₁₂ heterocyclisches Aryl oder C₂-C₆ heterocyclische Gruppe ist.

11. Verfahren zur Herstellung eines modifizierten Nitrilbutadienkautschuks (NBR) oder Styrol-Butadien-Kautschuks (SBR) durch Depolymerisation in Gegenwart eines oder mehrerer Übergangsmetallkomplexe nach Anspruch 1 bei 30-100 °C.

12. Verfahren zur Herstellung eines depolymerisierten HNBR (hydrierter Nitril-Butadien-Kautschuk) oder Styrol-Butadien-Kautschuks (SBR) durch Zugabe eines oder mehrerer Übergangsmetallkomplexe nach Anspruch 1, um zunächst die Depolymerisation von NBR durchzuführen, gefolgt von der Zugabe von Wasserstoff in die Reaktion unter hohem Druck zur Hydrierung bei 60-150 °C.

13. Verfahren zur Herstellung eines hydrierten Nitrilbutadienkautschuks (HNBR) oder Styrol-Butadien-Kautschuks zunächst durch Zugabe von Wasserstoff unter hohem Druck, gefolgt von der Zugabe eines oder mehrerer Übergangsmetallkomplexe nach Anspruch 1 bei 60-150 °C.

14. Verwendung von Übergangsmetallkomplexen nach Anspruch 1 bei der Depolymerisation eines Kautschuks, der mindestens eine Kohlenstoff-Kohlenstoff-Doppelbindung umfasst.

15. Verwendung von Übergangsmetallkomplexen nach Anspruch 1 bei der Hydrierung eines Kautschuks, der mindestens eine Kohlenstoff-Kohlenstoff-Doppelbindung umfasst.

## Revendications

1. Complexe métallique de transition ayant la structure suivante **IIb,** 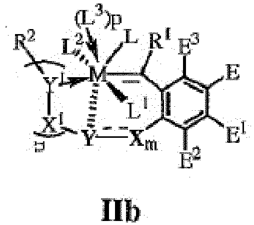 où :
m = 0 ou 1, n = 1 ;
p = 0 ;
M est du ruthénium ;
L¹ et L² sont identiques ou différents et sont chacun choisis à partir d'un anion halogène (anion Cl⁻, Br⁻ or I⁻) ;
L est un ligand donneur d'électrons ayant la structure suivante **IIIa** ou **IIId** : 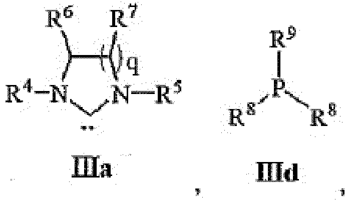
et dans **IIIa,** q = 1, R⁴ et R⁵ sont chacun du 2,4,6-triméthylphényle, R⁶ et R⁷ sont chacun de l'H ; ou dans **IIId,** R⁸ et R⁹ sont chacun du cyclohexyle (Cy) ; lorsque m = 1, X est du CH₂; Y est du NH, de l'alkylimino en C₁-C₂₀ ou de l'arylamino en C₆-C₂₀ ;
« Y---X » est une liaison simple ;
lorsque m = 0, Y est du NH, de l'alkylimino en C₁-C₂₀ ou de l'arylamino en C₆-C₂₀ ;
X¹ et Y¹ sont chacun de l'oxygène, du carbonyle, de l'aryle en C₆-C₂₀ ou du CH₂ ;
R¹ est de l'H ;
R² est de l'H ou de l'alkyle en C₁-C₂₀ ;
E est de l'H, de l'halogène, de la nitro, de l'alkoxy en C₁-C₄, de l'alkoxycarbonyle en C₁-C₄ ou de l'alkylaminosulfonyle en C₁-C₈ ;
E¹ et E² sont tous deux de l'H ou de l'halogène ;
E³ est de l'H ;
E⁴ est de l'H ou de l'alkyle en C₁-C₄ ;
E⁵ et E⁶ est de l'H, de l'halogène, de l'alkyle en C₁-C₄ ou de l'alkoxy en C₁-C₆ ;
E⁷ est de l'H ou de l'alkyle en C₁-C₄.

2. Complexe métallique de transition selon la revendication 1, dans lequel
R² est du méthyle, de l'éthyle ou de l'isopropyle.

3. Complexe métallique de transition selon la revendication 1, dans lequel le complexe métallique de transition est représenté par l'une quelconque des structures suivantes,

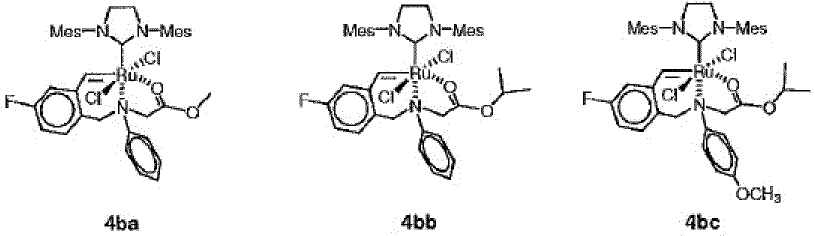

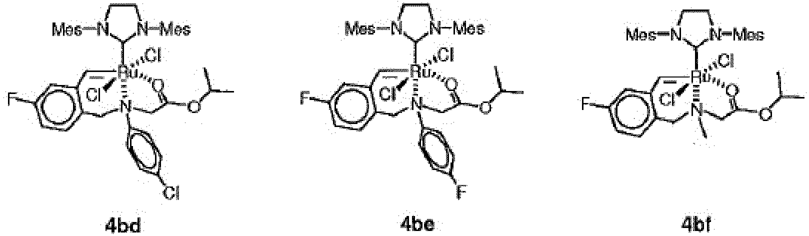

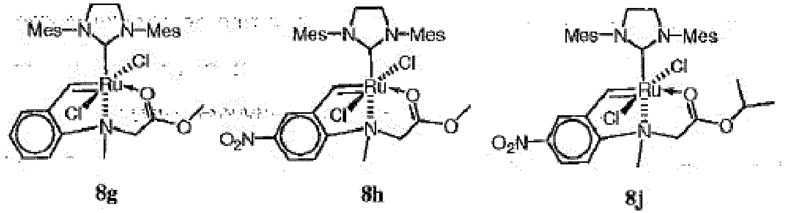

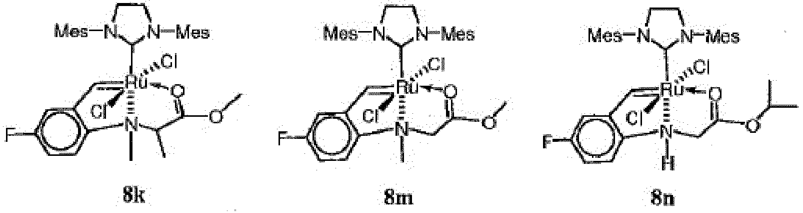

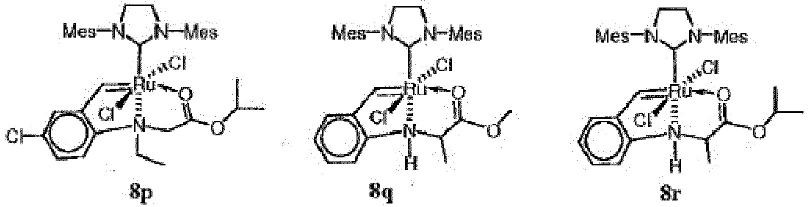

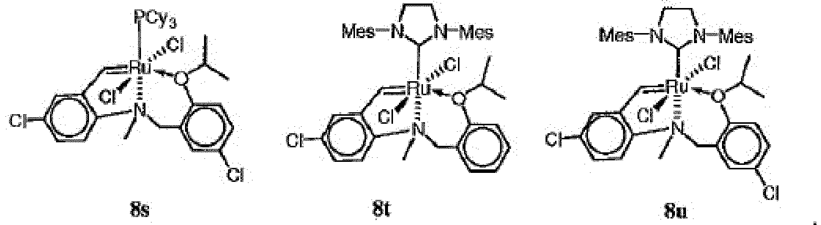

4. Procédé permettant d'effectuer une réaction de métathèse avec un substrat d'oléfine, comprenant une métathèse par fermeture de cycle intramoléculaire (RCM), une métathèse croisée intermoléculaire (CM), une métathèse de diène acyclique (ADMET) ou une polymérisation de métathèse par ouverture de cycle (ROMP) de substrat de cyclooléfine en présence d'un ou plusieurs complexes métalliques de transition selon la

5. Procédé selon la revendication 4, dans lequel le substrat de cyclooléfine pour la ROMP est sélectionné à partir de dicyclopentadiène (DCPD), norbornène, cyclooctène, ou un type de cyclooléfine sous tension ; chacun étant facultativement substitué ou non substitué avec un ou plusieurs des éléments parmi F, Cl, Br, alkyle en C₁-C₁₅, alkoxy en C₁-C₁₅, alkylthio en C₁-C₁₅, alkenyloxy en C₂-C₁₅, silanyle en C₁-C₁₅, alkylsilyloxy en C₁-C₁₅, aryle en C₆-C₁₅, aryloxy en C₆-C₁₅, alkylcarbonyle en C₁-C₁₅, arylcarbonyle en C₆-C₁₅, alkoxycarbonyle en C₁-C₁₅, aryloxycarbonyle en C₆-C₁₅, alkylaminocarbonyle en C₁-C₁₅, arylaminocarbonyle en C₆-C₅, alkylamido en C₁-C₁₅, arylamido en C₆-C₁₅, alkylaminosulfonyle en C₁-C₁₅, arylaminosulfonyle en C₆-C₁₅, sulfonylamido en C₁-C₁₅, hétéroaryle en C₃-C₁₅ ou un groupe hétérocyclique en C₂-C₁₅.

6. Procédé selon la revendication 4, dans lequel le cyclooléfine en tension est un substrat de cyclooléfine ayant la structure suivante **VIa-VIc** ; 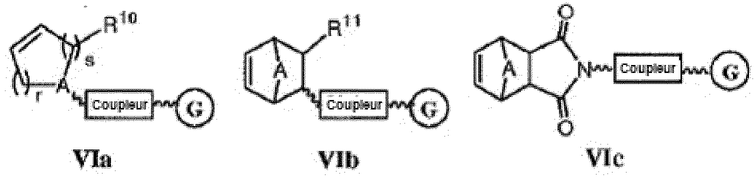
où r = 1, 2, 3 ou 4 ; s = 1, 2, 3 ou 4 ;
A est de l'O, du S, un alkyle en C₁-C₁₅, un alkoxy en C₁-C₁₅, un aryloxy en C₁-C₁₅, un alkylthio en C₁-C₁₅, un alkoxycarbonyle en C₁-C₁₅, un alkylamino en C₁-C₁₅, un arylamino en C₆-C₁₅, un alkylaminocarbonyle en C₁-C₁₅, un arylaminocarbonyle en C₆-C₁₅, un alkylamido en C₁-C₁₅, un arylamido en C₆-C₁₅ ou un groupe amido-hétérocyclique en C₁-C₁₅ ;
G est un groupe de composants ayant des propriétés et des utilisations spécifiques ; chacun est sélectionné facultativement à partir de médicaments se trouvant sur le marché ou de monomères de cristal liquide ;
R¹⁰ et R¹¹ sont chacun un H, un halogène, un alkyle en C₁-C₁₅, un alkoxy en C₁-C₁₅, un alkylthio en C₁-C₁₅, un alkylsilyoxy en C₁-C₁₅, un aryloxy en C₆-C₁₅, un aryle en C₆-C₁₅, un hétérocyclique en C₂-C₁₅, un aryle hétérocyclique en C₃-C₁₅, un alkylcarbonyle en C₁-C₁₅, un alkyloxycarbonyle en C₁-C₁₅, un aryloxycarbonyle en C₆-C₁₅, un alkylaminocarbonyle en C₁-C₁₅, un arylaminocarbonyle en C₆-C₁₅, un alkylamido en C₁-C₁₅, un alkylsulfonyle en C₁-C₁₅, un alkylsulfonamido en C₁-C₁₅, un monomère de cristal liquide ou un promédicament modifié ;
le « Coupleur » est un alkyle en C₁-C₁₅, un alkoxy en C₁-C₁₅, un alkylthio en C₁-C₁₅, un allcylsilyoxy en C₁-C₁₅, un aryloxy en C₆-C₁₅, un aryle en C₆-C₁₅, un alkoxycarbonyle en C₁-C₁₅, un aryloxycarbonyle en C₆-C₁₅, un alkylaminocarbonyle en C₁-C₁₅, un arylaminocarbonyle en C₆-C₁₅, un alkylamido en C₁-C₁₅, un arylamido en C₆-C₁₅, un alkylsulfonamido en C₁-C₁₅, un arylsulfonamido en C₆-C₁₅, un hétéroaryle en C₃-C₁₅ ou un groupe hétérocyclique en C₂-C₁₅.

7. Procédé selon la revendication 4, dans lequel r =1, 2, 3 ou 4 ; s = 1, 2, 3 ou 4 ;
A est de l'O, du S, un alkyle en C₁-C₈, un alkoxy en C₁-C₈, un aryloxy en C₁-C₈, un alkylthio en C₁-C₁₅, un alkoxycarbonyle en C₁-C₈, un alkylamino en C₁-C₈, un arylamino en C₆-C₁₂, un alkylaminocarbonyle en C₁-C₈, un arylaminocarbonyle en C₆-C₁₂, un alkylamido en C₁-C₈, un arylamido en C₆-C₁₂ ou un groupe amido-hétérocyclique en C₁-C₈ ;
G est un type de composants ayant des propriétés et des utilisations spécifiques ; chacun est sélectionné facultativement à partir de monomères de cristal liquides se trouvant sur le marché ou de promédicaments modifiés ;
R¹⁰ et R¹¹ sont chacun un H, un halogène, un alkyle en C₁-C₈, un alkoxy en C₁-C₈, un alkylthio en C₁-C₈, un alkylsilyoxy en C₁-C₈, un aryloxy en C₆-C₁₂, un aryle en C₆-C₁₂, un hétérocyclique en C₂-C₈, un aryle hétérocyclique en C₃-C₁₂, un alkylcarbonyle en C₁-C₈, un alkyloxycarbonyle en C₁-C₈, un aryloxycarbonyle en C₆-C₁₂, un alkylaminocarbonyle en C₁-C₈, un arylaminocarbonyle en C₆-C₁₂, un alkylamido en C₁-C₈, un alkylsulfonyle en C₁-C₈, un alkylsulfonamido en C₁-C₈, un monomère de cristal liquide ou un promédicament modifié ;
le « Coupleur » est un alkyle en C₁-C₈, un alkoxy en C₁-C₈, un alkylthio en C₁-C₈, un alkylsilyoxy en C₁-C₈, un aryloxy en C₆-C₁₂, un aryle en C₆-C₁₂, un alkoxycarbonyle en C₁-C₈, un aryloxycarbonyle en C₆-C₁₂, un alkylaminocarbonyle en C₁-C₈, un arylaminocarbonyle en C₆-C₁₂, un alkylamido en C₁-C₈, un arylamido en C₆-C₁₂, un alkylsulfonamido en C₁-C₈, un arylsulfonamido en C₆-C₁₂, un hétéroaryle en C₃-C₁₂ ou un groupe hétérocyclique en C₂-C₈.

8. Procédé selon la revendication 4, dans lequel dans la structure **VIa-VIc,** r = 1 ou 2 et s = 1 ou 2 ;
A est de l'O, du CH₂, un alkyle-amino en C₁-C₅, un alkoxy en C₁-C₅, un alkylaminocarbonyle en C₁-C₅ ou un groupe amido-hétérocyclique en C₁-C₅ ;
le « Coupleur » est un alkyle en C₁-C₆, un alkoxy en C₁-C₅, un alkylthio en C₁-C₅, un alkoxycarbonyle en C₁-C₅, un alkylaminocarbonyle en C₁-C₅, un arylaminocarbonyle en C₆-C₁₂, un alkylamido en C₁-C₅ ou un groupe arylamido en C₆-C₁₂ ;
R¹⁰ et R¹¹ sont chacun un H, un alkoxy en C₁-C₅, un aryloxy en C₆-C₁₂, un alkoxycarbonyle en C₁-C₅, un aryloxycarbonyle en C₆-C₁₂, un alkylaminocarbonyle en C₁-C₅, un arylaminocarbonyle en C₆-C₁₂, un alkylamido en C₁-C₅, un arylamido en C₆-C₁₂, un monomère de cristal liquide ou des promédicaments.

9. Procédé selon la revendication 4, dans lequel G est un type de promédicament éventuellement modifié de l'atorvastatine, médicament que l'on trouve dans le commerce ayant la structure suivante **VIIa-VIId** : 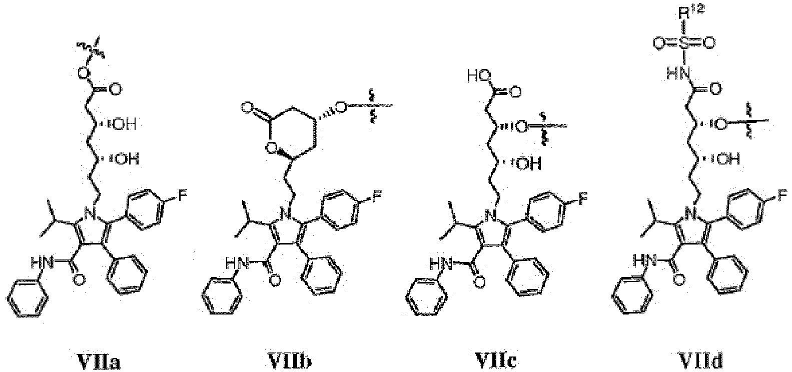 où R¹² est du cyclopropoyle, un alkyle en C¹-C₁₅, un cycloalkyle en C₁-C₁₅, un alkoxy en C₁-C₁₅, un aryle en C₆-C₁₅, un aryloxy en C₆-C₁₅, un alkylamino en C₁-C₁₅, un arylamino en C₆-C₁₅, un alkylsulfonamido en C₁-C₁₅, un arylsulfonamido en C₆-C₁₅, un aryle hétérocyclique en C₃-C₁₅ ou un groupe hétérocyclique en C₂-C₁₅.

10. Procédé selon la revendication 4, où R¹² est du cyclopropyle, un alkyle en C₁-C₆, un cycloalkyle en C₃-C₆, un alkoxy en C₁-C₆, un aryle en C₆-C₁₂, un aryloxy en C₆-C₁₂, un alkylamino en C₁-C₆, un arylamino en C₆-C₁₂, un alkylsulfonamido en C₁-C₆, un arylsulfonamido en C₆-C₁₂, un aryle hétérocyclique en C₃-C₁₂ ou un groupe hétérocyclique en C₂-C₆.

11. Procédé de fabrication d'un caoutchouc nitrile butadiène modifié (NBR) ou d'un caoutchouc butadiène-styrène (SBR) par dépolymérisation en présence d'un ou plusieurs complexes métalliques de transition selon la revendication 1 à 30-100 °C.

12. Procédé de fabrication d'un HNBR dépolymérisé (caoutchouc nitrile butadiène hydrogéné) ou d'un caoutchouc butadiène-styrène (SBR) en ajoutant un ou plusieurs complexes métalliques de transition selon la revendication 1, d'abord pour effectuer une dépolymérisation du NBR, suivi d'un ajout d'hydrogène dans la réaction sous haute pression pour une hydrogénation à 60-150 °C.

13. Procédé de fabrication d'un caoutchouc nitrile butadiène hydrogéné (HNBR) ou d'un caoutchouc butadiène-styrène en ajoutant d'abord de l'hydrogène sous haute pression suivi par l'ajout d'un ou plusieurs complexes métalliques de transition selon la revendication 1 à 60-150 °C.

14. Utilisation de complexes métalliques de transition selon la revendication 1 dans la dépolymérisation d'un caoutchouc comprenant au moins une liaison double carbone-carbone.

15. Utilisation de complexes métalliques de transition selon la revendication 1 dans l'hydrogénation d'un caoutchouc comprenant au moins une liaison double carbone-carbone.
